# EUROPEAN PATENT APPLICATION

(11) **EP 3 945 089 A1**
(43) Date of publication of application: **02.02.2022**
(21) Application number: 20188910.2
(22) Date of filing: 31.07.2020
(51) Int. Cl.: C07C 251/58, A01N 35/10

(54) **USE OF STROBILURIN TYPE COMPOUNDS FOR COMBATING PHYTOPATHOGENIC FUNGI CONTAINING AN AMINO ACID SUBSTITUTION F129L IN THE MITOCHONDRIAL CYTOCHROME B PROTEIN CONFERRING RESISTANCE TO QO INHIBITORS V**

(71) Applicant: BASF SE, 67056 Ludwigshafen am Rhein (DE)
(72) Inventor: Kulkami, Sarang, 412210 Pune (IN); Dey, Chandan, 400705 Navi Mumbai (IN); Poonoth, Manojkumar, 67056 Ludwigshafen (DE); Le Vezouet, Ronan, 67056 Ludwigshafen (DE); Lohmann, Jan Klaas, 67056 Ludwigshafen (DE); Craig, Ian Robert, 67056 Ludwigshafen (DE); Grote, Thomas, 67157 Wachenheim (DE); Khanna, Smriti, 400705 Navi Mumbai (IN); Rath, Rakesh, 400705 Navi Mumbai (IN); Koch, Andreas, 67117 Limburgerhof (DE); Fehr, Marcus, 67117 Limburgerhof (DE); Tegge, Vanessa, 67117 Limburgerhof (DE)
(74) Representative: BASF IP Association

(57) **Abstract**

The present invention relates to the use of strobilurin type compounds of formula I and the N-oxides and the salts thereof for combating phytopathogenic fungi containing an amino acid substitution F129L in the mitochondrial cytochrome b protein (also referred to as F129L mutation in the mitochondrial cytochrome b gene) conferring resistance to Qo inhibitors, and to methods for combating such fungi. The invention also relates to novel compounds, processes for preparing these compounds, to compositions comprising at least one such compound, and to seeds coated with at least one such compound.

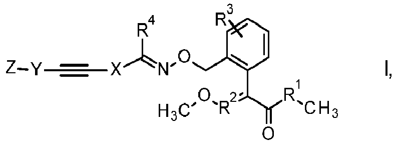

## Description

The present invention relates the use of strobilurin type compounds of formula I and the N-oxides and the salts thereof for combating phytopathogenic fungi containing an amino acid substitution F129L in the mitochondrial cytochrome b protein (also referred to as F129L mutation in the mitochondrial cytochrome b gene) conferring resistance to Qo inhibitors (QoI), and to methods for combating such fungi. The invention also relates to novel compounds, processes for preparing these compounds, to compositions comprising at least one such compound, to plant health applications, and to seeds coated with at least one such compound. The present invention also relates to a method for controlling soybean rust fungi (*Phakopsora pachyrhizi*) with the amino acid substitution F129L in the mitochondrial cytochrome b protein.

"Qo inhibitor," as used herein, includes any substance that is capable of diminishing and/or inhibiting respiration by binding to a ubihydroquinone oxidation center of a cytochrome bc₁ complex in mitochondria. The oxidation center is typically located on the outer side of the inner mitochrondrial membrane. Many of these compounds are also known as strobilurin-type or strobilurin analogue compounds.

The mutation F129L in the mitochondrial cytochrome b (CYTB) gene shall mean any substitution of nucleotides of codon 129 encoding "F" (phenylalanine; e.g. TTT or TTC) that leads to a codon encoding "L" (leucine; e.g. TTA, TTG, TTG, CTT, CTC, CTA or CTG), for example the substitution of the first nucleotide of codon 129 'T' to 'C' (TTT to CTT), in the CYTB (cytochrome b) gene resulting in a single amino acid substitution in the position 129 from F to L in the cytochrome b protein. Such F129L mutation is known to confer resistance to Qo inhibitors.

Qol fungicides, often referred to as strobilurin-type fungicides, are conventionally used to control a number of fungal pathogens in crops. Qo inhibitors typically work by inhibiting respiration by binding to a ubihydroquinone oxidation center of a cytochrome bc₁ complex (electron transport complex III) in mitochondria. Said oxidation center is located on the outer side of the inner mitochrondrial membrane. A prime example of the use of QoIs includes the use of, for example, strobilurins on wheat for the control of *Septoria tritici* (also known as *Mycosphaerella graminicola*)*,* which is the cause of wheat leaf blotch. Unfortunately, widespread use of such Qols has resulted in the selection of mutant pathogens which are resistant to such Qols. Resistance to Qols has been detected in several phytopathogenic fungi such as *Blumeria graminis, Mycosphaerella fijiensis, Pseudoperonspora cubensis* or *Venturia inaequalis.* The major part of resistance to Qols in agricultural uses has been attributed to pathogens containing a single amino acid residue substitution G143A in the cytochrome b gene for their cytochrome bc₁ complex, the target protein of Qols which have been found to be controlled by specific Qols (WO 2013/092224). Despite several commercial Qol fungicides have also been widely used in soybean rust control, the single amino acid residue substitution G143A in the cytochrome b protein conferring resistance to QoI fungicides was not observed.

Instead soybean rust acquired a different genetic mutation in the cytochrome b gene causing a single amino acid substitution F129L which also confers resistance against QoI fungicides. The efficacy of QoI fungicides used against soybean rust conventionally, i.e. pyraclostrobin, azoxystrobin, picoxystrobin, orysastrobin, dimoxystrobin and metominostrobin, has decreased to a level with practical problems for agricultural practice.

Although it seems that trifloxystrobin was less affected by the F129L mutation to the same degree as other QoI fungicides such as azoxystrobin and pyraclostrobin, trifloxystrobin was never as efficacious on a fungal population bearing the F129L Qol resistance mutation as on a sensitive population (Crop Protection 27, (2008) 427-435).

Thus, new methods are desirable for controlling pathogen induced diseases in crops comprising plants subjected to pathogens containing a F129L mutation in the mitochondrial cytochrome b gene conferring resistance to Qo inhibitors. Furthermore, in many cases, in particular at low application rates, the fungicidal activity of the known fungicidal strobilurin compounds is unsatisfactory, especially in case that a high proportion of the fungal pathogens contain a mutation in the mitochondrial cytochrome b gene conferring resistance to Qo inhibitors. Besides there is an ongoing need for new fungicidally active compounds which are more effective, less toxic and/or environmentally safer. Based on this, it was also an object of the present invention to provide compounds having improved activity and/or a broader activity spectrum against phytopathogenic fungi and/or even further reduced toxicity against non target organisms such as vertebrates and invertebrates.

Certain strobilurin type compounds have been described in EP 463488. However, it is not mentioned that these compounds inhibit fungal pathogens containing a F129L substitution in the mitochondrial cytochrome b protein conferring resistance to Qo inhibitors.

The compounds according to the present invention differ from those described in the abovementioned publications that the methyl oxime side chain contains an alkyne linker group as defined herein.

Therefore, the invention provides novel compounds of formula I 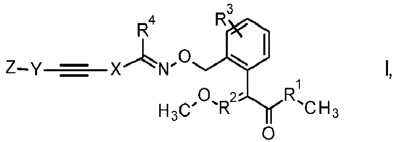 wherein
- R¹: is selected from O and NH;
- R²: is selected from CH and N;
- R³: is selected from hydrogen, halogen, CN, C₁-C₄-alkyl, C₂-C₄-alkenyl, C₂-C₄-alkynyl, C₁-C₄-haloalkyl, C₂-C₄-haloalkenyl, C₂-C₄-haloalkynyl, C₃-C₆-cycloalkyl, -O-C₁-C₄-alkyl, -O-C₁-C₄-haloalkyl, -O-C₃-C₆-cycloalkyl, -C₁-C₂-alkyl-C₃-C₆-cycloalkyl, phenyl, 3- to 6-membered heterocycloalkyl and 5- or 6-membered heteroaryl,
wherein said heterocycloalkyl and heteroaryl besides carbon atoms contain 1, 2 or 3 heteroatoms selected from N, O and S provided that such heterocycle cannot contain 2 contiguous atoms selected from O and S,
wherein said phenyl, heterocycloalkyl and heteroaryl are bound directly or via an oxygen atom or via a C₁-C₂-alkylene linker, and wherein said phenyl and heteroaryl are unsubstituted or substituted by 1, 2 or 3 identical or different substituents selected from halogen, CN, NH₂, NO₂, C₁-C₄-alkyl, C₁-C₄-haloalkyl, -O-C₁-C₄-alkyl and -O-C₁-C₄-haloalkyl;
- R⁴: is selected from hydrogen, C₁-C₆-alkyl, C₂-C₄-alkenyl, C₂-C₄-alkynyl, C₁-C₆-haloalkyl, C₂-C₄-haloalkenyl, C₂-C₄-haloalkynyl, -(C₁-C₂-alkyl)-O-(C₁-C₂-alkyl), -(C₁-C₂-alkyl)-O-(C₁-C₂-haloalkyl) and -C₁-C₄-alkyl-C₃-C₆-cycloalkyl;
- X, Y ,: independently of each other, are a direct bond or the divalent group -CL¹L²-;
- L¹, L²,: independently of each other, are selected from hydrogen, C₁-C₃-alkyl, C₁-C₃-haloalkyl, C₂-C₃-haloalkenyl, C₂-C₃-haloalkynyl, -(C₁-C₂-alkyl)-O-(C₁-C₂-alkyl), -(C₁-C₂-alkyl)-O-(C₁-C₂-haloalkyl), cyclopropyl and -C₁-C₂-alkyl-cyclopropyl; or
L¹ and L², together with the interjacent carbon atom, form a cyclopropyl; wherein the cyclic moieties of L¹ and L², independently of each other, are unsubstituted or carry 1 or 2 identical or different groups R^{L}:
R^{L} is selected from halogen, CN, NO₂, C₁-C₄-alkyl, C₁-C₄-haloalkyl, -O-C₁-C₄-alkyl and -O-C₁-C₄-haloalkyl;
- Z: is selected from C₃-C₆-cycloalkyl, phenyl, 3- to 6-membered heterocycloalkyl, 3- to 6-membered heterocycloalkenyl and 5- or 6-membered heteroaryl,
wherein said heterocycloalkyl, heterocycloalkenyl and heteroaryl besides carbon atoms contain 1, 2 or 3 heteroatoms selected from N, O and S provided that such heterocycle cannot contain 2 contiguous atoms selected from O and S,
and wherein Z is unsubstituted or carries 1, 2, 3 or up to the maximum number of identical or different groups R^{a}:
R^{a} is selected from halogen, CN, C₁-C₄-alkyl, C₂-C₄-alkenyl, C₂-C₄-alkynyl, -O-C₁-C₄-alkyl, -C(=N-O-C₁-C₄-alkyl)-C₁-C₄-alkyl, -C(=O)-C₁-C₄-alkyl, -O-CH₂-C(=N-O-C₁-C₄-alkyl)-C₁-C₄-alkyl, C₃-C₆-cycloalkyl, C₃-C₆-cycloalkenyl, -C₁-C₂-alkyl-C₃-C₆-cycloalkyl, -O-C₃-C₆-cycloalkyl, phenyl, 3- to 6-membered heterocycloalkyl, 3- to 6-membered heterocycloalkenyl and 5- or 6-membered heteroaryl,
wherein said heterocycloalkyl, heterocycloalkenyl and heteroaryl besides carbon atoms contain 1, 2 or 3 heteroatoms selected from N, O and S provided that such heterocycle cannot contain 2 contiguous atoms selected from O and S,
wherein said phenyl, heterocycloalkyl, heterocycloalkenyl and heteroaryl are bound directly or via an oxygen atom or via a C₁-C₂-alkylene linker, and wherein the aliphatic and cyclic moieties of R^{a} are unsubstituted or carry 1, 2, 3, 4 or up to the maximum number of identical or different groups R^{b}:
R^{b} is selected from halogen, CN, NO₂, C₁-C₄-alkyl, C₁-C₄-haloalkyl, -O-C₁-C₄-alkyl, and -O-C₁-C₄-haloalkyl;
and in form or stereoisomers and tautomers thereof, and the N-oxides and the agriculturally acceptable salts thereof.

Although the present invention will be described with respect to particular embodiments, this description is not to be construed in a limiting sense.

Before describing in detail exemplary embodiments of the present invention, definitions important for understanding the present invention are given. As used in this specification and in the appended claims, the singular forms of "a" and "an" also include the respective plurals unless the context clearly dictates otherwise. In the context of the present invention, the terms "about" and "approximately" denote an interval of accuracy that a person skilled in the art will understand to still ensure the technical effect of the feature in question. The term typically indicates a deviation from the indicated numerical value of ±20 %, preferably ±15 %, more preferably ±10 %, and even more preferably ±5 %. It is to be understood that the term "comprising" is not limiting. For the purposes of the present invention the term "consisting of" is considered to be a preferred embodiment of the term "comprising of".

Unless otherwise indicated, the following definitions are set forth to illustrate and define the meaning and scope of the various terms used to describe the invention herein and the appended claims. These definitions should not be interpreted in the literal sense as they are not intended to be general definitions and are relevant only for this application.

The term "compounds I" refers to compounds of formula I. Likewise, this terminology applies to all sub-formulae, e. g. "compounds I.2" refers to compounds of formula 1.2 or "compounds V" refers to compounds of formula V, etc..

The term "independently" when used in the context of selection of substituents for a variable, it means that where more than one substituent is selected from a number of possible substituents, those substituents may be the same or different.
The organic moieties or groups mentioned in the above definitions of the variables are collective terms for individual listings of the individual group members. The term "Cᵥ-C_{w}" indicates the number of carbon atom possible in each case.

The term "halogen" refers to fluorine, chlorine, bromine and iodine.

The term "C₁-C₄-alkyl" refers to a straight-chained or branched saturated hydrocarbon group having 1 to 4 carbon atoms, for example, methyl (CH₃), ethyl (C₂H₅), propyl, 1-methylethyl (isopropyl), butyl, 1-methylpropyl, 2-methylpropyl, 1,1-dimethylethyl.

The term "C₂-C₄-alkenyl" refers to a straight-chain or branched unsaturated hydrocarbon radical having 2 to 4 carbon atoms and a double bond in any position such as ethenyl, 1-propenyl, 2-propenyl, 1-methylethenyl, 1-butenyl, 2-butenyl, 3-butenyl, 1-methyl-1-propenyl, 2-methyl-1-propenyl, 1-methyl-2-propenyl, 2-methyl-2-propenyl.

The term "C₂-C₄-alkynyl" refers to a straight-chain or branched unsaturated hydrocarbon radical having 2 to 4 carbon atoms and containing at least one triple bond such as ethynyl, prop-1-ynyl, prop-2-ynyl, but-1-ynyl, but-2-ynyl, but-3-ynyl, 1-methyl-prop-2-ynyl.

The term "C₁-C₄-haloalkyl" refers to a straight-chained or branched alkyl group having 1 to 4 carbon atoms wherein some or all of the hydrogen atoms in these groups may be replaced by halogen atoms as mentioned above, for example chloromethyl, bromomethyl, dichloromethyl, trichloromethyl, fluoromethyl, difluoromethyl, trifluoromethyl, chlorofluoromethyl, dichlorofluoromethyl, chlorodifluoromethyl, 1-chloroethyl, 1-bromoethyl, 1-fluoroethyl, 2-fluoroethyl, 2,2-difluoroethyl, 2,2,2-trifluoroethyl, 2-chloro-2-fluoroethyl, 2-chloro-2,2-difluoroethyl, 2,2-dichloro-2-fluoroethyl, 2,2,2-trichloroethyl and pentafluoroethyl, 2-fluoropropyl, 3-fluoropropyl, 2,2-difluoropropyl, 2,3-difluoropropyl, 2-chloropropyl, 3-chloropropyl, 2,3-dichloropropyl, 2-bromopropyl, 3-bromopropyl, 3,3,3-trifluoropropyl, 3,3,3-trichloropropyl, CH₂-C₂F₅, CF₂-C₂F₅, CF(CF₃)₂, 1-(fluoromethyl)-2-fluoroethyl, 1-(chloromethyl)-2-chloroethyl, 1-(bromomethyl)-2-bromoethyl, 4-fluorobutyl, 4-chlorobutyl, 4-bromobutyl or nonafluorobutyl.

The term "-O-C₁-C₄-alkyl" refers to a straight-chain or branched alkyl group having 1 to 4 carbon atoms which is bonded via an oxygen, at any position in the alkyl group, e.g. OCH₃, OCH₂CH₃, O(CH₂)₂CH₃, 1-methylethoxy, O(CH₂)₃CH₃, 1-methyl¬propoxy, 2-methylpropoxy or 1,1-dimethylethoxy.

The term "C₃-C₆-cycloalkyl" refers to monocyclic saturated hydrocarbon radicals having 3 to 6 carbon ring members, such as cyclopropyl (C₃H₅), cyclobutyl, cyclopentyl or cyclohexyl. The term "C₃-C₆-cycloalkenyl " refers to monocyclic saturated hydrocarbon radicals having 3 to 6 carbon ring members and one or more double bonds.

The term "3- to 6-membered heterocycloalkyl" refers to 3- to 6-membered monocyclic saturated ring system having besides carbon atoms one or more heteroatoms, such as O, N, S as ring members. The term "C₃-C₆-membered heterocycloalkenyl" refers to 3- to 6-membered monocyclic ring system having besides carbon atoms one or more heteroatoms, such as O, N and S as ring members, and one or more double bonds.

The term "-C₁-C₄-alkyl-C₃-C₆-cycloalkyl" refers to alkyl having 1 to 4 carbon atoms (as defined above), wherein one hydrogen atom of the alkyl radical is replaced by a cycloalkyl radical having 3 to 6 carbon atoms.

The term "phenyl" refers to C₆H₅.

The term "5- or 6-membered heteroaryl" which contains 1, 2, 3 or 4 heteroatoms from the group consisting of O, N and S, is to be understood as meaning aromatic heterocycles having 5 or 6 ring atoms. Examples include:
- 5-membered heteroaryl which in addition to carbon atoms, e.g. contain 1, 2 or 3 N atoms and/or one sulfur and/or one oxygen atom: for example 2-thienyl, 3-thienyl, 3-pyrazolyl, 4-pyrazolyl, 5-pyrazolyl, 2-oxazolyl, 4-oxazolyl, 5-oxazolyl, 2-thiazolyl, 4-thiazolyl, 5-thiazolyl, 2-imidazolyl, 4-imidazolyl and 1,3,4-triazol-2-yl;
- 6-membered heteroaryl which, in addition to carbon atoms, e.g. contain 1, 2, 3 or 4 N atoms as ring members, e.g. 2-pyridinyl, 3-pyridinyl, 4-pyridinyl, 3-pyridazinyl, 4-pyridazinyl, 2-pyrimidinyl, 4-pyrimidinyl, 5-pyrimidinyl and 2-pyrazinyl.

The term "C₁-C₂-alkylene linker" means a divalent alkyl group such as -CH₂- or -CH₂-CH₂-that is bound at one end to the core structure of formula I and at the other end to the particular substituent.

As used herein, the "compounds", in particular "compounds I" include all the stereoisomeric and tautomeric forms and mixtures thereof in all ratios, prodrugs, isotopic forms, their agriculturally acceptable salts, N-oxides and S-oxides thereof.

The term "stereoisomer" is a general term used for all isomers of individual compounds that differ only in the orientation of their atoms in space. The term stereoisomer includes mirror image isomers (enantiomers), mixtures of mirror image isomers (racemates, racemic mixtures), geometric (cis/trans or E/Z) isomers, and isomers of compounds with more than one chiral center that are not mirror images of one another (diastereoisomers). The term "tautomer" refers to the coexistence of two (or more) compounds that differ from each other only in the position of one (or more) mobile atoms and in electron distribution, for example, keto-enol tautomers. The term "agriculturally acceptable salts" as used herein, includes salts of the active compounds which are prepared with acids or bases, depending on the particular substituents found on the compounds described herein. "N-oxide" refers to the oxide of the nitrogen atom of a nitrogen-containing heteroaryl or heterocycle. N-oxide can be formed in the presence of an oxidizing agent for example peroxide such as m-chloro-perbenzoic acid or hydrogen peroxide. N-oxide refers to an amine oxide, also known as amine-N-oxide, and is a chemical compound that contains N→O bond.

In respect of the variables, the embodiments of the intermediates correspond to the embodiments of the compounds I.

Preference is given to those compounds I and where applicable also to compounds of all sub-formulae provided herein, e. g. formulae I.1 and 1.2, and to the intermediates such as compounds II, III, IV and V, wherein the substituents and variables (such as n, X, Y, Z, R¹, R², R³, R⁴, R^{a}, and R^{b}) have independently of each other or more preferably in combination (any possible combination of 2 or more substituents as defined herein) the following meanings:
Preference is also given to the uses, methods, mixtures and compositions, wherein the definitions (such as phytopathogenic fungi, treatments, crops, compounds II, further active ingredients, solvents, solid carriers) have independently of each other or more preferably in combination the following meanings and even more preferably in combination (any possible combination of 2 or more definitions as provided herein) with the preferred meanings of compounds I herein:
One embodiment of the invention relates to preferred compounds I, wherein R¹ is selected from O and NH; and R² is selected from CH and N, provided that R² is N in case R¹ is NH. Another embodiment related to compounds wherein R² is N. A further embodiment relates to compounds I, wherein R² is CH.

According to a further embodiment, R³ is selected from hydrogen, halogen, CN, C₁-C₄-alkyl, C₂-C₄-alkenyl, C₁-C₄-haloalkyl, C₂-C₄-haloalkenyl, C₃-C₆-cycloalkyl, -O-C₁-C₄-alkyl, -O-C₁-C₄-haloalkyl, -C₁-C₂-alkyl-C₃-C₆-cycloalkyl and 3- to 6-membered heterocycloalkyl; more preferably from is selected from hydrogen, halogen, CN, C₁-C₄-alkyl, C₂-C₄-alkenyl, C₂-C₄-alkynyl, C₁-C₄-haloalkyl, C₃-C₄-cycloalkyl, -O-C₁-C₄-alkyl, -O-C₁-C₄-haloalkyl and 3- to 4-membered heterocycloalkyl, wherein said heterocycloalkyl besides carbon atoms contain 1 or 2 heteroatoms selected from N, O and S provided that such heterocycle cannot contain 2 contiguous atoms selected from O and S, and wherein said heterocycloalkyl is bound directly or via an oxygen atom or via a C₁-C₂-alkylene linker; even more preferably from hydrogen, C₁-C₂-alkyl, C₂-alkenyl, C₁-C₂-haloalkyl, -O-C₁-C₂-alkyl, O-C₁-C₂-haloalkyl, C₃-C₄-cycloalkyl, -C₁-C₂-alkyl-C₃-C₄-cycloalkyl, and 3- to 4-membered heterocycloalkyl; further more preferably form hydrogen, C₁-C₂-alkyl, C₁-C₂-haloalkyl, C₃-C₄-cycloalkyl, -O-C₁-C₂-alkyl and -O-C₁-C₂-haloalkyl; particularly preferred from hydrogen, halogen, C₁-C₂-alkyl, and C₁-C₂-haloalkyl, in particular hydrogen or methyl.

Preferably, R³ is in ortho position to the methyl oxime side chain of the molecule, which compounds are of formula I.A:

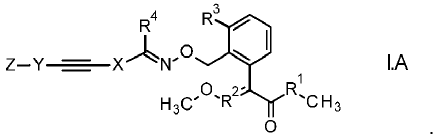

According to a further embodiment, R⁴ is selected from is selected from hydrogen, C₁-C₄-alkyl, C₂-C₄-alkenyl, C₁-C₄-haloalkyl, C₂-C₄-haloalkenyl, -(C₁-C₂-alkyl)-O-(C₁-C₂-alkyl) and -CH₂-cyclopropyl; more preferably from hydrogen, C₁-C₄-alkyl and C₁-C₄-haloalkyl, even more preferably from methyl and C₁-haloalkyl; in particular methyl.

According to a further embodiment, X is a direct bond.

According to a further embodiment, Y is a direct bond. More preferably, X and Y are direct bonds.

According to a further embodiment, at least one of X and Y is a divalent group -CL¹L²-.

According to a further embodiment, L¹ and L², independently of each other, are selected from hydrogen, C₁-C₃-alkyl, C₁-C₃-haloalkyl, cyclopropyl and -CH₂-cyclopropyl; or, L¹ and L², together with the interjacent carbon atom, form a cyclopropyl; more preferably L¹ and L², independently of each other, are selected from hydrogen and C₁-C₃-alkyl; or, L¹ and L², together with the interjacent carbon atom, form a cyclopropyl; in particular L¹ and L² are both hydrogen.

According to a further embodiment, Z is selected from C₃-C₆-cycloalkyl, phenyl and 5- or 6-membered heteroaryl, wherein said heteroaryl besides carbon atoms contain 1, 2 or 3 heteroatoms selected from N, O and S provided that such heteroaryl cannot contain 2 contiguous atoms selected from O and S, and wherein Z is unsubstituted or carries 1, 2 or 3 identical or different groups R^{a} as defined herein; more preferably Z is unsubstituted or carries 1 or 2 identical or different groups R^{a} as defined herein; in particular Z is unsubstituted or carries 1 group R^{a} as defined herein.

According to a further embodiment, Z is selected from C₃-C₆-cycloalkyl, phenyl and 5- or 6-membered heteroaryl, wherein said heteroaryl besides carbon atoms contain 1, 2 or 3 heteroatoms selected from N, O and S provided that such heteroaryl cannot contain 2 contiguous atoms selected from O and S, and wherein Z carries 1, 2 or 3 identical or different groups R^{a} as defined herein; more preferably Z carries 1 or 2 identical or different groups R^{a} as defined herein; in particular Z carries 1 group R^{a} as defined herein.

According to a further embodiment, Z is selected from cyclopropyl and phenyl, and wherein Z is unsubstituted or carries 1, 2 or 3 identical or different groups R^{a} as defined herein; more preferably Z is unsubstituted or carries 1 or 2 identical or different groups R^{a} as defined herein; in particular Z is unsubstituted or carries 1 group R^{a} as defined herein.

According to the abovementioned embodiments, R^{a} is preferably selected from halogen, CN, C₁-C₄-alkyl, C₂-C₄-alkenyl, C₂-C₄-alkynyl, -O-C₁-C₄-alkyl, -C(=N-O-C₁-C₄-alkyl)-C₁-C₄-alkyl, -C(=O)-C₁-C₄-alkyl, -O-CH₂-C(=N-O-C₁-C₄-alkyl)-C₁-C₄-alkyl, C₃-C₄-cycloalkyl, -C₁-C₂-alkyl-C₃-C₄-cycloalkyl, -O-C₃-C₄-cycloalkyl, phenyl, 3- to 5-membered heterocycloalkyl, 3- to 5-membered heterocycloalkenyl and 5- or 6-membered heteroaryl, wherein said heterocycloalkyl, heterocycloalkenyl and heteroaryl besides carbon atoms contain 1, 2 or 3 heteroatoms selected from N, O and S provided that such heterocycle cannot contain 2 contiguous atoms selected from O and S, wherein said phenyl, heterocycloalkyl, heterocycloalkenyl and heteroaryl are bound directly or via an oxygen atom or via a C₁-C₂-alkylene linker.

Preferably, R^{a} is selected from halogen, CN, C₁-C₄-alkyl, C₂-C₄-alkenyl, C₂-C₄-alkynyl, -O-C₁-C₄-alkyl, -C(=O)-C₁-C₂-alkyl, -C(=N-O-C₁-C₄-alkyl)-C₁-C₄-alkyl, C₃-C₄-cycloalkyl, -O-C₃-C₄-cycloalkyl, phenyl, 3- to 5-membered heterocycloalkyl and 5- or 6-membered heteroaryl, wherein said heterocycloalkyl and heteroaryl besides carbon atoms contain 1 or 2 heteroatoms selected from N, O and S provided that such heterocycle cannot contain 2 contiguous atoms selected from O and S, wherein said phenyl, heterocycloalkyl and heteroaryl are bound directly or via an oxygen atom or via a methylene linker.

More preferably, R^{a} is selected from halogen, CN, C₁-C₃-alkyl, -O-C₁-C₃-alkyl, -C(=N-O-CH₃)-CH₃, C₃-C₄-cycloalkyl, -O-C₃-C₄-cycloalkyl, phenyl, 3- to 5-membered heterocycloalkyl and 5- or 6-membered heteroaryl, wherein said heterocycloalkyl and heteroaryl besides carbon atoms contain 1 or 2 heteroatoms selected from N, O and S provided that such heterocycle cannot contain 2 contiguous atoms selected from O and S, wherein said phenyl, heterocycloalkyl and heteroaryl are bound directly or via an oxygen atom or via a methylene linker.

In particular, R^{a} is selected from halogen, CN, C₁-C₂-alkyl, -O-C₁-C₂-alkyl, ethenyl, ethynyl and -C(=N-O-CH₃)-CH₃.

According to the abovementioned embodiments for R^{a}, the abovementioned heterocycloalkyl is more preferably a 4-membered heterocycloalkyl, wherein said heterocycloalkyl besides carbon atoms contains 1 heteroatom selected from N, O and S, preferably N.

According to the abovementioned embodiments for R^{a}, the abovementioned heteroaryl is more preferably a 5-membered heteroaryl, wherein said heteroaryl besides carbon atoms contains 1 or 2 heteroatoms selected from N, O and S provided that such heteroaryl cannot contain 2 contiguous atoms selected from O and S, preferably the heteroatoms are selected from N and O.

According to the abovementioned embodiments for R^{a}, the aliphatic and cyclic moieties of R^{a} are unsubstituted or carry 1, 2, 3, 4 or up to the maximum number of identical or different groups R^{b} selected from halogen, CN, NO₂, C₁-C₄-alkyl, C₁-C₄-haloalkyl, -O-C₁-C₄-alkyl and -O-C₁-C₄-haloalkyl; more preferably only the cyclic moieties of R^{a} are unsubstituted or carry 1, 2, 3, 4 or up to the maximum number of identical or different groups R^{b} selected from halogen, CN, NO₂, C₁-C₄-alkyl, C₁-C₄-haloalkyl, -O-C₁-C₄-alkyl and -O-C₁-C₄-haloalkyl; even more preferably only the phenyl moiety of R^{a} is unsubstituted or carries 1, 2, 3, 4 or 5 identical or different groups R^{b} selected from halogen, CN, C₁-C₄-alkyl, C₁-C₄-haloalkyl, -O-C₁-C₄-alkyl and -O-C₁-C₄-haloalkyl; in particular said phenyl is unsubstituted or carries 1, 2 or 3 identical or different groups R^{b} selected from halogen, CN, C₁-C₂-alkyl, C₁-C₂-haloalkyl, -O-C₁-C₂-alkyl and -O-C₁-C₂-haloalkyl.

According to a further preferred embodiment, the present invention relates to compounds of formula I wherein:
- R¹: is selected from O and NH;
- R²: is N;
- R³: is hydrogen, halogen or C₁-C₄-alkyl, wherein R³ is in ortho position to the methyl oxime side chain;
- R⁴: is selected from C₁-C₆-alkyl;
- X, Y: are both direct bonds;
- Z: is selected from cyclopropyl, phenyl and 5-membered heteroaryl,
wherein said heteroaryl besides carbon atoms contains 1, 2 or 3 heteroatoms selected from N, O and S provided that such heteroaryl cannot contain 2 contiguous atoms selected from O and S,
and wherein Z is unsubstituted or carries 1, 2, 3 or up to the maximum number of identical or different groups R^{a}:
R^{a} is selected from halogen, CN, C₁-C₄-haloalkyl, C₁-C₄-alkyl, -O-C₁-C₄-alkyl, -O-C₁-C₄-haloalkyl, -C(=N-O-C₁-C₄-alkyl)-C₁-C₄-alkyl, -C(=O)-C₁-C₄-alkyl, C₃-C₄-cycloalkyl, -C₁-C₂-alkyl-C₃-C₄-Cycloalkyl, -O-C₃-C₄-cycloalkyl, phenyl, 3- to 5-membered heterocycloalkyl and 5- or 6-membered heteroaryl, wherein said heterocycloalkyl and heteroaryl besides carbon atoms contain 1 or 2 heteroatoms selected from N, O and S provided that such heterocycle cannot contain 2 contiguous atoms selected from O and S, wherein said phenyl, heterocycloalkyl and heteroaryl are bound directly or via an oxygen atom or via a methylene linker, and wherein the abovementioned cyclic moieties of R^{a} are unsubstituted or carry 1, 2 or 3 identical or different groups R^{b} selected from halogen, CN, C₁-C₂-alkyl, C₁-C₂-haloalkyl, -O-C₁-C₂-alkyl and -O-C₁-C₂-haloalkyl;
and in form or stereoisomers and tautomers thereof, and the N-oxides and the agriculturally acceptable salts thereof.

According to a further embodiment, R³ is in ortho position to the methyl oxime side chain, R¹ is NH and R² is N, which compounds are of formula I.A1:

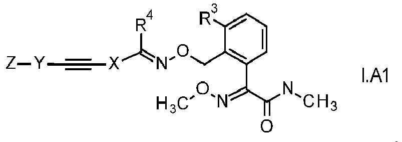

According to a further embodiment, R³ is in ortho position to the methyl oxime side chain, R¹ is O and R² is N, which compounds are of formula I.A2:

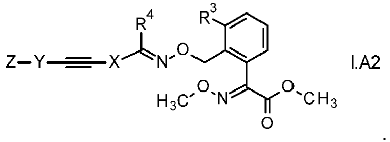

According to a further embodiment, R¹ is NH, R² is N and R³ is H, which compounds are of formula I.B1:

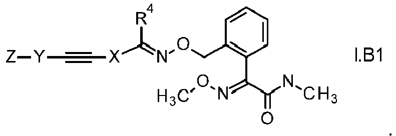

According to a further embodiment, R¹ is O, R² is N and R³ is H, which compounds are of formula I.B2:

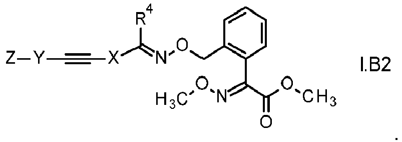

Preferably, R³ of compounds I is one of the following radicals 3-1 to 3-8:

| **No.** | **R³** |
|---|---|
| 3-1 | CH₃ |
| 3-2 | OCH₃ |
| 3-3 | CHF₂ |
| 3-4 | C₃H₅ |
| 3-5 | CH=CH₂ |
| 3-6 | CH₂CH=C(CH₃)₂ |
| 3-7 | CF₃ |
| 3-8 | C(=NOCH₃)CH₃ |

Even more preferably R³ is CH₃, OCH₃, CF₃, CHF₂ or C₃H₅, in particular CH₃.

Particularly preferred embodiments of the invention relate to compounds I, wherein the R⁴ is one of the following radicals 4-1 to 4-10:

| **No.** | **R⁴** |
|---|---|
| 4-1 | CH₃ |
| 4-2 | C₂H₅ |
| 4-3 | CH₂OCH₃ |
| 4-4 | CH₂CF₃ |
| 4-5 | CH₂C₆H₅ |
| 4-6 | CHF₂ |
| 4-7 | CH₂C₃H₅ |
| 4-8 | CH₂-C(=NOCH₃)CH₃ |
| 4-9 | C≡CH |
| 4-10 | C≡CCH₃ |

Particularly preferred embodiments of the invention relate to compounds I, wherein X and Y are direct bonds and Z is phenyl. Even more preferably, X and Y are direct bonds, Z is phenyl, R³ is in ortho position to the methyl oxime side chain, R¹ is NH and R² is N, wherein the phenyl Z is unsubstituted or substituted by 1, 2 or 3 groups R^{a} as defined herein, which compounds are of formula I.A1.1:

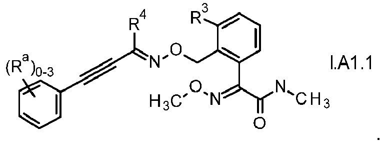

According to a further embodiment, X and Y are direct bonds, Z is phenyl, R³ is in ortho position to the methyl oxime side chain, R¹ is O and R² is N, wherein the phenyl Z is unsubstituted or substituted by 1, 2 or 3 groups R^{a} as defined herein, which compounds are of formula I.A2.1:

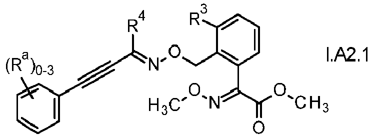

According to a further embodiment, X and Y are direct bonds, Z is phenyl, R¹ is NH, R² is N and R³ is H, wherein the phenyl Z is unsubstituted or substituted by 1, 2 or 3 groups R^{a} as defined herein, which compounds are of formula I.B1.1:

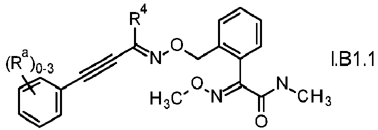

According to a further embodiment, X and Y are direct bonds, Z is phenyl, R¹ is O, R² is N and R³ is H, wherein the phenyl Z is unsubstituted or substituted by 1, 2 or 3 groups R^{a} as defined herein, which compounds are of formula I.B2.1:

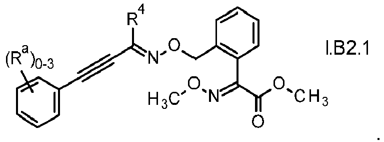

Particularly preferred embodiments of the invention relate to compounds I, wherein the R^{a} is selected of one of the following radicals a-1 to a-17:

| **No.** | **R^{a}** |
|---|---|
| a-1 | F |
| a-2 | Cl |
| a-3 | Br |
| a-4 | CH₃ |
| a-5 | CHF₂ |
| a-6 | CF₃ |
| a-7 | OCH₃ |
| a-8 | OCHF₂ |
| a-9 | OCF₃ |
| a-10 | C₂H₅ |
| a-11 | CH₂CF₃ |
| a-12 | CH=CH₂ |
| a-13 | C₆H₅ |
| a-14 | C=CH |
| a-15 | C=CCH₃ |
| a-16 | C₃H₅ |
| a-17 | C(=NOCH₃)CH₃ |

In an embodiment, compounds I are of formula I.A1.1 wherein R³ is CH₃ and R^{a} and R⁴ are as per any row of Table A below, which compounds are named I.A1.1-A-1 to I.A1.1-A-385.

In another embodiment, compounds I are of formula I.A2.1 wherein R³ is CH₃ and R^{a} and R⁴ are as per any row of Table A below, which compounds are named I.A2.1-A-1 to I.A2.1-A-385.

In another embodiment, compounds I are of formula I.B1.1 and R^{a} and R⁴ are as per any row of Table A below, which compounds are named I.B1.1-A-1 to I.B1.1-A-385.

In another embodiment, compounds I are of formula I.B2.1 and R^{a} and R⁴ are as per any row of Table A below, which compounds are named I.B2.1-A-1 to I.B2.1-A-385.

**Table A:**

| **No.** | **(R^{a})**₀₋₃ | **R⁴** |
|---|---|---|
| A-1 | - | CH₃ |
| A-2 | 2-F | CH₃ |
| A-3 | 2-Cl | CH₃ |
| A-4 | 2-Br | CH₃ |
| A-5 | 2-CH₃ | CH₃ |
| A-6 | 2-CHF₂ | CH₃ |
| A-7 | 2-CF₃ | CH₃ |
| A-8 | 2-OCH₃ | CH₃ |
| A-9 | 2-OCHF₂ | CH₃ |
| A-10 | 2-OCF₃ | CH₃ |
| A-11 | 2-C₂H₅ | CH₃ |
| A-12 | 2-CH₂CF₃ | CH₃ |
| A-13 | 2-CH=CH₂ | CH₃ |
| A-14 | 2-C₆H₅ | CH₃ |
| A-15 | 2-C≡CH | CH₃ |
| A-16 | 2-C≡CCH₃ | CH₃ |
| A-17 | 2-C₃H₅ | CH₃ |
| A-18 | 2-C(=NOCH₃)CH₃ | CH₃ |
| A-19 | 2-CN | CH₃ |
| A-20 | 3-F | CH₃ |
| A-21 | 3-Cl | CH₃ |
| A-22 | 3-Br | CH₃ |
| A-23 | 3-CH₃ | CH₃ |
| A-24 | 3-CHF₂ | CH₃ |
| A-25 | 3-CF₃ | CH₃ |
| A-26 | 3-OCH₃ | CH₃ |
| A-27 | 3-OCHF₂ | CH₃ |
| A-28 | 3-OCF₃ | CH₃ |
| A-29 | 3-C₂H₅ | CH₃ |
| A-30 | 3-CH₂CF₃ | CH₃ |
| A-31 | 3-CH=CH₂ | CH₃ |
| A-32 | 3-C₆H₅ | CH₃ |
| A-33 | 3-C≡CH | CH₃ |
| A-34 | 3-C≡CCH₃ | CH₃ |
| A-35 | 3-C₃H₅ | CH₃ |
| A-36 | 3-C(=NOCH₃)CH₃ | CH₃ |
| A-37 | 3-CN | CH₃ |
| A-38 | 4-F | CH₃ |
| A-39 | 4-Cl | CH₃ |
| A-40 | 4-Br | CH₃ |
| A-41 | 4-CH₃ | CH₃ |
| A-42 | 4-CHF₂ | CH₃ |
| A-43 | 4-CF₃ | CH₃ |
| A-44 | 4-OCH₃ | CH₃ |
| A-45 | 4-OCHF₂ | CH₃ |
| A-46 | 4-OCF₃ | CH₃ |
| A-47 | 4-C₂H₅ | CH₃ |
| A-48 | 4-CH₂CF₃ | CH₃ |
| A-49 | 4-CH=CH₂ | CH₃ |
| A-50 | 4-C₆H₅ | CH₃ |
| A-51 | 4-C≡CH | CH₃ |
| A-52 | 4-C≡CCH₃ | CH₃ |
| A-53 | 4-C₃H₅ | CH₃ |
| A-54 | 4-C(=NOCH₃)CH₃ | CH₃ |
| A-55 | 4-CN | CH₃ |
| A-56 | - | C₂H₅ |
| A-57 | 2-F | C₂H₅ |
| A-58 | 2-Cl | C₂H₅ |
| A-59 | 2-Br | C₂H₅ |
| A-60 | 2-CH₃ | C₂H₅ |
| A-61 | 2-CHF₂ | C₂H₅ |
| A-62 | 2-CF₃ | C₂H₅ |
| A-63 | 2-OCH₃ | C₂H₅ |
| A-64 | 2-OCHF₂ | C₂H₅ |
| A-65 | 2-OCF₃ | C₂H₅ |
| A-66 | 2-C₂H₅ | C₂H₅ |
| A-67 | 2-CH₂CF₃ | C₂H₅ |
| A-68 | 2-CH=CH₂ | C₂H₅ |
| A-69 | 2-C₆H₅ | C₂H₅ |
| A-70 | 2-C≡CH | C₂H₅ |
| A-71 | 2-C≡CCH₃ | C₂H₅ |
| A-72 | 2-C₃H₅ | C₂H₅ |
| A-73 | 2-C(=NOCH₃)CH₃ | C₂H₅ |
| A-74 | 2-CN | C₂H₅ |
| A-75 | 3-F | C₂H₅ |
| A-76 | 3-Cl | C₂H₅ |
| A-77 | 3-Br | C₂H₅ |
| A-78 | 3-CH₃ | C₂H₅ |
| A-79 | 3-CHF₂ | C₂H₅ |
| A-80 | 3-CF₃ | C₂H₅ |
| A-81 | 3-OCH₃ | C₂H₅ |
| A-82 | 3-OCHF₂ | C₂H₅ |
| A-83 | 3-OCF₃ | C₂H₅ |
| A-84 | 3-C₂H₅ | C₂H₅ |
| A-85 | 3-CH₂CF₃ | C₂H₅ |
| A-86 | 3-CH=CH₂ | C₂H₅ |
| A-87 | 3-C₆H₅ | C₂H₅ |
| A-88 | 3-C≡CH | C₂H₅ |
| A-89 | 3-C≡CCH₃ | C₂H₅ |
| A-90 | 3-C₃H₅ | C₂H₅ |
| A-91 | 3-C(=NOCH₃)CH₃ | C₂H₅ |
| A-92 | 3-CN | C₂H₅ |
| A-93 | 4-F | C₂H₅ |
| A-94 | 4-Cl | C₂H₅ |
| A-95 | 4-Br | C₂H₅ |
| A-96 | 4-CH₃ | C₂H₅ |
| A-97 | 4-CHF₂ | C₂H₅ |
| A-98 | 4-CF₃ | C₂H₅ |
| A-99 | 4-OCH₃ | C₂H₅ |
| A-100 | 4-OCHF₂ | C₂H₅ |
| A-101 | 4-OCF₃ | C₂H₅ |
| A-102 | 4-C₂H₅ | C₂H₅ |
| A-103 | 4-CH₂CF₃ | C₂H₅ |
| A-104 | 4-CH=CH₂ | C₂H₅ |
| A-105 | 4-C₆H₅ | C₂H₅ |
| A-106 | 4-C≡CH | C₂H₅ |
| A-107 | 4-C≡CCH₃ | C₂H₅ |
| A-108 | 4-C₃H₅ | C₂H₅ |
| A-109 | 4-C(=NOCH₃)CH₃ | C₂H₅ |
| A-110 | 4-CN | C₂H₅ |
| A-111 | - | CH₂CF₃ |
| A-112 | 2-F | CH₂CF₃ |
| A-113 | 2-Cl | CH₂CF₃ |
| A-114 | 2-Br | CH₂CF₃ |
| A-115 | 2-CH₃ | CH₂CF₃ |
| A-116 | 2-CHF₂ | CH₂CF₃ |
| A-117 | 2-CF₃ | CH₂CF₃ |
| A-118 | 2-OCH₃ | CH₂CF₃ |
| A-119 | 2-OCHF₂ | CH₂CF₃ |
| A-120 | 2-OCF₃ | CH₂CF₃ |
| A-121 | 2-C₂H₅ | CH₂CF₃ |
| A-122 | 2-CH₂CF₃ | CH₂CF₃ |
| A-123 | 2-CH=CH₂ | CH₂CF₃ |
| A-124 | 2-C₆H₅ | CH₂CF₃ |
| A-125 | 2-C≡CH | CH₂CF₃ |
| A-126 | 2-C≡CCH₃ | CH₂CF₃ |
| A-127 | 2-C₃H₅ | CH₂CF₃ |
| A-128 | 2-C(=NOCH₃)CH₃ | CH₂CF₃ |
| A-129 | 2-CN | CH₂CF₃ |
| A-130 | 3-F | CH₂CF₃ |
| A-131 | 3-Cl | CH₂CF₃ |
| A-132 | 3-Br | CH₂CF₃ |
| A-133 | 3-CH₃ | CH₂CF₃ |
| A-134 | 3-CHF₂ | CH₂CF₃ |
| A-135 | 3-CF₃ | CH₂CF₃ |
| A-136 | 3-OCH₃ | CH₂CF₃ |
| A-137 | 3-OCHF₂ | CH₂CF₃ |
| A-138 | 3-OCF₃ | CH₂CF₃ |
| A-139 | 3-C₂H₅ | CH₂CF₃ |
| A-140 | 3-CH₂CF₃ | CH₂CF₃ |
| A-141 | 3-CH=CH₂ | CH₂CF₃ |
| A-142 | 3-C₆H₅ | CH₂CF₃ |
| A-143 | 3-C≡CH | CH₂CF₃ |
| A-144 | 3-C≡CCH₃ | CH₂CF₃ |
| A-145 | 3-C₃H₅ | CH₂CF₃ |
| A-146 | 3-C(=NOCH₃)CH₃ | CH₂CF₃ |
| A-147 | 3-CN | CH₂CF₃ |
| A-148 | 4-F | CH₂CF₃ |
| A-149 | 4-Cl | CH₂CF₃ |
| A-150 | 4-Br | CH₂CF₃ |
| A-151 | 4-CH₃ | CH₂CF₃ |
| A-152 | 4-CHF₂ | CH₂CF₃ |
| A-153 | 4-CF₃ | CH₂CF₃ |
| A-154 | 4-OCH₃ | CH₂CF₃ |
| A-155 | 4-OCHF₂ | CH₂CF₃ |
| A-156 | 4-OCF₃ | CH₂CF₃ |
| A-157 | 4-C₂H₅ | CH₂CF₃ |
| A-158 | 4-CH₂CF₃ | CH₂CF₃ |
| A-159 | 4-CH=CH₂ | CH₂CF₃ |
| A-160 | 4-C₆H₅ | CH₂CF₃ |
| A-161 | 4-C≡CH | CH₂CF₃ |
| A-162 | 4-C≡CCH₃ | CH₂CF₃ |
| A-163 | 4-C₃H₅ | CH₂CF₃ |
| A-164 | 4-C(=NOCH₃)CH₃ | CH₂CF₃ |
| A-165 | 4-CN | CH₂CF₃ |
| A-166 | - | CH₂OCH₃ |
| A-167 | 2-F | CH₂OCH₃ |
| A-168 | 2-Cl | CH₂OCH₃ |
| A-169 | 2-Br | CH₂OCH₃ |
| A-170 | 2-CH₃ | CH₂OCH₃ |
| A-171 | 2-CHF₂ | CH₂OCH₃ |
| A-172 | 2-CF₃ | CH₂OCH₃ |
| A-173 | 2-OCH₃ | CH₂OCH₃ |
| A-174 | 2-OCHF₂ | CH₂OCH₃ |
| A-175 | 2-OCF₃ | CH₂OCH₃ |
| A-176 | 2-C₂H₅ | CH₂OCH₃ |
| A-177 | 2-CH₂CF₃ | CH₂OCH₃ |
| A-178 | 2-CH=CH₂ | CH₂OCH₃ |
| A-179 | 2-C₆H₅ | CH₂OCH₃ |
| A-180 | 2-C≡CH | CH₂OCH₃ |
| A-181 | 2-C≡CCH₃ | CH₂OCH₃ |
| A-182 | 2-C₃H₅ | CH₂OCH₃ |
| A-183 | 2-C(=NOCH₃)CH₃ | CH₂OCH₃ |
| A-184 | 2-CN | CH₂OCH₃ |
| A-185 | 3-F | CH₂OCH₃ |
| A-186 | 3-Cl | CH₂OCH₃ |
| A-187 | 3-Br | CH₂OCH₃ |
| A-188 | 3-CH₃ | CH₂OCH₃ |
| A-189 | 3-CHF₂ | CH₂OCH₃ |
| A-190 | 3-CF₃ | CH₂OCH₃ |
| A-191 | 3-OCH₃ | CH₂OCH₃ |
| A-192 | 3-OCHF₂ | CH₂OCH₃ |
| A-193 | 3-OCF₃ | CH₂OCH₃ |
| A-194 | 3-C₂H₅ | CH₂OCH₃ |
| A-195 | 3-CH₂CF₃ | CH₂OCH₃ |
| A-196 | 3-CH=CH₂ | CH₂OCH₃ |
| A-197 | 3-C₆H₅ | CH₂OCH₃ |
| A-198 | 3-C=CH | CH₂OCH₃ |
| A-199 | 3-C≡CCH₃ | CH₂OCH₃ |
| A-200 | 3-C₃H₅ | CH₂OCH₃ |
| A-201 | 3-C(=NOCH₃)CH₃ | CH₂OCH₃ |
| A-202 | 3-CN | CH₂OCH₃ |
| A-203 | 4-F | CH₂OCH₃ |
| A-204 | 4-Cl | CH₂OCH₃ |
| A-205 | 4-Br | CH₂OCH₃ |
| A-206 | 4-CH₃ | CH₂OCH₃ |
| A-207 | 4-CHF₂ | CH₂OCH₃ |
| A-208 | 4-CF₃ | CH₂OCH₃ |
| A-209 | 4-OCH₃ | CH₂OCH₃ |
| A-210 | 4-OCHF₂ | CH₂OCH₃ |
| A-211 | 4-OCF₃ | CH₂OCH₃ |
| A-212 | 4-C₂H₅ | CH₂OCH₃ |
| A-213 | 4-CH₂CF₃ | CH₂OCH₃ |
| A-214 | 4-CH=CH₂ | CH₂OCH₃ |
| A-215 | 4-C₆H₅ | CH₂OCH₃ |
| A-216 | 4-C≡CH | CH₂OCH₃ |
| A-217 | 4-C≡CCH₃ | CH₂OCH₃ |
| A-218 | 4-C₃H₅ | CH₂OCH₃ |
| A-219 | 4-C(=NOCH₃)CH₃ | CH₂OCH₃ |
| A-220 | 4-CN | CH₂OCH₃ |
| A-221 | - | CH₂-C(=NOCH₃)CH₃ |
| A-222 | 2-F | CH₂-C(=NOCH₃)CH₃ |
| A-223 | 2-Cl | CH₂-C(=NOCH₃)CH₃ |
| A-224 | 2-Br | CH₂-C(=NOCH₃)CH₃ |
| A-225 | 2-CH₃ | CH₂-C(=NOCH₃)CH₃ |
| A-226 | 2-CHF₂ | CH₂-C(=NOCH₃)CH₃ |
| A-227 | 2-CF₃ | CH₂-C(=NOCH₃)CH₃ |
| A-228 | 2-OCH₃ | CH₂-C(=NOCH₃)CH₃ |
| A-229 | 2-OCHF₂ | CH₂-C(=NOCH₃)CH₃ |
| A-230 | 2-OCF₃ | CH₂-C(=NOCH₃)CH₃ |
| A-231 | 2-C₂H₅ | CH₂-C(=NOCH₃)CH₃ |
| A-232 | 2-CH₂CF₃ | CH₂-C(=NOCH₃)CH₃ |
| A-233 | 2-CH=CH₂ | CH₂-C(=NOCH₃)CH₃ |
| A-234 | 2-C₆H₅ | CH₂-C(=NOCH₃)CH₃ |
| A-235 | 2-C≡CH | CH₂-C(=NOCH₃)CH₃ |
| A-236 | 2-C≡CCH₃ | CH₂-C(=NOCH₃)CH₃ |
| A-237 | 2-C₃H₅ | CH₂-C(=NOCH₃)CH₃ |
| A-238 | 2-C(=NOCH₃)CH₃ | CH₂-C(=NOCH₃)CH₃ |
| A-239 | 2-CN | CH₂-C(=NOCH₃)CH₃ |
| A-240 | 3-F | CH₂-C(=NOCH₃)CH₃ |
| A-241 | 3-Cl | CH₂-C(=NOCH₃)CH₃ |
| A-242 | 3-Br | CH₂-C(=NOCH₃)CH₃ |
| A-243 | 3-CH₃ | CH₂-C(=NOCH₃)CH₃ |
| A-244 | 3-CHF₂ | CH₂-C(=NOCH₃)CH₃ |
| A-245 | 3-CF₃ | CH₂-C(=NOCH₃)CH₃ |
| A-246 | 3-OCH₃ | CH₂-C(=NOCH₃)CH₃ |
| A-247 | 3-OCHF₂ | CH₂-C(=NOCH₃)CH₃ |
| A-248 | 3-OCF₃ | CH₂-C(=NOCH₃)CH₃ |
| A-249 | 3-C₂H₅ | CH₂-C(=NOCH₃)CH₃ |
| A-250 | 3-CH₂CF₃ | CH₂-C(=NOCH₃)CH₃ |
| A-251 | 3-CH=CH₂ | CH₂-C(=NOCH₃)CH₃ |
| A-252 | 3-C₆H₅ | CH₂-C(=NOCH₃)CH₃ |
| A-253 | 3-C≡CH | CH₂-C(=NOCH₃)CH₃ |
| A-254 | 3-C≡CCH₃ | CH₂-C(=NOCH₃)CH₃ |
| A-255 | 3-C₃H₅ | CH₂-C(=NOCH₃)CH₃ |
| A-256 | 3-C(=NOCH₃)CH₃ | CH₂-C(=NOCH₃)CH₃ |
| A-257 | 3-CN | CH₂-C(=NOCH₃)CH₃ |
| A-258 | 4-F | CH₂-C(=NOCH₃)CH₃ |
| A-259 | 4-Cl | CH₂-C(=NOCH₃)CH₃ |
| A-260 | 4-Br | CH₂-C(=NOCH₃)CH₃ |
| A-261 | 4-CH₃ | CH₂-C(=NOCH₃)CH₃ |
| A-262 | 4-CHF₂ | CH₂-C(=NOCH₃)CH₃ |
| A-263 | 4-CF₃ | CH₂-C(=NOCH₃)CH₃ |
| A-264 | 4-OCH₃ | CH₂-C(=NOCH₃)CH₃ |
| A-265 | 4-OCHF₂ | CH₂-C(=NOCH₃)CH₃ |
| A-266 | 4-OCF₃ | CH₂-C(=NOCH₃)CH₃ |
| A-267 | 4-C₂H₅ | CH₂-C(=NOCH₃)CH₃ |
| A-268 | 4-CH₂CF₃ | CH₂-C(=NOCH₃)CH₃ |
| A-269 | 4-CH=CH₂ | CH₂-C(=NOCH₃)CH₃ |
| A-270 | 4-C₆H₅ | CH₂-C(=NOCH₃)CH₃ |
| A-271 | 4-C≡CH | CH₂-C(=NOCH₃)CH₃ |
| A-272 | 4-C≡CCH₃ | CH₂-C(=NOCH₃)CH₃ |
| A-273 | 4-C₃H₅ | CH₂-C(=NOCH₃)CH₃ |
| A-274 | 4-C(=NOCH₃)CH₃ | CH₂-C(=NOCH₃)CH₃ |
| A-275 | 4-CN | CH₂-C(=NOCH₃)CH₃ |
| A-276 | - | CHF₂ |
| A-277 | 2-F | CHF₂ |
| A-278 | 2-Cl | CHF₂ |
| A-279 | 2-Br | CHF₂ |
| A-280 | 2-CH₃ | CHF₂ |
| A-281 | 2-CHF₂ | CHF₂ |
| A-282 | 2-CF₃ | CHF₂ |
| A-283 | 2-OCH₃ | CHF₂ |
| A-284 | 2-OCHF₂ | CHF₂ |
| A-285 | 2-OCF₃ | CHF₂ |
| A-286 | 2-C₂H₅ | CHF₂ |
| A-287 | 2-CH₂CF₃ | CHF₂ |
| A-288 | 2-CH=CH₂ | CHF₂ |
| A-289 | 2-C₆H₅ | CHF₂ |
| A-290 | 2-C≡CH | CHF₂ |
| A-291 | 2-C≡CCH₃ | CHF₂ |
| A-292 | 2-C₃H₅ | CHF₂ |
| A-293 | 2-C(=NOCH₃)CH₃ | CHF₂ |
| A-294 | 2-CN | CHF₂ |
| A-295 | 3-F | CHF₂ |
| A-296 | 3-CI | CHF₂ |
| A-297 | 3-Br | CHF₂ |
| A-298 | 3-CH₃ | CHF₂ |
| A-299 | 3-CHF₂ | CHF₂ |
| A-300 | 3-CF₃ | CHF₂ |
| A-301 | 3-OCH₃ | CHF₂ |
| A-302 | 3-OCHF₂ | CHF₂ |
| A-303 | 3-OCF₃ | CHF₂ |
| A-304 | 3-C₂H₅ | CHF₂ |
| A-305 | 3-CH₂CF₃ | CHF₂ |
| A-306 | 3-CH=CH₂ | CHF₂ |
| A-307 | 3-C₆H₅ | CHF₂ |
| A-308 | 3-C≡CH | CHF₂ |
| A-309 | 3-C≡CCH₃ | CHF₂ |
| A-310 | 3-C₃H₅ | CHF₂ |
| A-311 | 3-C(=NOCH₃)CH₃ | CHF₂ |
| A-312 | 3-CN | CHF₂ |
| A-313 | 4-F | CHF₂ |
| A-314 | 4-Cl | CHF₂ |
| A-315 | 4-Br | CHF₂ |
| A-316 | 4-CH₃ | CHF₂ |
| A-317 | 4-CHF₂ | CHF₂ |
| A-318 | 4-CF₃ | CHF₂ |
| A-319 | 4-OCH₃ | CHF₂ |
| A-320 | 4-OCHF₂ | CHF₂ |
| A-321 | 4-OCF₃ | CHF₂ |
| A-322 | 4-C₂H₅ | CHF₂ |
| A-323 | 4-CH₂CF₃ | CHF₂ |
| A-324 | 4-CH=CH₂ | CHF₂ |
| A-325 | 4-C₆H₅ | CHF₂ |
| A-326 | 4-C≡CH | CHF₂ |
| A-327 | 4-C≡CCH₃ | CHF₂ |
| A-328 | 4-C₃H₅ | CHF₂ |
| A-329 | 4-C(=NOCH₃)CH₃ | CHF₂ |
| A-330 | 4-CN | CHF₂ |
| A-331 | - | CH₂C₃H₅ |
| A-332 | 2-F | CH₂C₃H₅ |
| A-333 | 2-Cl | CH₂C₃H₅ |
| A-334 | 2-Br | CH₂C₃H₅ |
| A-335 | 2-CH₃ | CH₂C₃H₅ |
| A-336 | 2-CHF₂ | CH₂C₃H₅ |
| A-337 | 2-CF₃ | CH₂C₃H₅ |
| A-338 | 2-OCH₃ | CH₂C₃H₅ |
| A-339 | 2-OCHF₂ | CH₂C₃H₅ |
| A-340 | 2-OCF₃ | CH₂C₃H₅ |
| A-341 | 2-C₂H₅ | CH₂C₃H₅ |
| A-342 | 2-CH₂CF₃ | CH₂C₃H₅ |
| A-343 | 2-CH=CH₂ | CH₂C₃H₅ |
| A-344 | 2-C₆H₅ | CH₂C₃H₅ |
| A-345 | 2-C≡CH | CH₂C₃H₅ |
| A-346 | 2-C≡CCH₃ | CH₂C₃H₅ |
| A-347 | 2-C₃H₅ | CH₂C₃H₅ |
| A-348 | 2-C(=NOCH₃)CH₃ | CH₂C₃H₅ |
| A-349 | 2-CN | CH₂C₃H₅ |
| A-350 | 3-F | CH₂C₃H₅ |
| A-351 | 3-Cl | CH₂C₃H₅ |
| A-352 | 3-Br | CH₂C₃H₅ |
| A-353 | 3-CH₃ | CH₂C₃H₅ |
| A-354 | 3-CHF₂ | CH₂C₃H₅ |
| A-355 | 3-CF₃ | CH₂C₃H₅ |
| A-356 | 3-OCH₃ | CH₂C₃H₅ |
| A-357 | 3-OCHF₂ | CH₂C₃H₅ |
| A-358 | 3-OCF₃ | CH₂C₃H₅ |
| A-359 | 3-C₂H₅ | CH₂C₃H₅ |
| A-360 | 3-CH₂CF₃ | CH₂C₃H₅ |
| A-361 | 3-CH=CH₂ | CH₂C₃H₅ |
| A-362 | 3-C₆H₅ | CH₂C₃H₅ |
| A-363 | 3-C=CH | CH₂C₃H₅ |
| A-364 | 3-C=CCH₃ | CH₂C₃H₅ |
| A-365 | 3-C₃H₅ | CH₂C₃H₅ |
| A-366 | 3-C(=NOCH₃)CH₃ | CH₂C₃H₅ |
| A-367 | 3-CN | CH₂C₃H₅ |
| A-368 | 4-F | CH₂C₃H₅ |
| A-369 | 4-Cl | CH₂C₃H₅ |
| A-370 | 4-Br | CH₂C₃H₅ |
| A-371 | 4-CH₃ | CH₂C₃H₅ |
| A-372 | 4-CHF₂ | CH₂C₃H₅ |
| A-373 | 4-CF₃ | CH₂C₃H₅ |
| A-374 | 4-OCH₃ | CH₂C₃H₅ |
| A-375 | 4-OCHF₂ | CH₂C₃H₅ |
| A-376 | 4-OCF₃ | CH₂C₃H₅ |
| A-377 | 4-C₂H₅ | CH₂C₃H₅ |
| A-378 | 4-CH₂CF₃ | CH₂C₃H₅ |
| A-379 | 4-CH=CH₂ | CH₂C₃H₅ |
| A-380 | 4-C₆H₅ | CH₂C₃H₅ |
| A-381 | 4-C=CH | CH₂C₃H₅ |
| A-382 | 4-C=CCH₃ | CH₂C₃H₅ |
| A-383 | 4-C₃H₅ | CH₂C₃H₅ |
| A-384 | 4-C(=NOCH₃)CH₃ | CH₂C₃H₅ |
| A-385 | 4-CN | CH₂C₃H₅ |

The compounds can be obtained by various routes in analogy to prior art processes known (e.g EP 463488) and, advantageously, by the synthesis shown in the following Schemes 1 to 4 and in the experimental part of this application.

A suitable method to prepare compounds I is illustrated in Scheme 1. 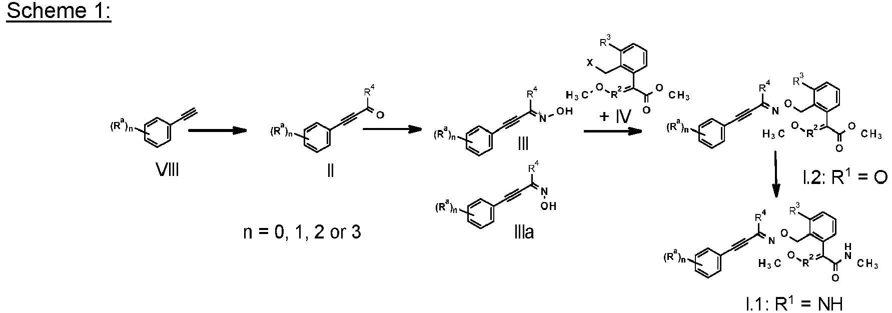 It starts with the formation of a ketone II from the corresponding acetylene compound VIII. Acetylenic proton is first abstracted by a base such as n-butyl lithium in a solvent such as tetrahydrofuran (THF) or 2-methyl-THF at reaction temperatures of -78 °C followed by quenching it with an electrophile such as ethyl acetate in the presence of BF₃-diethyl etherate at -78 °C. The conversion of the ketone II to the corresponding oxime is performed using hydxroxylamine hydrochloride and a base such as pyridine or sodium acetate in polar solvents such as methanol-water mixture at reaction temperatures of about 10 to 25 °C for 2 to 4 h, preferably at about 15 °C. The reaction provides a mixture of *E*-oxime III and *Z*-oxime IIIa. The *E*/*Z* oxime mixture (III and IIIa) is subjected to a reaction with the intermediate IV, wherein X is a leaving group such as halogen, toluene-methanesulfonates, preferably X is Cl or Br, under basic conditions using bases such as sodium hydride, cesium carbonate or potassium carbonate and using an organic solvent such as dimethyl formamide (DMF) or acetonitrile, preferably cesium carbonate as base and acetonitrile (AcN) as solvent at room temperature (RT) of about 24 °C for 12h. The resulting ester compound I.2 wherein R¹ is O can be converted to the amide of formula I.1 wherein R¹ is NH by reaction with methyl amine (preferably 40% aq. solution) using THF as solvent at RT.

A general method for preparation of intermediate IV is shown in Scheme 2. 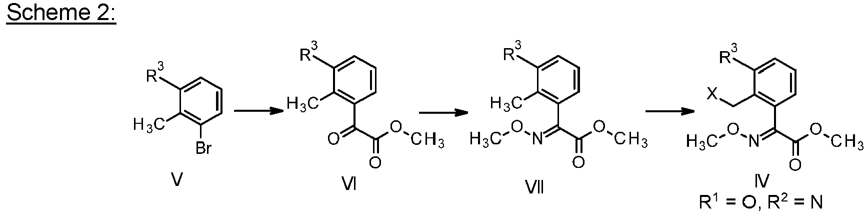 Compound VI can be obtained from compound V by lithium-halogen exchange or by generating Grignard reagent and further reaction with dimethyl oxalate or chloromethyl oxalate in presence of a solvent. The preferred solvent is THF or 2-methyl-THF and the temperature can be between -70 and -78 °C. Conversion of intermediate VI to intermediate VII can be achieved using N-methylhydroxylamine hydrochloride and a base such as pyridine or sodium acetate in polar solvents such as methanol. The reaction temperature is preferably about 65 °C. An *E*/*Z* mixture is usually obtained These isomers can be separated by purification techniques known in art (e.g. column chromatography, crystallization). Bromination of intermediate VII provides the desired intermediate compounds IV, wherein R¹ is O and R² = N and X is Br. This reaction of intermediate VII with N-bromosuccinimide is carried out in solvents such as carbon tetrachloride, chlorobenzene and acetonitrile, using radical initiators such as 1,1'-azobis(cyclo-hexanecarbonitrile) or azobisisobutyronitrile at temperatures of about 70 to 100 °C. The preferred radical initiator is 1,1'-azobis(cyclohexanecarbonitrile), preferred solvent chlorobenzene and preferred temperature 80 °C.

The synthesis of compounds containing different substituents R³ follows similar sequence as in Scheme 2, wherein R³ is bromo. Coupling of intermediate III with intermediate IV, wherein R³ is bromo, provides compounds I as described above. Using standard chemical reactions, such as Suzuki or Stille reaction, the bromo group can be converted e.g. to other R³ substituents such as cycloalkyl, alkoxy and alkenyl. Additional transformations e.g. of ethenyl provide compounds I with other R³ substituents such as ethyl, CN and haloalkyl.

Preparation of ketones II can also be carried out using other known methods (Scheme 3). 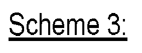 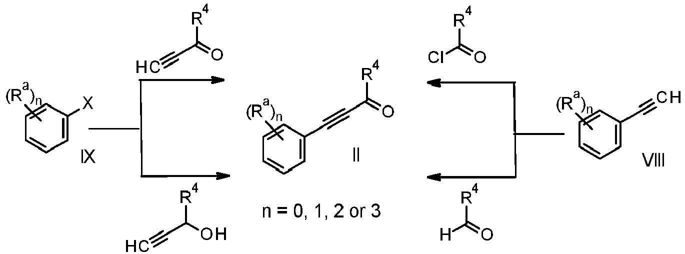 Palladium and/or copper-catalyzed cross couplings of terminal acetylenic compounds VIII with acid chlorides are usually applied (J. Org. Chem. 2004, 69, 1615). Another possibility is by formation of base-catalyzed alumination of terminal alkynes VIII followed by reaction of it with acid chlorides (J. Org. Chem. 2005, 70, 6126-6128). Alternatively, ketones II can be obtained from acetylene VIII by abstraction of acetylenic proton followed by quenching it with an aldehyde and subsequent oxidation of alcohol (Org. Biomol. Chem. 2018, 16, 6659). Ketone II can also be obtained from precursors IX, wherein X is halogen preferably iodine b palladium and copper-catalyzed cross couplings of aryl halide IX with terminal alkynones (Chem. Cat. Chem. 2015, 7, 3266) or with alkynyl alcohol followed by oxidation of alcohol (Adv. Synth. Cat. 2017, 22, 4062).

Compounds VIII are generally commercially available, otherwise accessible using methods known in prior art (Org. Lett. 2019, 21, 3990).

Another general method to prepare the compounds I is depicted in Scheme 4. 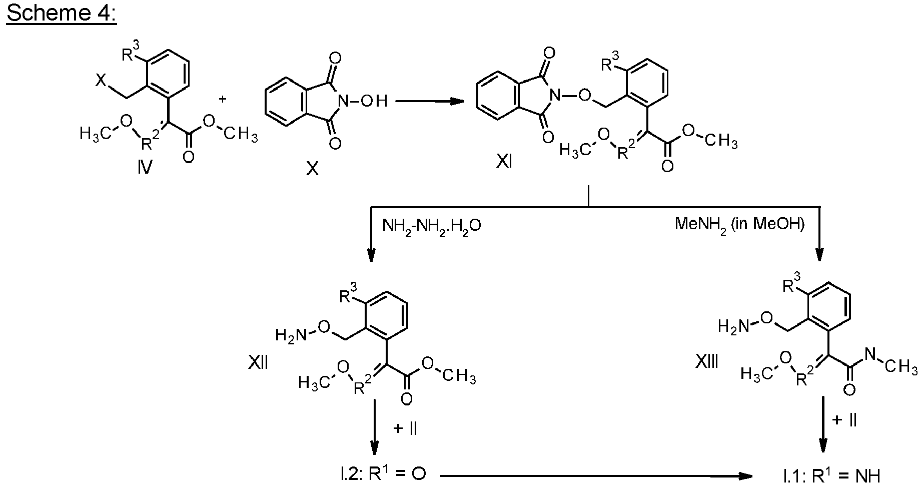 Intermediate IV is reacted with *N*-hydroxysuccimide X, using a base such as triethylamine in DMF. The reaction temperature is usually 50 to 70 °C preferably about 70 °C. Conversion to the corresponding *O*-benzylhydroxyl amine XII, is achieved through removal of the phthalimide group, preferably using hydrazine hydrate in methanol as solvent at about 25 °C. Alternatively, removal of the phthalimide group using methyl amine in methanol as solvent at about 25 °C can provide intermediate XIII. Intermediate XII and XIII, respectively can be condensed with ketones II using acetic acid or pyridine in methanol as solvent at about 50 to 65 °C. Alternatively, the condensation can also be carried out with titanium (IV) ethoxide (Ti(OEt)₄) using THF as solvent at about 70 °C. The desired product is usually accompanied by an undesired isomer, which can be removed e.g by column chromatography, crystallization.

The Schemes 1 to 4 presented above describe the synthesis of compounds I wherein R³ is in ortho position to the methyl oxime side chain but can also be applied likewise to corresponding compounds wherein R³ is at different position of the phenyl ring.

The compounds I and the compositions thereof, respectively, are suitable as fungicides effective against a broad spectrum of phytopathogenic fungi, including soil-borne fungi, in particular from the classes of Plasmodiophoromycetes, Peronosporomycetes (syn. Oomycetes), Chytridiomycetes, Zygomycetes, Ascomycetes, Basidiomycetes, and Deuteromycetes (syn. Fungi imperfecti). They can be used in crop protection as foliar fungicides, fungicides for seed dressing, and soil fungicides.

The compounds I and the compositions thereof are preferably useful in the control of phytopathogenic fungi on various cultivated plants, such as cereals, e. g. wheat, rye, barley, triticale, oats, or rice; beet, e. g. sugar beet or fodder beet; fruits, e. g. pomes (apples, pears, etc.), stone fruits (e.g. plums, peaches, almonds, cherries), or soft fruits, also called berries (strawberries, raspberries, blackberries, gooseberries, etc.); leguminous plants, e. g. lentils, peas, alfalfa, or soybeans; oil plants, e. g. oilseed rape, mustard, olives, sunflowers, coconut, cocoa beans, castor oil plants, oil palms, ground nuts, or soybeans; cucurbits, e. g. squashes, cucumber, or melons; fiber plants, e. g. cotton, flax, hemp, or jute; citrus fruits, e. g. oranges, lemons, grapefruits, or mandarins; vegetables, e. g. spinach, lettuce, asparagus, cabbages, carrots, onions, tomatoes, potatoes, cucurbits, or paprika; lauraceous plants, e. g. avocados, cinnamon, or camphor; energy and raw material plants, e. g. corn, soybean, oilseed rape, sugar cane, or oil palm; corn; tobacco; nuts; coffee; tea; bananas; vines (table grapes and grape juice grape vines); hop; turf; sweet leaf (also called Stevia); natural rubber plants; or ornamental and forestry plants, e. g. flowers, shrubs, broad-leaved trees, or evergreens (conifers, eucalypts, etc.); on the plant propagation material, such as seeds; and on the crop material of these plants.

More preferably, compounds I and compositions thereof, respectively are used for controlling fungi on field crops, such as potatoes, sugar beets, tobacco, wheat, rye, barley, oats, rice, corn, cotton, soybeans, oilseed rape, legumes, sunflowers, coffee or sugar cane; fruits; vines; ornamentals; or vegetables, such as cucumbers, tomatoes, beans or squashes.

The term "plant propagation material" is to be understood to denote all the generative parts of the plant, such as seeds; and vegetative plant materials, such as cuttings and tubers (e. g. potatoes), which can be used for the multiplication of the plant. This includes seeds, roots, fruits, tubers, bulbs, rhizomes, shoots, sprouts and other parts of plants; including seedlings and young plants to be transplanted after germination or after emergence from soil.

Preferably, treatment of plant propagation materials with compounds I and compositions thereof, respectively, is used for controlling fungi on cereals, such as wheat, rye, barley and oats; rice, corn, cotton and soybeans.

The term "cultivated plants" is to be understood as including plants which have been modified by mutagenesis or genetic engineering to provide a new trait to a plant or to modify an already present trait. Mutagenesis includes random mutagenesis using X-rays or mutagenic chemicals, but also targeted mutagenesis to create mutations at a specific locus of a plant genome. Targeted mutagenesis frequently uses oligonucleotides or proteins like CRISPR/Cas, zinc-finger nucleases, TALENs or meganucleases. Genetic engineering usually uses recombinant DNA techniques to create modifications in a genome which under natural circumstances cannot readily be obtained by cross breeding, mutagenesis or natural recombination. Typically, one or more genes are integrated into the genome of a plant to add a trait or improve or modify a trait. These integrated genes are also referred to as transgenes, while plant comprising such transgenes are referred to as transgenic plants. The process of plant transformation usually produces several transformation events, which differ in the genomic locus in which a transgene has been integrated. Plants comprising a specific transgene on a specific genomic locus are usually described as comprising a specific "event", which is referred to by a specific event name. Traits which have been introduced in plants or have been modified include herbicide tolerance, insect resistance, increased yield and tolerance to abiotic conditions, like drought. Herbicide tolerance has been created by using mutagenesis and genetic engineering. Plants which have been rendered tolerant to acetolactate synthase (ALS) inhibitor herbicides by mutagenesis and breeding are e.g. available under the name Clearfield^{®}. Herbicide tolerance to glyphosate, glufosinate, 2,4-D, dicamba, oxynil herbicides, like bromoxynil and ioxynil, sulfonylurea herbicides, ALS inhibitors and 4-hydroxyphenylpyruvate dioxygenase (HPPD) inhibitors, like isoxaflutole and mesotrione, has been created via the use of transgenes.

Transgenes to provide herbicide tolerance traits comprise: for tolerance to glyphosate: cp4 epsps, epsps grg23ace5, mepsps, 2mepsps, gat4601, gat4621, goxv247; for tolerance to glufosinate: pat and bar, for tolerance to 2,4-D: aad-1, aad-12; for tolerance to dicamba: dmo; for tolerance to oxynil herbicies: bxn; for tolerance to sulfonylurea herbicides: zm-hra, csr1-2, gm-hra, S4-HrA; for tolerance to ALS inhibitors: csr1-2; and for tolerance to HPPD inhibitors: hppdPF, W336, avhppd-03.

Transgenic corn events comprising herbicide tolerance genes include, but are not limited to, DAS40278, MON801, MON802, MON809, MON810, MON832, MON87411, MON87419, MON87427, MON88017, MON89034, NK603, GA21, MZHG0JG, HCEM485, VCO-Ø1981-5, 676, 678, 680, 33121, 4114, 59122, 98140, Bt10, Bt176, CBH-351, DBT418, DLL25, MS3, MS6, MZIR098, T25, TC1507 and TC6275. Transgenic soybean events comprising herbicide tolerance genes include, but are not limited to, GTS 40-3-2, MON87705, MON87708, MON87712, MON87769, MON89788, A2704-12, A2704-21, A5547-127, A5547-35, DP356043, DAS44406-6, DAS68416-4, DAS-81419-2, GU262, SYHTØH2, W62, W98, FG72 and CV127. Transgenic cotton events comprising herbicide tolerance genes include, but are not limited to, 19-51 a, 31707, 42317, 81910, 281-24-236, 3006-210-23, BXN10211, BXN10215, BXN10222, BXN10224, MON1445, MON1698, MON88701, MON88913, GHB119, GHB614, LLCotton25, T303-3 and T304-40. Transgenic canola events comprising herbicide tolerance genes are e.g., but not excluding others, MON88302, HCR-1, HCN10, HCN28, HCN92, MS1, MS8, PHY14, PHY23, PHY35, PHY36, RF1, RF2 and RF3.

Transgenes to provide insect resistance preferably are toxin genes of *Bacillus* spp. and synthetic variants thereof, like cry1A, cry1Ab, cry1Ab-Ac, cry1Ac, cry1A.105, cry1F, cry1Fa2, cry2Ab2, cry2Ae, mcry3A, ecry3.1Ab, cry3Bb1, cry34Ab1, cry35Ab1, cry9C, vip3A(a), vip3Aa20. In addition, transgenes of plant origin, such as genes coding for protease inhibitors, like CpTI and pinll, can be used. A further approach uses transgenes such as dvsnf7 to produce double-stranded RNA in plants.

Transgenic corn events comprising genes for insecticidal proteins or double stranded RNA include, but are not limited to, Bt10, Bt11, Bt176, MON801, MON802, MON809, MON810, MON863, MON87411, MON88017, MON89034, 33121, 4114, 5307, 59122, TC1507, TC6275, CBH-351, MIR162, DBT418 and MZIR098. Transgenic soybean events comprising genes for insecticidal proteins include, but are not limited to, MON87701, MON87751 and DAS-81419. Transgenic cotton events comprising genes for insecticidal proteins include, but are not limited to, SGK321, MON531, MON757, MON1076, MON15985, 31707, 31803, 31807, 31808, 42317, BNLA-601, Event1, COT67B, COT102, T303-3, T304-40, GFM Cry1A, GK12, MLS 9124, 281-24-236, 3006-210-23, GHB119 and SGK321.

Cultivated plants with increased yield have been created by using the transgene athb17 (e.g. corn event MON87403), or bbx32 (e.g. soybean event MON87712).

Cultivated plants comprising a modified oil content have been created by using the transgenes: gm-fad2-1, Pj.D6D, Nc.Fad3, fad2-1A and fatb1-A (e.g. soybean events 260-05, MON87705 and MON87769).

Tolerance to abiotic conditions, such as drought, has been created by using the transgene cspB (corn event MON87460) and Hahb-4 (soybean event IND-ØØ41Ø-5).

Traits are frequently combined by combining genes in a transformation event or by combining different events during the breeding process resulting in a cultivated plant with stacked traits. Preferred combinations of traits are combinations of herbicide tolerance traits to different groups of herbicides, combinations of insect tolerance to different kind of insects, in particular tolerance to lepidopteran and coleopteran insects, combinations of herbicide tolerance with one or several types of insect resistance, combinations of herbicide tolerance with increased yield as well as combinations of herbicide tolerance and tolerance to abiotic conditions.

Plants comprising singular or stacked traits as well as the genes and events providing these traits are well known in the art. For example, detailed information as to the mutagenized or integrated genes and the respective events are available from websites of the organizations "International Service for the Acquisition of Agri-biotech Applications (ISAAA)" (http://www.isaaa.org/gmapprovaldatabase) and the "Center for Environmental Risk Assessment (CERA)" (http://cera-gmc.org/GMCropDatabase). Further information on specific events and methods to detect them can be found for canola events MS1, MS8, RF3, GT73, MON88302, KK179 in WO01/031042, WO01/041558, WO01/041558, WO02/036831, WO11/153186, WO13/003558; for cotton events MON1445, MON15985, MON531 (MON15985), LLCotton25, MON88913, COT102, 281-24-236, 3006-210-23, COT67B, GHB614, T304-40, GHB119, MON88701, 81910 in WO02/034946, WO02/100163, WO02/100163, WO03/013224, WO04/072235, WO04/039986, WO05/103266, WO05/103266, WO06/128573, WO07/017186, WO08/122406, WO08/151780, WO12/134808, WO13/112527; for corn events GA21, MON810, DLL25, TC1507, MON863, MIR604, LY038, MON88017, 3272, 59122, NK603, MIR162, MON89034, 98140, 32138, MON87460, 5307, 4114, MON87427, DAS40278, MON87411, 33121, MON87403, MON87419 in WO98/044140, US02/102582, US03/126634, WO04/099447, WO04/011601, WO05/103301, WO05/061720, WO05/059103, WO06/098952, WO06/039376, US2007/292854, WO07/142840, WO07/140256, WO08/112019, WO09/103049, WO09/111263, WO10/077816, WO11/084621, WO11/062904, WO11/022469, WO13/169923, WO14/116854, WO15/053998, WO15/142571; for potato events E12, F10, J3, J55, V11, X17, Y9 in WO14/178910, WO14/178913, WO14/178941, WO14/179276, WO16/183445, WO17/062831, WO17/062825; for rice events LLRICE06, LLRICE601, LLRICE62 in WO00/026345, WO00/026356, WO00/026345; and for soybean events H7-1, MON89788, A2704-12, A5547-127, DP305423, DP356043, MON87701, MON87769, CV127, MON87705, DAS68416-4, MON87708, MON87712, SYHT0H2, DAS81419, DAS81419 x DAS44406-6, MON87751 in WO04/074492, WO06/130436, WO06/108674, WO06/108675, WO08/054747, WO08/002872, WO09/064652, WO09/102873, WO10/080829, WO10/037016, WO11/066384, WO11/034704, WO12/051199, WO12/082548, WO13/016527, WO13/016516, WO14/201235.

The use of compounds I and compositions thereof, respectively, on cultivated plants may result in effects which are specific to a cultivated plant comprising a certain transgene or event. These effects might involve changes in growth behavior or resistance to biotic or abiotic stress factors. Such effects may in particular comprise enhanced yield, enhanced resistance or tolerance to insect, nematode, fungal, bacterial, mycoplasma, viral or viroid pathogens as well as early vigour, early or delayed ripening, cold or heat tolerance as well as changed amino acid or fatty acid spectrum or content.

The compounds I and compositions thereof, respectively, are particularly suitable for controlling the following causal agents of plant diseases:
*Albugo* spp. (white rust) on ornamentals, vegetables (e. g. *A. candida*) and sunflowers (e. g. *A. tragopogonis*); *Alternaria* spp. (Alternaria leaf spot) on vegetables (e.g. *A. dauci* or *A. porri*), oilseed rape (*A. brassicicola* or *brassicae*), sugar beets (*A. tenuis*), fruits (e.g. *A. grandis*), rice, soybeans, potatoes and tomatoes (e. g. *A. solani*, *A. grandis* or *A. alternata*), tomatoes (e. g. *A. solani* or *A. alternata*) and wheat (e.g. *A. triticina*); *Aphanomyces* spp. on sugar beets and vegetables; *Ascochyta* spp. on cereals and vegetables, e. g. *A. tritici* (anthracnose) on wheat and *A. hordei* on barley; *Aureobasidium zeae* (syn. *Kapatiella* zeae) on corn; *Bipolaris* and *Drechslera* spp. (teleomorph: *Cochliobolus* spp.), e. g. Southern leaf blight (*D. maydis*) or Northern leaf blight (*B. zeicola*) on corn, e. g. spot blotch (*B. sorokiniana*) on cereals and e. g. *B. oryzae* on rice and turfs; *Blumeria* (formerly *Erysiphe) graminis* (powdery mildew) on cereals (e. g. on wheat or barley); *Botrytis cinerea* (teleomorph: *Botryotinia fuckeliana*: grey mold) on fruits and berries (e. g. strawberries), vegetables (e. g. lettuce, carrots, celery and cabbages); *B*. *squamosa* or *B*. *allii* on onion family), oilseed rape, ornamentals (e.g. *B eliptica),* vines, forestry plants and wheat; *Bremia lactucae* (downy mildew) on lettuce; *Ceratocystis* (syn. *Ophiostoma*) spp. (rot or wilt) on broad-leaved trees and evergreens, e. g. *C*. *ulmi* (Dutch elm disease) on elms; *Cercospora* spp. (Cercospora leaf spots) on corn (e. g. Gray leaf spot: *C*. *zeae-maydis),* rice, sugar beets (e. g. *C*. *beticola*), sugar cane, vegetables, coffee, soybeans (e. g. *C*. *sojina* or *C. kikuchii*) and rice; *Cladobotryum* (*syn. Dactylium*) spp. (e.g. *C*. *mycophilum* (formerly *Dactylium dendroides*, teleomorph: *Nectria albertinii*, *Nectria rosella* syn. *Hypomyces rosellus)* on mushrooms; *Cladosporium* spp. on tomatoes (e. g. *C*. *fulvum:* leaf mold) and cereals, e. g. *C*. *herbarum* (black ear) on wheat; *Claviceps purpurea* (ergot) on cereals; *Cochliobolus* (anamorph: *Helminthosporium* or *Bipolaris*) spp. (leaf spots) on corn (*C*. *carbonum),* cereals (e. g. *C*. *sativus,* anamorph: *B. sorokiniana)* and rice (e. g. *C*. *miyabeanus,* anamorph: *H. oryzae*); *Colletotrichum* (teleomorph: *Glomerella)* spp. (anthracnose) on cotton (e. g. *C*. *gossypii*), corn (e. g. *C*. *graminicola:* Anthracnose stalk rot), soft fruits, potatoes (e. g. *C. coccodes:* black dot), beans (e. g. *C*. *lindemuthianum),* soybeans (e. g. *C*. *truncatum* or *C*. *gloeosporioides*), vegetables (e.g. *C*. *lagenarium* or *C*. *capsici*), fruits (e.g. *C*. *acutatum*), coffee (e.g. *C*. *coffeanum* or *C*. *kahawae*) and *C*. *gloeosporioides* on various crops; *Corticium* spp., e. g. *C*. *sasakii* (sheath blight) on rice; *Corynespora cassiicola* (leaf spots) on soybeans, cotton and ornamentals; *Cycloconium* spp., e. g. *C*. *oleaginum* on olive trees; *Cylindrocarpon* spp. (e. g. fruit tree canker or young vine decline, teleomorph: *Nectria* or *Neonectria* spp.) on fruit trees, vines (e. g. *C*. *liriodendri*, teleomorph: *Neonectria liriodendri*: Black Foot Disease) and ornamentals; *Dematophora* (teleomorph: *Rosellinia) necatrix* (root and stem rot) on soybeans; *Diaporthe* spp., e. g. *D. phaseolorum* (damping off) on soybeans; *Drechslera* (syn. *Helminthosporium,* teleomorph: *Pyrenophora*) spp. on corn, cereals, such as barley (e. g. *D*. *teres,* net blotch) and wheat (e. g. *D*. *tritici-repentis:* tan spot), rice and turf; Esca (dieback, apoplexy) on vines, caused by *Formitiporia* (syn. *Phellinus*) *punctata*, *F. mediterranea*, *Phaeomoniella chlamydospora* (formerly *Phaeoacremonium chlamydosporum*), *Phaeoacremonium aleophilum* and/or *Botryosphaeria obtusa; Elsinoe* spp. on pome fruits (*E*. *pyri*), soft fruits (*E*. *veneta:* anthracnose) and vines (*E*. *ampelina:* anthracnose); *Entyloma oryzae* (leaf smut) on rice; *Epicoccum* spp. (black mold) on wheat; *Erysiphe* spp. (powdery mildew) on sugar beets (*E*. *betae*), vegetables (e. g. *E. pisi*), such as cucurbits (e. g. *E*. *cichoracearum*), cabbages, oilseed rape (e. g. *E. cruciferarum*); *Eutypa lata* (Eutypa canker or dieback, anamorph: *Cytosporina lata,* syn. *Libertella blepharis*) on fruit trees, vines and ornamental woods; *Exserohilum* (syn. *Helminthosporium*) spp. on corn (e. g. *E. turcicum*); *Fusarium* (teleomorph: *Gibberella*) spp. (wilt, root or stem rot) on various plants, such as *F*. *graminearum* or *F. culmorum* (root rot, scab or head blight) on cereals (e. g. wheat or barley), *F*. *oxysporum* on tomatoes, *F. solani* (f. sp. *glycines* now syn. *F. virguliforme*) and *F. tucumaniae* and *F. brasiliense* each causing sudden death syndrome on soybeans, and *F. verticillioides* on corn; *Gaeumannomyces graminis* (take-all) on cereals (e. g. wheat or barley) and corn; *Gibberella* spp. on cereals (e. g. *G. zeae*) and rice (e. g. *G. fujikuroi*: Bakanae disease); *Glomerella cingulata* on vines, pome fruits and other plants and *G. gossypii* on cotton; Grain-staining complex on rice; *Guignardia bidwellii* (black rot) on vines; *Gymnosporangium* spp. on rosaceous plants and junipers, e. g. *G. sabinae* (rust) on pears; *Helminthosporium* spp. (syn. *Drechslera,* teleomorph: *Cochliobolus*) on corn, cereals, potatoes and rice; *Hemileia* spp., e. g. *H. vastatrix* (coffee leaf rust) on coffee; *Isariopsis clavispora* (syn. *Cladosporium vitis*) on vines; *Macrophomina phaseolina* (syn. *phaseoli*) (root and stem rot) on soybeans and cotton; *Microdochium* (syn. *Fusarium*) *nivale* (pink snow mold) on cereals (e. g. wheat or barley); *Microsphaera diffusa* (powdery mildew) on soybeans; *Monilinia* spp., e. g. *M. laxa*, *M. fructicola* and *M. fructigena* (syn. *Monilia* spp.: bloom and twig blight, brown rot) on stone fruits and other rosaceous plants; *Mycosphaerella* spp. on cereals, bananas, soft fruits and ground nuts, such as e. g. *M. graminicola* (anamorph: *Zymoseptoria tritici* formerly *Septoria tritici*: Septoria blotch) on wheat or *M. fijiensis* (syn. *Pseudocercospora fijiensis:* black Sigatoka disease) and *M*. *musicola* on bananas, *M. arachidicola* (syn. *M. arachidis* or *Cercospora arachidis*), *M. berkeleyi* on peanuts, *M. pisi* on peas and *M. brassiciola* on brassicas; *Peronospora* spp. (downy mildew) on cabbage (e. g. *P. brassicae),* oilseed rape (e. g. *P. parasitica*), onions (e. g. *P. destructor*), tobacco (*P*. *tabacina*) and soybeans (e. g. *P. manshurica*); *Phakopsora pachyrhizi* and *P*. *meibomiae* (soybean rust) on soybeans; *Phialophora* spp. e. g. on vines (e. g. *P. tracheiphila* and *P*. *tetraspora*) and soybeans (e. g. *P. gregata:* stem rot); *Phoma lingam* (syn. *Leptosphaeria biglobosa* and *L. maculans*: root and stem rot) on oilseed rape and cabbage, *P. betae* (root rot, leaf spot and damping-off) on sugar beets and *P*. *zeae-maydis* (syn. *Phyllostica zeae*) on corn; *Phomopsis* spp. on sunflowers, vines (e. g. *P*. *viticola*: can and leaf spot) and soybeans (e. g. stem rot: *P. phaseoli,* teleomorph: *Diaporthe phaseolorum*); *Physoderma maydis* (brown spots) on corn; *Phytophthora* spp. (wilt, root, leaf, fruit and stem root) on various plants, such as paprika and cucurbits (e. g. *P. capsici*), soybeans (e. g. *P. megasperma*, syn. *P. sojae*), potatoes and tomatoes (e. g. *P. infestans:* late blight) and broad-leaved trees (e. g. *P. ramorum*: sudden oak death); *Plasmodiophora brassicae* (club root) on cabbage,oilseed rape, radish and other plants; *Plasmopara* spp., e. g. *P*. *viticola* (grapevine downy mildew) on vines and *P*. *halstedii* on sunflowers; *Podosphaera* spp. (powdery mildew) on rosaceous plants, hop, pome and soft fruits (e. g. *P. leucotricha* on apples) and curcurbits (*P*. *xanthii*); *Polymyxa* spp., e. g. on cereals, such as barley and wheat (*P*. *graminis*) and sugar beets (*P*. *betae*) and thereby transmitted viral diseases; *Pseudocercosporella herpotrichoides* (syn. *Oculimacula yallundae*, *O. acuformis:* eyespot, teleomorph: *Tapesia yallundae*) on cereals, e. g. wheat or barley; *Pseudoperonospora* (downy mildew) on various plants, e. g. *P. cubensis* on cucurbits or *P*. *humili* on hop; *Pseudopezicula tracheiphila* (red fire disease or, rotbrenner', anamorph: *Phialophora*) on vines; *Puccinia* spp. (rusts) on various plants, e. g. *P. triticina* (brown or leaf rust), *P. striiformis* (stripe or yellow rust), *P. hordei* (dwarf rust), *P. graminis* (stem or black rust) or *P*. *recondita* (brown or leaf rust) on cereals, such as e. g. wheat, barley or rye, *P. kuehnii* (orange rust) on sugar cane and *P*. *asparagi* on asparagus; *Pyrenopeziza spp.,* e.g. *P*. *brassicae* on oilseed rape; *Pyrenophora* (anamorph: *Drechslera*) *tritici-repentis* (tan spot) on wheat or *P*. *teres* (net blotch) on barley; *Pyricularia* spp., e. g. *P*. *oryzae* (teleomorph: *Magnaporthe grisea*: rice blast) on rice and *P*. *grisea* on turf and cereals; *Pythium* spp. (damping-off) on turf, rice, corn, wheat, cotton, oilseed rape, sunflowers, soybeans, sugar beets, vegetables and various other plants (e. g. *P*. *ultimum* or *P*. *aphanidermatum*) and *P*. *oligandrum* on mushrooms; *Ramularia* spp., e. g. *R. collo-cygni* (Ramularia leaf spots, Physiological leaf spots) on barley, *R. areola* (teleomorph: *Mycosphaerella areola*) on cotton and *R. beticola* on sugar beets; *Rhizoctonia* spp. on cotton, rice, potatoes, turf, corn, oilseed rape, potatoes, sugar beets, vegetables and various other plants, e. g. *R. solani* (root and stem rot) on soybeans, *R*. *solani* (sheath blight) on rice or *R. cerealis* (Rhizoctonia spring blight) on wheat or barley; *Rhizopus stolonifer* (black mold, soft rot) on strawberries, carrots, cabbage, vines and tomatoes; *Rhynchosporium secalis* and *R. commune* (scald) on barley, rye and triticale; *Sarocladium oryzae* and *S*. *attenuatum* (sheath rot) on rice; *Sclerotinia* spp. (stem rot or white mold) on vegetables (*S*. *minor* and *S*. *sclerotiorum*) and field crops, such as oilseed rape, sunflowers (e. g. *S*. *sclerotiorum*) and soybeans, *S*. *rolfsii* (syn. *Athelia rolfsii*) on soybeans, peanut, vegetables, corn, cereals and ornamentals; *Septoria* spp. on various plants, e. g. *S*. *glycines* (brown spot) on soybeans, *S*. *tritici* (syn. *Zymoseptoria tritici,* Septoria blotch) on wheat and *S*. (syn. *Stagonospora) nodorum* (Stagonospora blotch) on cereals; *Uncinula* (syn. *Erysiphe*) *necator* (powdery mildew, anamorph: *Oidium tuckeri*) on vines; *Setosphaeria* spp. (leaf blight) on corn (e. g. *S*. *turcicum*, syn. *Helminthosporium turcicum)* and turf; *Sphacelotheca* spp. (smut) on corn, (e. g. *S*. *reiliana*, syn. *Ustilago reiliana:* head smut), sorghum und sugar cane; *Sphaerotheca fuliginea* (syn. *Podosphaera xanthii:* powdery mildew) on cucurbits; *Spongospora subterranea* (powdery scab) on potatoes and thereby transmitted viral diseases; *Stagonospora* spp. on cereals, e. g. *S*. *nodorum* (Stagonospora blotch, teleomorph: *Leptosphaeria* [syn. *Phaeosphaeria] nodorum*, syn. *Septoria nodorum*) on wheat; *Synchytrium endobioticum* on potatoes (potato wart disease); *Taphrina* spp., e. g. *T. deformans* (leaf curl disease) on peaches and *T. pruni* (plum pocket) on plums; *Thielaviopsis* spp. (black root rot) on tobacco, pome fruits, vegetables, soybeans and cotton, e. g. *T. basicola* (syn. *Chalara elegans*); *Tilletia* spp. (common bunt or stinking smut) on cereals, such as e. g. *T. tritici* (syn. *T. caries,* wheat bunt) and *T. controversa* (dwarf bunt) on wheat; *Trichoderma harzianum on mushrooms*; *Typhula incarnata* (grey snow mold) on barley or wheat; *Urocystis* spp., e. g. *U. occulta* (stem smut) on rye; *Uromyces* spp. (rust) on vegetables, such as beans (e. g. *U. appendiculatus*, syn. *U. phaseoli*), sugar beets (e. g. *U. betae* or *U. beticola*) and on pulses (e.g. *U. vignae*, *U. pisi*, *U. viciae-fabae* and *U. fabae); Ustilago* spp. (loose smut) on cereals (e. g. *U. nuda* and *U*. *avaenae),* corn (e. g. *U. maydis:* corn smut) and sugar cane; *Venturia* spp. (scab) on apples (e. g. *V. inaequalis*) and pears; and *Verticillium* spp. (wilt) on various plants, such as fruits and ornamentals, vines, soft fruits, vegetables and field crops, e. g. *V. longisporum* on oilseed rape, *V. dahliae* on strawberries, oilseed rape, potatoes and tomatoes, and *V. fungicola* on mushrooms; *Zymoseptoria tritici* on cereals.

The compounds I and compositions thereof, respectively, are particularly suitable for controlling the following causal agents of plant diseases: rusts on soybean and cereals (e.g. *Phakopsora pachyrhizi* and *P*. *meibomiae* on soy; *Puccinia tritici*, *P. graminis, P. recondita* and *P. striiformis* on wheat); molds on specialty crops, soybean, oil seed rape and sunflowers (e.g. *Botrytis cinerea* on strawberries and vines, *Sclerotinia sclerotiorum, S. minor* and *S*. *rolfsii* on oil seed rape, sunflowers and soybean); Fusarium diseases on cereals (e.g. *Fusarium culmorum* and *F. graminearum* on wheat); downy mildews on specialty crops (e.g. *Plasmopara viticola* on vines, *Phytophthora infestans* on potatoes); powdery mildews on specialty crops and cereals (e.g. *Uncinula necator* on vines, *Erysiphe* spp. on various specialty crops, *Blumeria graminis* on cereals); and leaf spots on cereals, soybean and corn (e.g. *Zymoseptoria tritici* and *Septoria nodorum* on cereals, *S*. *glycines* on soybean, *Cercospora* spp. on corn and soybean).

A further embodiment relates to the use of compound of formula (I) for combating soybean rust on soybean plants and on the plant propagation material, such as seeds, and the crop material of these plants. Soybean rust is cause by two fungal pathogens called *Phakopsora pachyrhizi* and *P*. *meibomiae.*

Consequently, a further embodiment relates to the use of compounds I for combating *Phakopsora pachyrhizi* and/or *P*. *meibomiae* on soybean plants and on the plant propagation material, such as seeds, and the crop material of these plants. A more preferred embodiment the use of compounds I for combating *Phakopsora pachyrhizi* on soybean plants and on the plant propagation material, such as seeds, and the crop material of these plants.

Accordingly, the present invention relates to the method for combating soybean rust *(Phakopsora pachyrhizi* and/or *P*. *meibomiae*), comprising:
treating the soybean plants or soybean plant propagation material to be protected against attack by *Phakopsora pachyrhizi* and/or *P*. *meibomiae* with an effective amount of at least one compound I, or a composition comprising such compound I.

Treatment against soybean rust can be preventive or curative.

Preferably treatment of soybean plants against soybean rust shall be preventive. Preventive treatment shall be performed when the soybean plants are at risk of infection latest shortly after the first symptoms are visible. According to one embodiment, the first treating of the soybean plants shall take place at the vegetative growth stages V3 to V4 (meaning 4 to 4 fully expanded trifoliate leaves) onwards to the reproductive growth stage R2 (full bloom), more preferably place at the vegetative growth stages V6 to V8 (meaning 6 to 8 fully expanded trifoliate leaves) onwards to the reproductive growth stage R3 (beginning bloom). Depending on the disease pressure, two to four and under extreme conditions up to five applications may be necessary at application intervals of 14 to 28 days.

When employed as foliar spray against soybean rust, the amounts of the compounds I applied are, depending on the specific compound used and on the disease pressure, from 5 g to 500 g per ha, preferably from 10 to 200 per ha, more preferably from 15 to 150 g per ha, and in particular from 30 to 125 g per ha.

Furthermore, the present invention relates to the use of compounds of formula I as defined herein for combating phytopathogenic fungi containing an amino acid substitution F129L in the mitochondrial cytochrome b protein conferring resistance to Qo inhibitors.

The mutation F129L in the cytochrome b (cytb, also referred to as cob) gene shall mean any substitution of nucleotides of codon 129 encoding "F" (phenylalanine; e.g. TTT or TTC) that leads to a codon encoding "L" (leucine; e.g. TTA, TTG, TTG, CTT, CTC, CTA or CTG), for example the substitution of the first nucleotide of codon 129 'T' to 'C' (TTT to CTT), in the cytochrome b gene resulting in a single amino acid substitution in the position 129 from F (phenylalanine) to L (leucine) (F129L) in the cytochrome b protein (Cytb). In the present invention, the mutation F129L in the cytochrome b gene shall be understood to be a single amino acid substitution in the position 129 from F (phenylalanine) to L (leucine) (F129L) in the cytochrome b protein.

Many other phytopathogenic fungi acquired the F129L mutation in the cytochrome b gene conferring resistance to Qo inhibitors, such as rusts, in particular soybean rust (*Phakopsora pachyrhizi* and *Phakopsora meibromiae*) as well as fungi from the genera Alternaria, Pyrenophora and Rhizoctonia.

Preferred fungal species are *Alternaria solani*, *Phakopsora pachyrhizi*, *Phakopsora meibromiae, Pyrenophora teres*, *Pyrenophora tritici-repentis* and *Rhizoctonia solani*; in particular *Phakopsora pachyrhizi.*

In one aspect, the present invention relates to the method of protecting plants susceptible to and/or under attack by phytopathogenic fungi containing an amino acid substitution F129L in the mitochondrial cytochrome b protein conferring resistance to Qo inhibitors, which method comprises applying to said plants, treating plant propagation material of said plants with, and/or applying to said phytopathogenic fungi, at least one compound of formula I or a composition comprising at least one compound of formula I.

According to another embodiment, the method for combating phytopathogenic fungi, comprises: a) identifying the phytopathogenic fungi containing an amino acid substitution F129L in the mitochondrial cytochrome b protein conferring resistance to Qo inhibitors, or the materials, plants, the soil or seeds that are at risk of being diseased from phytopathogenic fungi as defined herein, and b) treating said fungi or the materials, plants, the soil or plant propagation material with an effective amount of at least one compound of formula I, or a composition comprising it thereof.

The term "phytopathogenic fungi an amino acid substitution F129L in the mitochondrial cytochrome b protein conferring resistance to Qo inhibitors" is to be understood that at least 10% of the fungal isolates to be controlled contain a such F129L substitution in the mitochondrial cytochrome b protein conferring resistance to Qo inhibitors, preferably at least 30%, more preferably at least 50%, even more preferably at at least 75% of the fungi, most preferably between 90 and 100%; in particular between 95 and 100%.

The compounds I and compositions thereof, respectively, are also suitable for controlling harmful microorganisms in the protection of stored products or harvest, and in the protection of materials.

The term "stored products or harvest" is understood to denote natural substances of plant or animal origin and their processed forms for which long-term protection is desired. Stored products of plant origin, for example stalks, leafs, tubers, seeds, fruits or grains, can be protected in the freshly harvested state or in processed form, such as pre-dried, moistened, comminuted, ground, pressed or roasted, which process is also known as post-harvest treatment. Also falling under the definition of stored products is timber, whether in the form of crude timber, such as construction timber, electricity pylons and barriers, or in the form of finished articles, such as furniture or objects made from wood. Stored products of animal origin are hides, leather, furs, hairs and alike. Preferably, "stored products" is understood to denote natural substances of plant origin and their processed forms, more preferably fruits and their processed forms, such as pomes, stone fruits, soft fruits and citrus fruits and their processed forms, where application of compounds I and compositions thereof can also prevent disadvantageous effects such as decay, discoloration or mold.

The term "protection of materials" is to be understood to denote the protection of technical and non-living materials, such as adhesives, glues, wood, paper, paperboard, textiles, leather, paint dispersions, plastics, cooling lubricants, fiber, or fabrics against the infestation and destruction by harmful microorganisms, such as fungi and bacteria.

When used in the protection of materials or stored products, the amount of active substance applied depends on the kind of application area and on the desired effect. Amounts customarily applied in the protection of materials are 0.001 g to 2 kg, preferably 0.005 g to 1 kg, of active substance per cubic meter of treated material.

The compounds I and compositions thereof, respectively, may be used for improving the health of a plant. The invention also relates to a method for improving plant health by treating a plant, its propagation material, and/or the locus where the plant is growing or is to grow with an effective amount of compounds I and compositions thereof, respectively.

The term "plant health" is to be understood to denote a condition of the plant and/or its products which is determined by several indicators alone or in combination with each other, such as yield (e. g. increased biomass and/or increased content of valuable ingredients), plant vigor (e. g. improved plant growth and/or greener leaves ("greening effect")), quality (e. g. improved content or composition of certain ingredients), and tolerance to abiotic and/or biotic stress. The above identified indicators for the health condition of a plant may be interdependent or may result from each other.

The compounds I are employed as such or in form of compositions by treating the fungi, the plants, plant propagation materials, such as seeds; soil, surfaces, materials, or rooms to be protected from fungal attack with a fungicidally effective amount of the active substances. The application can be carried out both before and after the infection of the plants, plant propagation materials, such as seeds; soil, surfaces, materials or rooms by the fungi.

Plant propagation materials may be treated with compounds I as such or a composition comprising at least one compound I prophylactically either at or before planting or transplanting.

The invention also relates to agrochemical compositions comprising an auxiliary and at least one compound I.

When employed in plant protection, the amounts of active substances applied are, depending on the kind of effect desired, from 0.001 to 2 kg per ha, preferably from 0.005 to 2 kg per ha, more preferably from 0.05 to 0.9 kg per ha, even more preferably from 0.075 to 0.75 kg per ha, and in particular from 0.1 to 0.3 kg per ha.

In treatment of plant propagation materials, such as seeds, e. g. by dusting, coating, or drenching, amounts of active substance of generally from 0.1 to 1000 g, preferably from 1 to 1000 g, more preferably from 1 to 100 g and most preferably from 5 to 100 g, per 100 kg of plant propagation material (preferably seeds) are required.

An agrochemical composition comprises a fungicidally effective amount of a compound I. The term "fungicidally effective amount" denotes an amount of the composition or of the compounds I, which is sufficient for controlling phytopathogenic fungi on cultivated plants or in the protection of stored products or harvest or of materials and which does not result in a substantial damage to the treated plants, the treated stored products or harvest, or to the treated materials. Such an amount can vary in a broad range and is dependent on various factors, such as the fungal species to be controlled, the treated cultivated plant, stored product, harvest or material, the climatic conditions and the specific compound I used.

The user applies the agrochemical composition usually from a predosage device, a knapsack sprayer, a spray tank, a spray plane, or an irrigation system. Usually, the agrochemical composition is made up with water, buffer, and/or further auxiliaries to the desired application concentration and the ready-to-use spray liquor or the agrochemical composition according to the invention is thus obtained. Usually, 20 to 2000 liters, preferably 50 to 400 liters, of the ready-to-use spray liquor are applied per hectare of agricultural useful area.

The compounds I, their N-oxides and salts can be converted into customary types of agrochemical compositions, e. g. solutions, emulsions, suspensions, dusts, powders, pastes, granules, pressings, capsules, and mixtures thereof. Examples for composition types (see also "Catalogue of pesticide formulation types and international coding system", Technical Monograph No. 2, 6th Ed. May 2008, CropLife International) are suspensions (e. g. SC, OD, FS), emulsifiable concentrates (e. g. EC), emulsions (e. g. EW, EO, ES, ME), capsules (e. g. CS, ZC), pastes, pastilles, wettable powders or dusts (e. g. WP, SP, WS, DP, DS), pressings (e. g. BR, TB, DT), granules (e. g. WG, SG, GR, FG, GG, MG), insecticidal articles (e. g. LN), as well as gel formulations for the treatment of plant propagation materials, such as seeds (e. g. GF). The compositions are prepared in a known manner, such as described by Mollet and Grubemann, Formulation technology, Wiley VCH, Weinheim, 2001; or by Knowles, New developments in crop protection product formulation, Agrow Reports DS243, T&F Informa, London, 2005.

Suitable auxiliaries are solvents, liquid carriers, solid carriers or fillers, surfactants, dispersants, emulsifiers, wetters, adjuvants, solubilizers, penetration enhancers, protective colloids, adhesion agents, thickeners, humectants, repellents, attractants, feeding stimulants, compatibilizers, bactericides, anti-freezing agents, anti-foaming agents, colorants, tackifiers, and binders.

Suitable solvents and liquid carriers are water and organic solvents, such as mineral oil fractions of medium to high boiling point, e. g. kerosene, diesel oil; oils of vegetable or animal origin; aliphatic, cyclic and aromatic hydrocarbons, e. g. toluene, paraffin, tetrahydronaphthalene, and alkylated naphthalenes; alcohols, e. g. ethanol, propanol, butanol, benzyl alcohol, cyclohexanol, glycols; DMSO; ketones, e. g. cyclohexanone; esters, e. g. lactates, carbonates, fatty acid esters, gamma-butyrolactone; fatty acids; phosphonates; amines; amides, e. g. *N*-methyl pyrrolidone, fatty acid dimethyl amides; and mixtures thereof.

Suitable solid carriers or fillers are mineral earths, e. g. silicates, silica gels, talc, kaolins, limestone, lime, chalk, clays, dolomite, diatomaceous earth, bentonite, calcium sulfate, magnesium sulfate, magnesium oxide; polysaccharides, e. g. cellulose, starch; fertilizers, e. g. ammonium sulfate, ammonium phosphate, ammonium nitrate, ureas; products of vegetable origin, e. g. cereal meal, tree bark meal, wood meal, nutshell meal, and mixtures thereof.

Suitable surfactants are surface-active compounds, such as anionic, cationic, nonionic and amphoteric surfactants, block polymers, polyelectrolytes, and mixtures thereof. Such surfactants can be used as emulsifier, dispersant, solubilizer, wetter, penetration enhancer, protective colloid, or adjuvant. Examples of surfactants are listed in McCutcheon's, Vol.1: Emulsifiers & Detergents, McCutcheon's Directories, Glen Rock, USA, 2008 (Int. Ed. or N. American Ed.).

Suitable anionic surfactants are alkali, alkaline earth or ammonium salts of sulfonates, sulfates, phosphates, carboxylates, and mixtures thereof. Examples of sulfonates are alkylaryl sulfonates, diphenyl sulfonates, alpha-olefin sulfonates, lignin sulfonates, sulfonates of fatty acids and oils, sulfonates of ethoxylated alkylphenols, sulfonates of alkoxylated arylphenols, sulfonates of condensed naphthalenes, sulfonates of dodecyl- and tridecylbenzenes, sulfonates of naphthalenes and of alkyl naphthalenes, sulfosuccinates, or sulfosuccinamates. Examples of sulfates are sulfates of fatty acids, of oils, of ethoxylated alkylphenols, of alcohols, of ethoxylated alcohols, or of fatty acid esters. Examples of phosphates are phosphate esters. Examples of carboxylates are alkyl carboxylates, and carboxylated alcohol or alkylphenol ethoxylates.

Suitable nonionic surfactants are alkoxylates, *N*-substituted fatty acid amides, amine oxides, esters, sugar-based surfactants, polymeric surfactants, and mixtures thereof. Examples of alkoxylates are compounds such as alcohols, alkylphenols, amines, amides, arylphenols, fatty acids or fatty acid esters which have been alkoxylated with 1 to 50 equivalents. Ethylene oxide and/or propylene oxide may be employed for the alkoxylation, preferably ethylene oxide. Examples of *N*-substituted fatty acid amides are fatty acid glucamides or fatty acid alkanolamides. Examples of esters are fatty acid esters, glycerol esters, or monoglycerides. Examples of sugar-based surfactants are sorbitans, ethoxylated sorbitans, sucrose and glucose esters, or alkylpolyglucosides. Examples of polymeric surfactants are home- or copolymers of vinyl pyrrolidone, vinyl alcohols, or vinyl acetate.

Suitable cationic surfactants are quaternary surfactants, for example quaternary ammonium compounds with one or two hydrophobic groups, or salts of long-chain primary amines. Suitable amphoteric surfactants are alkylbetains and imidazolines. Suitable block polymers are block polymers of the A-B or A-B-A type comprising blocks of polyethylene oxide and polypropylene oxide, or of the A-B-C type comprising alkanol, polyethylene oxide, and polypropylene oxide. Suitable polyelectrolytes are polyacids or polybases. Examples of polyacids are alkali salts of polyacrylic acid or polyacid comb polymers. Examples of polybases are polyvinyl amines or polyethylene amines.

Suitable adjuvants are compounds, which have a negligible or even no pesticidal activity themselves, and which improve the biological performance of the compound I on the target such as surfactants, mineral or vegetable oils, and other auxiliaries. Further examples are listed by Knowles, Adjuvants and additives, Agrow Reports DS256, T&F Informa UK, 2006, Ch. 5.

Suitable thickeners are polysaccharides (e. g. xanthan gum, carboxymethyl cellulose), inorganic clays (organically modified or unmodified), polycarboxylates, and silicates.

Suitable bactericides are bronopol and isothiazolinone derivatives, such as alkyliso-thiazolinones and benzisothiazolinones.

Suitable anti-freezing agents are ethylene glycol, propylene glycol, urea and glycerin.

Suitable anti-foaming agents are silicones, long chain alcohols, and salts of fatty acids.

Suitable colorants (e. g. in red, blue, or green) are pigments of low water solubility and water-soluble dyes. Examples are inorganic colorants (e. g. iron oxide, titan oxide, iron hexacyanoferrate) and organic colorants (e. g. alizarin-, azo- and phthalocyanine colorants).

Suitable tackifiers or binders are polyvinyl pyrrolidones, polyvinyl acetates, polyvinyl alcohols, polyacrylates, biological or synthetic waxes, and cellulose ethers.

The agrochemical compositions generally comprise between 0.01 and 95 %, preferably between 0.1 and 90 %, more preferably between 1 and 70 %, and in particular between 10 and 60 %, by weight of active substances (e.g. at least one compound I). The agrochemical compositions generally comprise between 5 and 99.9 %, preferably between 10 and 99.9 %, more preferably between 30 and 99 %, and in particular between 40 and 90 %, by weight of at least one auxiliary. The active substances (e.g. compounds I) are employed in a purity of from 90 % to 100 %, preferably from 95-% to 100 % (according to NMR spectrum).

For the purposes of treatment of plant propagation materials, particularly seeds, solutions for seed treatment (LS), suspoemulsions (SE), flowable concentrates (FS), powders for dry treatment (DS), water-dispersible powders for slurry treatment (WS), water-soluble powders (SS), emulsions (ES), emulsifiable concentrates (EC), and gels (GF) are usually employed. The compositions in question give, after two-to-tenfold dilution, active substance concentrations of from 0.01 to 60 % by weight, preferably from 0.1 to 40 %, in the ready-to-use preparations. Application can be carried out before or during sowing. Methods for applying compound I and compositions thereof, respectively, onto plant propagation material, especially seeds, include dressing, coating, pelleting, dusting, soaking, as well as in-furrow application methods. Preferably, compound I or the compositions thereof, respectively, are applied on to the plant propagation material by a method such that germination is not induced, e. g. by seed dressing, pelleting, coating, and dusting.

Various types of oils, wetters, adjuvants, fertilizers, or micronutrients, and further pesticides (e. g. fungicides, growth regulators, herbicides, insecticides, safeners) may be added to the compounds I or the compositions thereof as premix, or, not until immediately prior to use (tank mix). These agents can be admixed with the compositions according to the invention in a weight ratio of 1:100 to 100:1, preferably 1:10 to 10:1.

A pesticide is generally a chemical or biological agent (such as pestidal active ingredient, compound, composition, virus, bacterium, antimicrobial, or disinfectant) that through its effect deters, incapacitates, kills or otherwise discourages pests. Target pests can include insects, plant pathogens, weeds, mollusks, birds, mammals, fish, nematodes (roundworms), and microbes that destroy property, cause nuisance, spread disease or are vectors for disease. The term "pesticide" includes also plant growth regulators that alter the expected growth, flowering, or reproduction rate of plants; defoliants that cause leaves or other foliage to drop from a plant, usually to facilitate harvest; desiccants that promote drying of living tissues, such as unwanted plant tops; plant activators that activate plant physiology for defense of against certain pests; safeners that reduce unwanted herbicidal action of pesticides on crop plants; and plant growth promoters that affect plant physiology e.g. to increase plant growth, biomass, yield or any other quality parameter of the harvestable goods of a crop plant.

Biopesticides have been defined as a form of pesticides based on microorganisms (bacteria, fungi, viruses, nematodes, etc.) or natural products (compounds, such as metabolites, proteins, or extracts from biological or other natural sources) (U.S. Environmental Protection Agency: http://www.epa.gov/pesticides/biopesticides/). Biopesticides fall into two major classes, microbial and biochemical pesticides:
(1) Microbial pesticides consist of bacteria, fungi or viruses (and often include the metabolites that bacteria and fungi produce). Entomopathogenic nematodes are also classified as microbial pesticides, even though they are multi-cellular;
(2) Biochemical pesticides are naturally occurring substances that control pests or provide other crop protection uses as defined below, but are relatively non-toxic to mammals.

Mixing the compounds I or the compositions comprising them in the use form as fungicides with other fungicides results in many cases in an expansion of the fungicidal spectrum of activity or in a prevention of fungicide resistance development. Furthermore, in many cases, synergistic effects are obtained (synergistic mixtures).

The following list of pesticides II, in conjunction with which the compounds I can be used, is intended to illustrate the possible combinations but does not limit them:
A) Respiration inhibitors
   - Inhibitors of complex III at Qₒ site: azoxystrobin (A.1.1), coumethoxystrobin (A.1.2), coumoxystrobin (A.1.3), dimoxystrobin (A.1.4), enestroburin (A.1.5), fenaminstrobin (A.1.6), fenoxystrobin/flufenoxystrobin (A.1.7), fluoxastrobin (A.1.8), kresoxim-methyl (A.1.9), mandestrobin (A.1.10), metominostrobin (A.1.11), orysastrobin (A.1.12), picoxystrobin (A.1.13), pyraclostrobin (A.1.14), pyrametostrobin (A.1.15), pyraoxystrobin (A.1.16), trifloxy-strobin (A.1.17), 2-(2-(3-(2,6-dichlorophenyl)-1-methyl-allylideneaminooxymethyl)-phenyl)-2-methoxyimino-*N*-methyl-acetamide (A.1.18), pyribencarb (A.1.19), triclopyricarb/chloro-dincarb (A.1.20), famoxadone (A.1.21), fenamidone (A.1.21), methyl-*N*-[2-[(1,4-dimethyl-5-phenyl-pyrazol-3-yl)oxylmethyl]phenyl]-*N*-methoxy-carbamate (A.1.22), metyltetraprole (A.1.25), (*Z*,2*E*)-5-[1-(2,4-dichlorophenyl)pyrazol-3-yl]-oxy-2-methoxyimino-*N*,3-dimethyl-pent-3-enamide (A.1.34), (*Z*,2*E*)-5-[1-(4-chlorophenyl)pyrazol-3-yl]oxy-2-methoxyimino-*N*,3-dimethyl-pent-3-enamide (A.1.35), pyriminostrobin (A.1.36), bifujunzhi (A.1.37), 2-(ortho-((2,5-dimethylphenyl-oxymethylen)phenyl)-3-methoxy-acrylic acid methylester (A. 1.38);
   - inhibitors of complex III at Qᵢ site: cyazofamid (A.2.1), amisulbrom (A.2.2), [(6S,7R,8R)-8-benzyl-3-[(3-hydroxy-4-methoxy-pyridine-2-carbonyl)amino]-6-methyl-4,9-dioxo-1,5-dioxonan-7-yl] 2-methylpropanoate (A.2.3), fenpicoxamid (A.2.4), florylpicoxamid (A.2.5), [(1S,2S)-2-(4-fluoro-2-methyl-phenyl)-1,3-dimethyl-butyl] (2S)-2-[(3-hydroxy-4-methoxy-pyridine-2-carbonyl)amino]propanoate, [(1S,2S)-2-(2,4-dimethylphenyl)-1 ,3-dimethyl-butyl] (2S)-2-[(3-hydroxy-4-methoxy-pyridine-2-carbonyl)amino]propanoate, [(1S,2S)-2-(2,4-difluorophenyl)-1,3-dimethyl-butyl] (2S)-2-[(3-hydroxy-4-methoxy-pyridine-2-carbonyl)amino]-propanoate, [(1S,2S)-2-(2-fluoro-4-methyl-phenyl)-1,3-dimethyl-butyl] (2S)-2-[(3-hydroxy-4-methoxy-pyridine-2-carbonyl)amino]propanoate, [(1S,2S)-2-(4-fluoro-2-methyl-phenyl)-1,3-dimethyl-butyl] (2S)-2-[(3-acetoxy-4-methoxy-pyridine-2-carbonyl)amino]propanoate, [(1S,2S)-2-(2,4-dimethylphenyl)-1 ,3-dimethyl-butyl] (2S)-2-[(3-acetoxy-4-methoxy-pyridine-2-carbonyl)amino]propanoate, [(1S,2S)-2-(2,4-difluorophenyl)-1,3-dimethyl-butyl] (2S)-2-[(3-acetoxy-4-methoxy-pyridine-2-carbonyl)amino]propanoate, [(1S,2S)-2-(2-fluoro-4-methylphenyl)-1,3-dimethyl-butyl] (2S)-2-[(3-acetoxy-4-methoxy-pyridine-2-carbonyl)amino]propanoate, [2-[[(1S)-2-[(1S,2S)-2-(4-fluoro-2-methyl-phenyl)-1,3-dimethyl-butoxy]-1-methyl-2-oxoethyl]carbamoyl]-4-methoxy-3-pyridyl]oxymethyl 2-methylpropanoate, [2-[[(1S)-2-[(1S,2S)-2-(2,4-dimethylphenyl)-1,3-dimethyl-butoxy]-1-methyl-2-oxo-ethyl]carbamoyl]-4-methoxy-3-pyridyl]oxymethyl 2-methylpropanoate, [2-[[(1S)-2-[(1S,2S)-2-(2,4-difluorophenyl)-1,3-dimethyl-butoxy]-1-methyl-2-oxo-ethyl]carbamoyl]-4-methoxy-3-pyridyl]oxymethyl 2-methylpropanoate, [2-[[(1S)-2-[(1S,2S)-2-(2-fluoro-4-methyl-phenyl)-1,3-dimethyl-butoxy]-1-methyl-2-oxo-ethyl]carbamoyl]-4-methoxy-3-pyridyl]oxymethyl 2-methylpropanoate, [(1S,2S)-1-methyl-2-(o-tolyl)propyl] (2S)-2-[(3-acetoxy-4-methoxy-pyridine-2-carbonyl)amino]propanoate, [(1S,2S)-1-methyl-2-(o-tolyl)propyl] (2S)-2-[(4-methoxy-3-propanoyloxy-pyridine-2-carbonyl)-amino]propanoate, [(1S,2S)-1-methyl-2-(o-tolyl)propyl] (2S)-2-[(3-hydroxy-4-methoxy-pyridine-2-carbonyl)amino]propanoate, [4-methoxy-2-[[(1S)-1-methyl-2-[(1S,2S)-1-methyl-2-(o-tolyl)propoxy]-2-oxo-ethyl]carbamoyl]-3-pyridyl] 2-methylpropanoate, [(1S,2S)-2-(2,4-dimethylphenyl)-1-methyl-propyl] (2S)-2-[(3-acetoxy-4-methoxy-pyridine-2-carbonyl)amino]-propanoate, [2-[[(1S)-2-[(1S,2S)-2-(2,4-dimethylphenyl)-1-methyl-propoxy]-1-methyl-2-oxoethyl]carbamoyl]-4-methoxy-3-pyridyl] 2-methylpropanoate, [(1S,2S)-2-(2,4-dimethylphenyl)-1-methyl-propyl] (2S)-2-[(3-hydroxy-4-methoxy-pyridine-2-carbonyl)amino]propanoate, [(1S,2S)-2-(2,6-dimethylphenyl)-1-methyl-propyl] (2S)-2-[(3-acetoxy-4-methoxy-pyridine-2-carbonyl)amino]propanoate, [2-[[(1S)-2-[(1S,2S)-2-(2,6-dimethylphenyl)-1-methyl-propoxy]-1-methyl-2-oxo-ethyl]carbamoyl]-4-methoxy-3-pyridyl] 2-methylpropanoate, [(1S,2S)-2-(2,6-dimethylphenyl)-1-methyl-propyl] (2S)-2-[(3-hydroxy-4-methoxy-pyridine-2-carbonyl)-amino]propanoate, [(1S,2S)-2-[4-fluoro-2-(trifluoromethyl)phenyl]-1-methyl-propyl] (2S)-2-[(3-acetoxy-4-methoxy-pyridine-2-carbonyl)amino]propanoate, [2-[[(1S)-2-[(1S,2S)-2-[4-fluoro-2-(trifluoromethyl)phenyl]-1-methyl-propoxy]-1-methyl-2-oxo-ethyl]carbamoyl]-4-methoxy-3-pyridyl] 2-methylpropanoate, [(1S,2S)-2-[4-fluoro-2-(trifluoromethyl)phenyl]-1-methyl-propyl] (2S)-2-[(3-hydroxy-4-methoxy-pyridine-2-carbonyl)amino]propanoate, [(1S,2S)-2-(4-fluoro-2-methyl-phenyl)-1-methyl-propyl] (2S)-2-[(3-acetoxy-4-methoxy-pyridine-2-carbonyl)amino]propanoate, [2-[[(1S)-2-[(1S,2S)-2-(4-fluoro-2-methyl-phenyl)-1-methyl-propoxy]-1-methyl-2-oxo-ethyl]carbamoyl]-4-methoxy-3-pyridyl]2-methylpropanoate, [(1S,2S)-2-(4-fluoro-2-methyl-phenyl)-1-methyl-propyl] (2S)-2-[(3-hydroxy-4-methoxy-pyridine-2-carbonyl)amino]propanoate, [(1S,2S)-1-methyl-2-[2-(trifluoromethyl)phenyl]propyl] (2S)-2-[(3-acetoxy-4-methoxy-pyridine-2-carbonyl)amino]propanoate, [4-methoxy-2-[[(1S)-1-methyl-2-[(1S,2S)-1-methyl-2-[2-(trifluoromethyl)phenyl]propoxy]-2-oxo-ethyl]carbamoyl]-3-pyridyl] 2-methylpropanoate, [(1S,2S)-1-methyl-2-[2-(trifluoromethyl)phenyl]propyl] (2S)-2-[(3-hydroxy-4-methoxy-pyridine-2-carbonyl)amino]propanoate, [(1S,2S)-2-(4-fluoro-2,6-dimethyl-phenyl)-1-methyl-propyl] (2S)-2-[(3-acetoxy-4-methoxy-pyridine-2-carbonyl)-amino]propanoate, [2-[[(1S)-2-[(1S,2S)-2-(4-fluoro-2,6-dimethyl-phenyl)-1-methyl-propoxy]-1-methyl-2-oxo-ethyl]carbamoyl]-4-methoxy-3-pyridyl] 2-methylpropanoate, [(1S,2S)-2-(4-fluoro-2,6-dimethyl-phenyl)-1-methyl-propyl] (2S)-2-[(3-hydroxy-4-methoxy-pyridine-2-carbonyl)amino]propanoate;
   - inhibitors of complex II: benodanil (A.3.1), benzovindiflupyr (A.3.2), bixafen (A.3.3), boscalid (A.3.4), carboxin (A.3.5), fenfuram (A.3.6), fluopyram (A.3.7), flutolanil (A.3.8), fluxapyroxad (A.3.9), furametpyr (A.3.10), isofetamid (A.3.11), isopyrazam (A.3.12), mepronil (A.3.13), oxycarboxin (A.3.14), penflufen (A.3.15), penthiopyrad (A.3.16), pydiflumetofen (A.3.17), pyraziflumid (A.3.18), sedaxane (A.3.19), tecloftalam (A.3.20), thifluzamide (A.3.21), inpyrfluxam (A.3.22), pyrapropoyne (A.3.23), fluindapyr (A.3.28), N-[2-[2-chloro-4-(trifluoromethyl)phenoxy]phenyl]-3-(difluoromethyl)-5-fluoro-1-methyl-pyrazole-4-carboxamide (A.3.29), methyl (*E*)-2-[2-[(5-cyano-2-methyl-phenoxy)methyl]phenyl]-3-methoxy-prop-2-enoate (A.3.30), isoflucypram (A.3.31), 2-(difluoromethyl)-*N*-(1,1,3-trimethyl-indan-4-yl)-pyridine-3-carboxamide (A.3.32), 2-(difluoromethyl)-*N*-[(3*R*)-1,1,3-trimethylindan-4-yl]-pyridine-3-carboxamide (A.3.33), 2-(difluoromethyl)-*N*-(3-ethyl-1,1-dimethyl-indan-4-yl)-pyridine-3-carboxamide (A.3.34), 2-(difluoromethyl)-*N*-[(3*R*)-3-ethyl-1,1-dimethyl-indan-4-yl]-pyridine-3-carboxamide (A.3.35), 2-(difluoromethyl)-*N*-(1,1-dimethyl-3-propyl-indan-4-yl)pyridine-3-carboxamide (A.3.36), 2-(difluoromethyl)-*N*-[(3*R*)-1,1-dimethyl-3-propyl-indan-4-yl]-pyridine-3-carboxamide (A.3.37), 2-(difluoromethyl)-*N*-(3-isobutyl-1,1-dimethyl-indan-4-yl)-pyridine-3-carboxamide (A.3.38), 2-(difluoromethyl)-*N*-[(3R)-3-isobutyl-1,1-dimethyl-indan-4-yl]pyridine-3-carboxamide (A.3.39);
   - other respiration inhibitors: diflumetorim (A.4.1); nitrophenyl derivates: binapacryl (A.4.2), dinobuton (A.4.3), dinocap (A.4.4), fluazinam (A.4.5), meptyldinocap (A.4.6), ferimzone (A.4.7); organometal compounds: fentin salts, e. g. fentin-acetate (A.4.8), fentin chloride (A.4.9) or fentin hydroxide (A.4.10); ametoctradin (A.4.11); silthiofam (A.4.12);
B) Sterol biosynthesis inhibitors (SBI fungicides)
   - C14 demethylase inhibitors: triazoles: azaconazole (B.1.1), bitertanol (B.1.2), bromuconazole (B.1.3), cyproconazole (B.1.4), difenoconazole (B.1.5), diniconazole (B.1.6), diniconazole-M (B.1.7), epoxiconazole (B.1.8), fenbuconazole (B.1.9), fluquinconazole (B.1.10), flusilazole (B.1.11), flutriafol (B.1.12), hexaconazole (B.1.13), imibenconazole (B.1.14), ipconazole (B.1.15), metconazole (B.1.17), myclobutanil (B.1.18), oxpoconazole (B.1.19), paclobutrazole (B.1.20), penconazole (B.1.21), propiconazole (B.1.22), prothioconazole (B.1.23), simeconazole (B.1.24), tebuconazole (B.1.25), tetraconazole (B.1.26), triadimefon (B.1.27), triadimenol (B.1.28), triticonazole (B.1.29), uniconazole (B.1.30), 2-(2,4-difluorophenyl)-1,1-difluoro-3-(tetrazol-1-yl)-1-[5-[4-(2,2,2-trifluoroethoxy)phenyl]-2-pyridyl]propan-2-ol (B.1.31), 2-(2,4-difluorophenyl)-1,1-difluoro-3-(tetrazol-1-yl)-1-[5-[4-(trifluoromethoxy)-phenyl]-2-pyridyl]propan-2-ol (B.1.32), 4-[[6-[2-(2,4-difluorophenyl)-1,1-difluoro-2-hydroxy-3-(5-sulfanyl-1,2,4-triazol-1-yl)propyl]-3-pyridyl]oxy]benzonitrile (B.1.33), ipfentrifluconazole (B.1.37), mefentrifluconazole (B.1.38), (2*R*)-2-[4-(4-chlorophenoxy)-2-(trifluoromethyl)phenyl]-1-(1,2,4-triazol-1-yl)propan-2-ol, (2S)-2-[4-(4-chlorophenoxy)-2-(trifluoromethyl)phenyl]-1-(1,2,4-triazol-1-yl)propan-2-ol, 2-(chloromethyl)-2-methyl-5-(p-tolylmethyl)-1-(1,2,4-triazol-1-ylmethyl)cyclopentanol (B.1.43); imidazoles: imazalil (B.1.44), pefurazoate (B.1.45), prochloraz (B.1.46), triflumizol (B.1.47); pyrimidines, pyridines, piperazines: fenarimol (B.1.49), pyrifenox (B.1.50), triforine (B.1.51), [3-(4-chloro-2-fluoro-phenyl)-5-(2,4-difluorophenyl)isoxazol-4-yl]-(3-pyridyl)methanol (B.1.52), 4-[[6-[2-(2,4-difluorophenyl)-1,1-difluoro-2-hydroxy-3-(1,2,4-triazol-1-yl)propyl]-3-pyridyl]oxy]benzonitrile (B.1.53), 2-[6-(4-bromophenoxy)-2-(trifluoromethyl)-3-pyridyl]-1-(1,2,4-triazol-1-yl)propan-2-ol (B.1.54), 2-[6-(4-chlorophenoxy)-2-(trifluoromethyl)-3-pyridyl]-1-(1,2,4-triazol-1-yl)propan-2-ol (B.1.55);
   - Delta14-reductase inhibitors: aldimorph (B.2.1), dodemorph (B.2.2), dodemorph-acetate (B.2.3), fenpropimorph (B.2.4), tridemorph (B.2.5), fenpropidin (B.2.6), piperalin (B.2.7), spiroxamine (B.2.8);
   - Inhibitors of 3-keto reductase: fenhexamid (B.3.1);
   - Other Sterol biosynthesis inhibitors: chlorphenomizole (B.4.1);
C) Nucleic acid synthesis inhibitors
   - phenylamides or acyl amino acid fungicides: benalaxyl (C.1.1), benalaxyl-M (C.1.2), kiralaxyl (C.1.3), metalaxyl (C.1.4), metalaxyl-M (C.1.5), ofurace (C.1.6), oxadixyl (C.1.7);
   - other nucleic acid synthesis inhibitors: hymexazole (C.2.1), octhilinone (C.2.2), oxolinic acid (C.2.3), bupirimate (C.2.4), 5-fluorocytosine (C.2.5), 5-fluoro-2-(p-tolylmethoxy)pyrimidin-4-amine (C.2.6), 5-fluoro-2-(4-fluorophenylmethoxy)pyrimidin-4-amine (C.2.7), 5-fluoro-2-(4-chlorophenylmethoxy)pyrimidin-4 amine (C.2.8);
D) Inhibitors of cell division and cytoskeleton
   - tubulin inhibitors: benomyl (D.1.1), carbendazim (D.1.2), fuberidazole (D1.3), thiabendazole (D.1.4), thiophanate-methyl (D.1.5), pyridachlometyl (D.1.6), *N*-ethyl-2-[(3-ethynyl-8-methyl-6-quinolyl)oxy]butanamide (D.1.8), *N*-ethyl-2-[(3-ethynyl-8-methyl-6-quinolyl)oxy]-2-methylsulfanyl-acetamide (D.1.9), 2-[(3-ethynyl-8-methyl-6-quinolyl)oxy]-*N*-(2-fluoroethyl)butanamide (D.1.10), 2-[(3-ethynyl-8-methyl-6-quinolyl)oxy]-*N*-(2-fluoroethyl)-2-methoxy-acetamide (D.1.11), 2-[(3-ethynyl-8-methyl-6-quinolyl)oxy]-*N*-propyl-butanamide (D.1.12), 2-[(3-ethynyl-8-methyl-6-quinolyl)oxy]-2-methoxy-*N*-propyl-acetamide (D.1.13), 2-[(3-ethynyl-8-methyl-6-quinolyl)oxy]-2-methylsulfanyl-*N*-propyl-acetamide (D.1.14), 2-[(3-ethynyl-8-methyl-6-quinolyl)oxy]-*N*-(2-fluoroethyl)-2-methylsulfanyl-acetamide (D.1.15), 4-(2-bromo-4-fluoro-phenyl)-*N*-(2-chloro-6-fluoro-phenyl)-2,5-dimethyl-pyrazol-3-amine (D.1.16);
   - other cell division inhibitors: diethofencarb (D.2.1), ethaboxam (D.2.2), pencycuron (D.2.3), fluopicolide (D.2.4), zoxamide (D.2.5), metrafenone (D.2.6), pyriofenone (D.2.7), phenamacril (D.2.8);
E) Inhibitors of amino acid and protein synthesis
   - methionine synthesis inhibitors: cyprodinil (E.1.1), mepanipyrim (E.1.2), pyrimethanil (E.1.3);
   - protein synthesis inhibitors: blasticidin-S (E.2.1), kasugamycin (E.2.2), kasugamycin hydrochloride-hydrate (E.2.3), mildiomycin (E.2.4), streptomycin (E.2.5), oxytetracyclin (E.2.6);
F) Signal transduction inhibitors
   - MAP / histidine kinase inhibitors: fluoroimid (F.1.1), iprodione (F.1.2), procymidone (F.1.3), vinclozolin (F.1.4), fludioxonil (F.1.5);
   - G protein inhibitors: quinoxyfen (F.2.1);
G) Lipid and membrane synthesis inhibitors
   - Phospholipid biosynthesis inhibitors: edifenphos (G.1.1), iprobenfos (G.1.2), pyrazophos (G.1.3), isoprothiolane (G.1.4);
   - lipid peroxidation: dicloran (G.2.1), quintozene (G.2.2), tecnazene (G.2.3), tolclofos-methyl (G.2.4), biphenyl (G.2.5), chloroneb (G.2.6), etridiazole (G.2.7), zinc thiazole (G.2.8);
   - phospholipid biosynthesis and cell wall deposition: dimethomorph (G.3.1), flumorph (G.3.2), mandipropamid (G.3.3), pyrimorph (G.3.4), benthiavalicarb (G.3.5), iprovalicarb (G.3.6), valifenalate (G.3.7);
   - compounds affecting cell membrane permeability and fatty acides: propamocarb (G.4.1);
   - inhibitors of oxysterol binding protein: oxathiapiprolin (G.5.1), fluoxapiprolin (G.5.3), 4-[1-[2-[3-(difluoromethyl)-5-methyl-pyrazol-1-yl]acetyl]-4-piperidyl]-*N*-tetralin-1-yl-pyridine-2-carboxamide (G.5.4), 4-[1-[2-[3,5-bis(difluoromethyl)pyrazol-1-yl]acetyl]-4-piperidyl]-*N-*tetralin-1-yl-pyridine-2-carboxamide (G.5.5), 4-[1-[2-[3-(difluoromethyl)-5-(trifluoromethyl)pyrazol-1-yl]acetyl]-4-piperidyl]-*N*-tetralin-1-yl-pyridine-2-carboxamide (G.5.6), 4-[1-[2-[5-cyclopropyl-3-(difluoromethyl)pyrazol-1-yl]acetyl]-4-piperidyl]-*N*-tetralin-1-yl-pyridine-2-carboxamide (G.5.7), 4-[1-[2-[5-methyl-3-(trifluoromethyl)pyrazol-1-yl]acetyl]-4-piperidyl]-*N*-tetralin-1-yl-pyridine-2-carboxamide (G.5.8), 4-[1-[2-[5-(difluoromethyl)-3-(trifluoromethyl)pyrazol-1-yl]acetyl]-4-piperidyl]-*N*-tetralin-1-yl-pyridine-2-carboxamide (G.5.9), 4-[1-[2-[3,5-bis(trifluoromethyl)pyrazol-1-yl]acetyl]-4-piperidyl]-*N*-tetralin-1-yl-pyridine-2-carboxamide (G.5.10), (4-[1-[2-[5-cyclopropyl-3-(trifluoromethyl)pyrazol-1-yl]acetyl]-4-piperidyl]-*N*-tetralin-1-yl-pyridine-2-carboxamide (G.5.11);
H) Inhibitors with Multi Site Action
   - inorganic active substances: Bordeaux mixture (H.1.1), copper (H.1.2), copper acetate (H.1.3), copper hydroxide (H.1.4), copper oxychloride (H.1.5), basic copper sulfate (H.1.6), sulfur (H.1.7);
   - thio- and dithiocarbamates: ferbam (H.2.1), mancozeb (H.2.2), maneb (H.2.3), metam (H.2.4), metiram (H.2.5), propineb (H.2.6), thiram (H.2.7), zineb (H.2.8), ziram (H.2.9);
   - organochlorine compounds: anilazine (H.3.1), chlorothalonil (H.3.2), captafol (H.3.3), captan (H.3.4), folpet (H.3.5), dichlofluanid (H.3.6), dichlorophen (H.3.7), hexachlorobenzene (H.3.8), pentachlorphenole (H.3.9) and its salts, phthalide (H.3.10), tolylfluanid (H.3.11);
   - guanidines and others: guanidine (H.4.1), dodine (H.4.2), dodine free base (H.4.3), guazatine (H.4.4), guazatine-acetate (H.4.5), iminoctadine (H.4.6), iminoctadine-triacetate (H.4.7), iminoctadine-tris(albesilate) (H.4.8), dithianon (H.4.9), 2,6-dimethyl-1*H*,5*H*-[1,4]dithiino[2,3-c:5,6-c']dipyrrole-1,3,5,7(2*H*,6*H*)-tetraone (H.4.10);
I) Cell wall synthesis inhibitors
   - inhibitors of glucan synthesis: validamycin (I.1.1), polyoxin B (I.1.2);
   - melanin synthesis inhibitors: pyroquilon (I.2.1), tricyclazole (I.2.2), carpropamid (I.2.3), dicyclomet (I.2.4), fenoxanil (I.2.5);
J) Plant defence inducers
   - acibenzolar-S-methyl (J.1.1), probenazole (J.1.2), isotianil (J.1.3), tiadinil (J.1.4), prohexadione-calcium (J.1.5); phosphonates: fosetyl (J.1.6), fosetyl-aluminum (J.1.7), phosphorous acid and its salts (J.1.8), calcium phosphonate (J.1.11), potassium phosphonate (J.1.12), potassium or sodium bicarbonate (J.1.9), 4-cyclopropyl-*N*-(2,4-dimethoxyphenyl)thiadiazole-5-carboxamide (J.1.10);
K) Unknown mode of action
   - bronopol (K.1.1), chinomethionat (K.1.2), cyflufenamid (K.1.3), cymoxanil (K.1.4), dazomet (K.1.5), debacarb (K.1.6), diclocymet (K.1.7), diclomezine (K.1.8), difenzoquat (K.1.9), difenzoquat-methylsulfate (K.1.10), diphenylamin (K.1.11), fenitropan (K.1.12), fenpyrazamine (K.1.13), flumetover (K.1.14), flusulfamide (K.1.15), flutianil (K.1.16), harpin (K.1.17), methasulfocarb (K.1.18), nitrapyrin (K.1.19), nitrothal-isopropyl (K.1.20), tolprocarb (K.1.21), oxincopper (K.1.22), proquinazid (K.1.23), tebufloquin (K.1.24), tecloftalam (K.1.25), triazoxide (K.1.26), *N*'-(4-(4-chloro-3-trifluoromethyl-phenoxy)-2,5-dimethyl-phenyl)-*N*-ethyl-*N*-methyl formamidine (K.1.27), *N*'-(4-(4-fluoro-3-trifluoromethyl-phenoxy)-2,5-dimethyl-phenyl)-*N*-ethyl-*N*-methyl formamidine (K.1.28), *N'*-[4-[[3-[(4-chlorophenyl)methyl]-1,2,4-thiadiazol-5-yl]-oxy]-2,5-dimethyl-phenyl]-*N*-ethyl-*N*-methyl-formamidine (K.1.29), *N'*-(5-bromo-6-indan-2-yloxy-2-methyl-3-pyridyl)-*N*-ethyl-*N*-methyl-formamidine (K.1.30), *N'*-[5-bromo-6-[1-(3,5-difluorophenyl)ethoxy]-2-methyl-3-pyridyl]-*N*-ethyl-*N*-methyl-formamidine (K.1.31), *N'*-[5-bromo-6-(4-isopropylcyclohexoxy)-2-methyl-3-pyridyl]-*N*-ethyl-*N*-methyl-formamidine (K.1.32), *N'*-[5-bromo-2-methyl-6-(1-phenylethoxy)-3-pyridyl]-*N*-ethyl-*N*-methyl-formamidine (K.1.33), *N'*-(2-methyl-5-trifluoromethyl-4-(3-trimethylsilanyl-propoxy)-phenyl)-*N*-ethyl-*N*-methyl formamidine (K.1.34), *N'*-(5-difluoromethyl-2-methyl-4-(3-trimethylsilanyl-propoxy)-phenyl)-*N*-ethyl-*N*-methyl formamidine (K.1.35), 2-(4-chloro-phenyl)-*N*-[4-(3,4-dimethoxy-phenyl)-isoxazol-5-yl]-2-prop-2-ynyloxy-acetamide (K.1.36), 3-[5-(4-chloro-phenyl)-2,3-dimethyl-isoxazolidin-3-yl]-pyridine (pyrisoxazole) (K.1.37), 3-[5-(4-methylphenyl)-2,3-dimethyl-isoxazolidin-3 yl]-pyridine (K.1.38), 5-chloro-1-(4,6-dimethoxy-pyrimidin-2-yl)-2-methyl-1*H*-benzoimidazole (K.1.39), ethyl (Z)-3-amino-2-cyano-3-phenyl-prop-2-enoate (K.1.40), picarbutrazox (K.1.41), pentyl *N*-[6-[[(Z)-[(1-methyltetrazol-5-yl)-phenyl-methylene]amino]oxymethyl]-2-pyridyl]carbamate (K.1.42), but-3-ynyl *N*-[6-[[(*Z*)-[(1-methyltetrazol-5-yl)-phenyl-methylene]amino]oxymethyl]-2-pyridyl]carbamate (K.1.43), ipflufenoquin (K.1.44), quinofumelin (K.1.47), benziothiazolinone (K.1.48), bromothalonil (K.1.49), 2-(6-benzyl-2-pyridyl)quinazoline (K.1.50), 2-[6-(3-fluoro-4-methoxy-phenyl)-5-methyl-2-pyridyl]quinazoline (K.1.51), dichlobentiazox (K.1.52), *N*'-(2,5-dimethyl-4-phenoxy-phenyl)-*N*-ethyl-*N*-methyl-formamidine (K.1.53), aminopyrifen (K.1.54), fluopimomide (K.1.55), *N*'-[5-bromo-2-methyl-6-(1-methyl-2-propoxy-ethoxy)-3-pyridyl]-N-ethyl-N-methyl-formamidine (K.1.56), N'-[4-(4,5-dichlorothiazol-2-yl)oxy-2,5-dimethyl-phenyl]-N-ethyl-N-methyl-formamidine (K.1.57), N-(2-fluorophenyl)-4-[5-(trifluoromethyl)-1,2,4-oxadiazol-3-yl]benzamide (K.1.58), N-methyl-4-[5-(trifluoromethyl)-1,2,4-oxadiazol-3-yl]benzenecarbothioamide (K.1.59);
L) Biopesticides
   L1) Microbial pesticides with fungicidal, bactericidal, viricidal and/or plant defense activator activity: *Ampelomyces quisqualis*, *Aspergillus flavus*, *Aureobasidium pullulans*, *Bacillus altitudinis*, *B. amyloliquefaciens*, *B. amyloliquefaciens* ssp. *plantarum* (also referred to as *B*. *velezensis*), *B. megaterium*, *B. mojavensis, B. mycoides*, *B. pumilus*, *B. simplex*, *B. solisalsi*, *B. subtilis*, *B. subtilis* var. *amyloliquefaciens*, *B. velezensis*, *Candida oleophila*, *C*. *saitoana*, *Clavibacter michiganensis* (bacteriophages), *Coniothyrium minitans, Cryphonectria parasitica*, *Cryptococcus albidus*, *Dilophosphora alopecuri*, *Fusarium oxysporum*, *Clonostachys* rosea f. *catenulate* (also named *Gliocladium catenulatum*), *Gliocladium roseum*, *Lysobacter antibioticus*, *L. enzymogenes*, *Metschnikowia fructicola*, *Microdochium dimerum*, *Microsphaeropsis ochracea*, *Muscodor albus*, *Paenibacillus alvei*, *Paenibacillus epiphyticus*, *P. polymyxa*, *Pantoea vagans*, *Penicillium bilaiae*, *Phlebiopsis gigantea*, *Pseudomonas* sp., *Pseudomonas chloraphis*, *Pseudozyma flocculosa*, *Pichia anomala*, *Pythium oligandrum*, *Sphaerodes mycoparasitica*, *Streptomyces griseoviridis*, *S. lydicus*, *S. violaceusniger*, *Talaromyces flavus*, *Trichoderma asperelloides*, *T. asperellum*, *T. atroviride*, *T. fertile*, *T. gamsii*, *T. harmatum, T. harzianum*, *T. polysporum*, *T. stromaticum*, *T. virens*, *T. viride*, *Typhula phacorrhiza*, *Ulocladium oudemansii*, *Verticillium dahlia*, zucchini yellow mosaic virus (avirulent strain);
   L2) Biochemical pesticides with fungicidal, bactericidal, viricidal and/or plant defense activator activity: harpin protein, *Reynoutria sachalinensis* extract;
   L3) Microbial pesticides with insecticidal, acaricidal, molluscidal and/or nematicidal activity: *Agrobacterium radiobacter*, *Bacillus cereus*, *B. firmus*, *B. thuringiensis*, *B. thuringiensis* ssp. *aizawai*, *B. t.* ssp. *israelensis*, *B. t.* ssp. *galleriae*, *B. t.* ssp. *kurstaki*, *B. t.* ssp. *tenebrionis*, *Beauveria bassiana*, *B. brongniartii*, *Burkholderia* spp., *Chromobacterium subtsugae*, *Cydia pomonella* granulovirus (CpGV), *Cryptophlebia leucotreta* granulovirus (CrleGV), *Flavobacterium* spp., *Helicoverpa armigera* nucleopolyhedrovirus (HearNPV), *Helicoverpa zea* nucleopolyhedrovirus (HzNPV), *Helicoverpa zea* single capsid nucleopolyhedrovirus (HzSNPV), *Heterorhabditis bacteriophora*, *Isaria fumosorosea*, *Lecanicillium longisporum*, *L. muscarium*, *Metarhizium anisopliae*, *M. anisopliae* var. *anisopliae*, *M. anisopliae* var. *acridum*, *Nomuraea rileyi*, *Paecilomyces fumosoroseus*, *P. lilacinus*, *Paenibacillus popilliae*, *Pasteuria* spp., *P. nishizawae*, *P. penetrans*, *P*. *ramosa*, *P. thornea*, *P. usgae*, *Pseudomonas fluorescens*, *Spodoptera littoralis* nucleopolyhedrovirus (SpliNPV), *Steinernema carpocapsae*, *S. feltiae*, *S. kraussei*, *Streptomyces galbus*, *S. microflavus*;
   L4) Biochemical pesticides with insecticidal, acaricidal, molluscidal, pheromone and/or nematicidal activity: L-carvone, citral, (*E*,*Z*)-7,9-dodecadien-1-yl acetate, ethyl formate, (*E*,*Z*)-2,4-ethyl decadienoate (pear ester), (*Z*,*Z*,*E*)-7,11,13-hexadecatrienal, heptyl butyrate, isopropyl myristate, lavanulyl senecioate, cis-jasmone, 2-methyl 1-butanol, methyl eugenol, methyl jasmonate, (*E*,*Z*)-2,13-octadecadien-1-ol, (*E*,*Z*)-2,13-octadecadien-1-ol acetate, (*E*,*Z*)-3,13-octadecadien-1-ol, (*R*)-1-octen-3-ol, pentatermanone, (*E*,*Z*,*Z*)-3,8,11-tetradecatrienyl acetate, (*Z*,*E*)-9,12-tetradecadien-1-yl acetate, (Z)-7-tetradecen-2-one, (Z)-9-tetradecen-1-yl acetate, (Z)-11-tetradecenal, (*Z*)-11-tetradecen-1-ol, extract of *Chenopodium ambrosiodes,* Neem oil, Quillay extract;
   L5) Microbial pesticides with plant stress reducing, plant growth regulator, plant growth promoting and/or yield enhancing activity: *Azospirillum amazonense*, *A. brasilense*, *A. lipoferum*, *A. irakense*, *A. halopraeferens*, *Bradyrhizobium* spp., *B. elkanii*, *B. japonicum*, *B. liaoningense*, *B. lupini*, *Delftia acidovorans*, *Glomus intraradices*, *Mesorhizobium* spp., *Rhizobium leguminosarum* bv. *phaseoli*, *R. I.* bv. *trifolii*, *R. I.* bv. *viciae*, *R. tropici*, *Sinorhizobium meliloti*;
O) Insecticides from classes O.1 to O.29
   O.1 Acetylcholine esterase (AChE) inhibitors: aldicarb, alanycarb, bendiocarb, benfuracarb, butocarboxim, butoxycarboxim, carbaryl, carbofuran, carbosulfan, ethiofencarb, fenobucarb, formetanate, furathiocarb, isoprocarb, methiocarb, methomyl, metolcarb, oxamyl, pirimicarb, propoxur, thiodicarb, thiofanox, trimethacarb, XMC, xylylcarb, triazamate; acephate, azamethiphos, azinphos-ethyl, azinphosmethyl, cadusafos, chlorethoxyfos, chlorfenvinphos, chlormephos, chlorpyrifos, chlorpyrifos-methyl, coumaphos, cyanophos, demeton-S-methyl, diazinon, dichlorvos/ DDVP, dicrotophos, dimethoate, dimethylvinphos, disulfoton, EPN, ethion, ethoprophos, famphur, fenamiphos, fenitrothion, fenthion, fosthiazate, heptenophos, imicyafos, isofenphos, isopropyl O-(methoxyaminothio-phosphoryl) salicylate, isoxathion, malathion, mecarbam, methamidophos, methidathion, mevinphos, monocrotophos, naled, omethoate, oxydemeton-methyl, parathion, parathion-methyl, phenthoate, phorate, phosalone, phosmet, phosphamidon, phoxim, pirimiphos- methyl, profenofos, propetamphos, prothiofos, pyraclofos, pyridaphenthion, quinalphos, sulfotep, tebupirimfos, temephos, terbufos, tetrachlorvinphos, thiometon, triazophos, trichlorfon, vamidothion;
   O.2 GABA-gated chloride channel antagonists: endosulfan, chlordane; ethiprole, fipronil, flufiprole, pyrafluprole, pyriprole;
   O.3 Sodium channel modulators: acrinathrin, allethrin, d-cis-trans allethrin, d-trans allethrin, bifenthrin, kappa-bifenthrin, bioallethrin, bioallethrin S-cylclopentenyl, bioresmethrin, cycloprothrin, cyfluthrin, beta-cyfluthrin, cyhalothrin, lambda-cyhalothrin, gamma-cyhalothrin, cypermethrin, alpha-cypermethrin, beta-cypermethrin, theta-cypermethrin, zetacypermethrin, cyphenothrin, deltamethrin, empenthrin, esfenvalerate, etofenprox, fenpropathrin, fenvalerate, flucythrinate, flumethrin, tau-fluvalinate, halfenprox, heptafluthrin, imiprothrin, meperfluthrin, metofluthrin, momfluorothrin, epsilon-momfluorothrin, permethrin, phenothrin, prallethrin, profluthrin, pyrethrin (pyrethrum), resmethrin, silafluofen, tefluthrin, kappa-tefluthrin, tetramethylfluthrin, tetramethrin, tralomethrin, transfluthrin; DDT, methoxychlor;
   O.4 Nicotinic acetylcholine receptor (nAChR) agonists: acetamiprid, clothianidin, cycloxaprid, dinotefuran, imidacloprid, nitenpyram, thiacloprid, thiamethoxam; 4,5-dihydro-*N*-nitro-1-(2-oxiranylmethyl)-1*H*-imidazol-2-amine, (2*E*)-1-[(6-chloropyridin-3-yl)methyl]-*N*-nitro-2-pentylidenehydrazinecarboximidamide; 1-[(6-chloropyridin-3-yl)methyl]-7-methyl-8-nitro-5-propoxy-1,2,3,5,6,7-hexahydroimidazo[1,2-a]pyridine; nicotine; sulfoxaflor, flupyradifurone, triflumezopyrim, (3*R*)-3-(2-chlorothiazol-5-yl)-8-methyl-5-oxo-6-phenyl-2,3-dihydrothiazolo-[3,2-a]pyrimidin-8-ium-7-olate, (3*S*)-3-(6-chloro-3-pyridyl)-8-methyl-5-oxo-6-phenyl-2,3-dihydrothiazolo[3,2-a]pyrimidin-8-ium-7-olate, (3*S*)-8-methyl-5-oxo-6-phenyl-3-pyrimidin-5-yl-2,3-dihydrothiazolo[3,2-a]pyrimidin-8-ium-7-olate, (3*R*)-3-(2-chlorothiazol-5-yl)-8-methyl-5-oxo-6-[3-(trifluoromethyl)phenyl]-2,3-dihydrothiazolo[3,2-a]pyrimidin-8-ium-7-olate; (3*R*)-3-(2-chlorothiazol-5-yl)-6-(3,5-dichlorophenyl)-8-methyl-5-oxo-2,3-dihydrothiazolo-[3,2-a]pyrimidin-8-ium-7-olate, (3*R*)-3-(2-chlorothiazol-5-yl)-8-ethyl-5-oxo-6-phenyl-2,3-dihydrothiazolo[3,2-a]pyrimidin-8-ium-7-olate;
   O.5 Nicotinic acetylcholine receptor allosteric activators: spinosad, spinetoram;
   O.6 Chloride channel activators: abamectin, emamectin benzoate, ivermectin, lepimectin, milbemectin;
   O.7 Juvenile hormone mimics: hydroprene, kinoprene, methoprene; fenoxycarb, pyriproxyfen;
   O.8 miscellaneous non-specific (multi-site) inhibitors: methyl bromide and other alkyl halides; chloropicrin, sulfuryl fluoride, borax, tartar emetic;
   O.9 Chordotonal organ TRPV channel modulators: pymetrozine, pyrifluquinazon;
   O.10 Mite growth inhibitors: clofentezine, hexythiazox, diflovidazin; etoxazole;
   O.11 Microbial disruptors of insect midgut membranes: *Bacillus thuringiensis*, *Bacillus sphaericus* and the insecticdal proteins they produce: *Bacillus thuringiensis* subsp. *israelensis*, *Bacillus sphaericus*, *Bacillus thuringiensis* subsp. *aizawai, Bacillus thuringiensis* subsp. *kurstaki*, *Bacillus thuringiensis* subsp. *tenebrionis*, the Bt crop proteins: Cry1Ab, Cry1Ac, Cry1Fa, Cry2Ab, mCry3A, Cry3Ab, Cry3Bb, Cry34/35Ab1;
   0.12 Inhibitors of mitochondrial ATP synthase: diafenthiuron; azocyclotin, cyhexatin, fenbutatin oxide, propargite, tetradifon;
   0.13 Uncouplers of oxidative phosphorylation via disruption of the proton gradient: chlorfenapyr, DNOC, sulfluramid;
   0.14 Nicotinic acetylcholine receptor (nAChR) channel blockers: bensultap, cartap hydrochloride, thiocyclam, thiosultap sodium;
   0.15 Inhibitors of the chitin biosynthesis type 0: bistrifluron, chlorfluazuron, diflubenzuron, flucycloxuron, flufenoxuron, hexaflumuron, lufenuron, novaluron, noviflumuron, teflubenzuron, triflumuron;
   0.16 Inhibitors of the chitin biosynthesis type 1: buprofezin;
   0.17 Moulting disruptors: cyromazine;
   0.18 Ecdyson receptor agonists: methoxyfenozide, tebufenozide, halofenozide, fufenozide, chromafenozide;
   0.19 Octopamin receptor agonists: amitraz;
   O.20 Mitochondrial complex III electron transport inhibitors: hydramethylnon, acequinocyl, fluacrypyrim, bifenazate;
   0.21 Mitochondrial complex I electron transport inhibitors: fenazaquin, fenpyroximate, pyrimidifen, pyridaben, tebufenpyrad, tolfenpyrad; rotenone;
   O.22 Voltage-dependent sodium channel blockers: indoxacarb, metaflumizone, 2-[2-(4-cyanophenyl)-1-[3-(trifluoromethyl)phenyl]ethylidene]-*N*-[4-(difluoromethoxy)phenyl]-hydrazinecarboxamide, *N*-(3-chloro-2-methylphenyl)-2-[(4-chlorophenyl)-[4-[methyl(methylsulfonyl)-amino]phenyl]methylene]-hydrazinecarboxamide;
   O.23 Inhibitors of the of acetyl CoA carboxylase: spirodiclofen, spiromesifen, spirotetramat, spiropidion;
   O.24 Mitochondrial complex IV electron transport inhibitors: aluminium phosphide, calcium phosphide, phosphine, zinc phosphide, cyanide;
   O.25 Mitochondrial complex II electron transport inhibitors: cyenopyrafen, cyflumetofen;
   O.26 Ryanodine receptor-modulators: flubendiamide, chlorantraniliprole, cyantraniliprole, cyclaniliprole, tetraniliprole; (R)-3-chloro-*N*¹-{2-methyl-4-[1,2,2,2-tetrafluoro-1-(trifluoromethyl)-ethyl]phenyl}-*N*²-(1-methyl-2-methylsulfonylethyl)phthalamide, (*S*)-3-chloro-*N*¹-{2-methyl-4-[1,2,2,2-tetrafluoro-1-(trifluoromethyl)ethyl]phenyl}-*N*²-(1-methyl-2-methylsulfonylethyl)-phthalamide, methyl-2-[3,5-dibromo-2-({[3-bromo-1-(3-chloropyridin-2-yl)-1*H*-pyrazol-5-yl]-carbonyl}amino)benzoyl]-1,2-dimethylhydrazinecarboxylate; *N*-[4,6-dichioro-2-[(diethyllambda-4-sulfanylidene)carbamoyl]-phenyl]-2-(3-chloro-2-pyridyl)-5-(trifluoromethyl)pyrazole-3-carboxamide; *N*-[4-chloro-2-[(diethyl-lambda-4-sulfanylidene)carbamoyl]-6-methyl-phenyl]-2-(3-chloro-2-pyridyl)-5-(trifluoromethyl)pyrazole-3-carboxamide; *N*-[4-chloro-2-[(di-2-propyllambda-4-sulfanylidene)carbamoyl]-6-methyl-phenyl]-2-(3-chloro-2-pyridyl)-5-(trifluoromethyl)pyrazole-3-carboxamide; *N*-[4,6-dichloro-2-[(di-2-propyl-lambda-4-sulfanylidene)carbamoyl]-phenyl]-2-(3-chloro-2-pyridyl)-5-(trif*luoromethyl)pyrazole-3-carboxamide; *N*-[4,6-dibromo-2-[(diethyl-lambda-4-sulfanylidene)carbamoyl]-phenyl]-2-(3-chloro-2-pyridyl)-5-(trifluoromethyl)pyrazole-3-carboxamide; *N*-[2-(5-amino-1,3,4-thiadiazol-2-yl)-4-chloro-6-methylphenyl]-3-bromo-1-(3-chloro-2-pyridinyl)-1*H*-pyrazole-5-carboxamide; 3-chloro-1-(3-chloro-2-pyhdinyl)-*N*-[2,4-dichloro-6-[[(1-cyano-1-methylethyl)amino]carbonyl]phenyl]-1*H*-pyrazole-5-carboxamide; tetrachlorantraniliprole; *N*-[4-chloro-2-[[(1,1-dimethylethyl)amino]carbonyl]-6-methylphenyl]-1-(3-chloro-2-pyridinyl)-3-(fluoromethoxy)-1*H*-pyrazole-5-carboxamide; cyhalodiamide;
   O.27: Chordotonal organ modulators - undefined target site: flonicamid;
   O.28. insecticidal compounds of unknown or uncertain mode of action: afidopyropen, afoxolaner, azadirachtin, amidoflumet, benzoximate, broflanilide, bromopropylate, chinomethionat, cryolite, dicloromezotiaz, dicofol, flufenerim, flometoquin, fluensulfone, fluhexafon, fluopyram, fluralaner, metoxadiazone, piperonyl butoxide, pyflubumide, pyridalyl, tioxazafen, 11-(4-chloro-2,6-dimethylphenyl)-12-hydroxy-1,4-dioxa-9-azadispiro[4.2.4.2]-tetradec-11-en-10-one, 3-(4'-fluoro-2,4-dimethylbiphenyl-3-yl)-4-hydroxy-8-oxa-1-azaspiro[4.5]dec-3-en-2-one, 1-[2-fluoro-4-methyl-5-[(2,2,2-trifluoroethyl)sulfinyl]phenyl]-3-(trifluoromethyl)-1*H*-1,2,4-triazole-5-amine, *Bacillus firmus* I-1582; flupyrimin; fluazaindolizine; 4-[5-(3,5-dichlorophenyl)-5-(trifluoromethyl)-4H-isoxazol-3-yl]-2-methyl-N-(1-oxothietan-3-yl)benzamide; fluxametamide; 5-[3-[2,6-dichloro-4-(3,3-dichloroallyloxy)phenoxy]propoxy]-1*H*-pyrazole; 4-cyano-*N*-[2-cyano-5-[[2,6-dibromo-4-[1,2,2,3,3, 3-hexafluoro-1-(trifluoromethyl)propyl]phenyl]carbamoyl]phenyl]-2-methyl-benzamide; 4-cyano-3-[(4-cyano-2-methyl-benzoyl)amino]-*N*-[2,6-dichloro-4-[1,2,2,3,3,3-hexafluoro-1-(trifluoromethyl)propyl]phenyl]-2-fluoro-benzamide; *N*-[5-[[2-chloro-6-cyano-4-[1,2,2,3,3,3-hexafluoro-1-(trifluoromethyl)propyl]phenyl]-carbamoyl]-2-cyano-phenyl]-4-cyano-2-methyl-benzamide; *N*-[5-[[2-bromo-6-chloro-4-[2,2,2-trifluoro-1-hydroxy-1-(trifluoromethyl)ethyl]phenyl]carbamoyl]-2-cyano-phenyl]-4-cyano-2-methyl-benzamide; *N*-[5-[[2-bromo-6-chloro-4-[1,2,2,3,3,3-hexafluoro-1-(trifluoromethyl)propyl]phenyl]carbamoyl]-2-cyano-phenyl]-4-cyano-2-methyl-benzamide; 4-cyano-*N*-[2-cyano-5-[[2,6-dichloro-4-[1,2,2,3,3,3-hexafluoro-1-(trifluoromethyl)propyl]phenyl]carbamoyl]phenyl]-2-methyl-benzamide; 4-cyano-*N*-[2-cyano-5-[[2,6-dichloro-4-[1,2,2,2-tetrafluoro-1-(trifluoromethyl)ethyl]phenyl]carbamoyl]phenyl]-2-methyl-benzamide; *N*-[5-[[2-bromo-6-chloro-4-[1,2,2,2-tetrafluoro-1-(trifluoromethyl)ethyl]phenyl]carbamoyl]-2-cyano-phenyl]-4-cyano-2-methyl-benzamide; 2-(1,3-dioxan-2-yl)-6-[2-(3-pyridinyl)-5-thiazolyl]-pyridine; 2-[6-[2-(5-fluoro-3-pyridinyl)-5-thiazolyl]-2-pyridinyl]-pyrimidine; 2-[6-[2-(3-pyridinyl)-5-thiazolyl]-2-pyridinyl]-pyrimidine; *N*-methylsulfonyl-6-[2-(3-pyridyl)thiazol-5-yl]pyridine-2-carboxamide; *N*-methylsulfonyl-6-[2-(3-pyridyl)thiazol-5-yl]pyridine-2-carboxamide; 1-[(6-chloro-3-pyridinyl)methyl]-1,2,3,5,6,7-hexahydro-5-methoxy-7-methyl-8-nitro-imidazo[1,2-a]pyridine; 1-[(6-chloropyridin-3-yl)methyl]-7-methyl-8-nitro-1,2,3,5,6,7-hexahydroimidazo[1,2-a]pyridin-5-ol; 1-isopropyl-*N*,5-dimethyl-*N*-pyridazin-4-yl-pyrazole-4-carboxamide; 1-(1,2-dimethylpropyl)-*N*-ethyl-5-methyl-*N*-pyridazin-4-yl-pyrazole-4-carboxamide; *N*,5-dimethyl-*N*-pyridazin-4-yl-1-(2,2,2-trifluoro-1-methyl-ethyl)pyrazole-4-carboxamide; 1-[1-(1-cyanocyclopropyl)ethyl]-*N*-ethyl-5-methyl-*N*-pyridazin-4-yl-pyrazole-4-carboxamide; *N*-ethyl-1-(2-fluoro-1-methylpropyl)-5-meth-yl-*N*-pyridazin-4-yl-pyrazole-4-carboxamide; 1-(1,2-dimethylpropyl)-*N*,5-dimethyl-*N*-pyridazin-4-yl-pyrazole-4-carboxamide; 1-[1-(1-cyanocyclopropyl)ethyl]-*N*,5-dimethyl-*N*-pyridazin-4-yl-pyrazole-4-carboxamide; *N*-methyl-1-(2-fluoro-1-methyl-propyl]-5-methyl-*N*-pyridazin-4-yl-pyrazole-4-carboxamide; 1-(4,4-difluorocyclohexyl)-*N*-ethyl-5-methyl-*N*-pyridazin-4-yl-pyrazole-4-carboxamide; 1-(4,4-difluorocyclohexyl)-*N*,5-dimethyl-*N*-pyridazin-4-yl-pyrazole-4-carboxamide, *N*-(1-methylethyl)-2-(3-pyridinyl)-2*H*-indazole-4-carboxamide; *N*-cyclopropyl-2-(3-pyridinyl)-2*H*-indazole-4-carboxamide; *N*-cyclohexyl-2-(3-pyridinyl)-2*H*-indazole-4-carboxamide; 2-(3-pyridinyl)-*N*-(2,2,2-trifluoroethyl)-2*H*-indazole-4-carboxamide; 2-(3-pyridinyl)-*N*-[(tetrahydro-2-furanyl)methyl]-2*H*-indazole-5-carboxamide; methyl 2-[[2-(3-pyridinyl)-2*H*-indazol-5-yl]carbonyl]hydrazinecarboxylate; *N*-[(2,2-difluorocyclopropyl)methyl]-2-(3-pyridinyl)-2*H*-indazole-5-carboxamide; *N*-(2,2-difluoropropyl)-2-(3-pyridinyl)-2*H*-indazole-5-carboxamide; 2-(3-pyridinyl)-*N*-(2-pyrimidinylmethyl)-2*H*-indazole-5-carboxamide; *N*-[(5-methyl-2-pyrazinyl)methyl]-2-(3-pyridinyl)-2*H*-indazole-5-carboxamide, tyclopyrazoflor; sarolaner, lotilaner, *N*-[4-chloro-3-[[(phenylmethyl)amino]carbonyl]phenyl]-1-methyl-3-(1,1,2,2,2-pentafluoroethyl)-4-(trifluoromethyl)-1*H*-pyrazole-5-carboxamide; 2-(3-ethylsulfonyl-2-pyridyl)-3-methyl-6-(trifluoromethyl)imidazo[4,5-b]pyridine, 2-[3-ethylsulfonyl-5-(trifluoromethyl)-2-pyridyl]-3-methyl-6-(trifluoromethyl)imidazo[4,5-b]pyridine, isocycloseram, *N*-[4-chloro-3-(cyclopropylcarbamoyl)phenyl]-2-methyl-5-(1,1,2,2,2-pentafluoroethyl)-4-(trifluoromethyl)pyrazole-3-carboxamide, *N*-[4-chloro-3-[(1-cyanocyclopropyl)carbamoyl]phenyl]-2-methyl-5-(1,1,2,2,2-pentafluoroethyl)-4-(trifluoromethyl)-pyrazole-3-carboxamide; acynonapyr; benzpyrimoxan; tigolaner; chloro-*N*-(1-cyanocyclopropyl)-5-[1-[2-methyl-5-(1,1,2,2,2-pentafluoroethyl)-4-(trifluoromethyl)pyrazol-3-yl]pyrazol-4-yl]benzamide, oxazosulfyl, [(2*S*,3*R*,4*R*,5*S*,6*S*)-3,5-dimethoxy-6-methyl-4-propoxy-tetrahydropyran-2-yl]-*N*-[4-[1-[4-(trifluoromethoxy)phenyl]-1,2,4-triazol-3-yl]phenyl]carbamate, [(2S,3R,4R,5S,6S)-3,4,5-trimethoxy-6-methyl-tetrahydropyran-2-yl] N-[4-[1-[4-(trifluoromethoxy)phenyl]-1,2,4-triazol-3-yl]phenyl]carbamate, [(2*S*,3*R*,4*R*,5*S*,6*S*)-3,5-dimethoxy-6-methyl-4-propoxy-tetrahydropyran-2-yl]-*N*-[4-[1-[4-(1,1,2,2,2-pentafluoroethoxy)phenyl]-1,2,4-triazol-3-yl]phenyl]carbamate, [(2*S*,3*R*,4*R*,5*S*,6*S*)-3,4,5-trimethoxy-6-methyl-tetrahydropyran-2-yl]-*N*-[4-[1-[4-(1,1,2,2,2-pentafluoroethoxy)phenyl]-1,2,4-triazol-3-yl]phenyl]carbamate, (2*Z*)-3-(2-isopropylphenyl)-2-[(*E*)-[4-[1-[4-(1,1,2,2,2-pentafluoroethoxy)phenyl]-1,2,4-triazol-3-yl]phenyl]methylenehydrazono]thiazolidin-4-one; 2-(6-chloro-3-ethylsulfonyl-imidazo[1,2-a]pyridin-2-yl)-3-methyl-6-(trifluoromethyl)imidazo-[4,5-b]pyridine, 2-(6-bromo-3-ethylsulfonyl-imidazo[1,2-a]pyridin-2-yl)-3-methyl-6-(trifluoromethyl)imidazo[4,5-b]pyridine, 2-(3-ethylsulfonyl-6-iodo-imidazo[1,2-a]pyridin-2-yl)-3-methyl-6-(trifluoromethyl)imidazo[4,5-b]pyridine, 2-[3-ethylsulfonyl-6-(trifluoromethyl)imidazo-[1,2-a]pyridin-2-yl]-3-methyl-6-(trifluoromethyl)imidazo[4,5-b]pyridine, 2-(7-chloro-3-ethylsulfonyl-imidazo[1,2-a]pyridin-2-yl)-3-methyl-6-(trifluoromethyl)imidazo[4,5-b]pyridine, 2-(3-ethylsulfonyl-7-iodo-imidazo[1,2-a]pyridin-2-yl)-3-methyl-6-(trifluoromethyl)imidazo-[4,5-b]pyridine, 3-ethylsulfonyl-6-iodo-2-[3-methyl-6-(trifluoromethyl)imidazo[4,5-b]pyridin-2-yl]imidazo[1,2-a]pyridine-8-carbonitrile, 2-[3-ethylsulfonyl-8-fluoro-6-(trifluoromethyl)imidazo[1,2-a]pyridin-2-yl]-3-methyl-6-(trifluoromethyl)imidazo[4,5-b]pyridine, 2-[3-ethylsulfonyl-7-(trifluoromethyl)imidazo[1,2-a]pyridin-2-yl]-3-methyl-6-(trifluoromethylsulfinyl)imidazo-[4,5-b]pyridine, 2-[3-ethylsulfonyl-7-(trifluoromethyl)imidazo[1,2-a]pyridin-2-yl]-3-methyl-6-(trifluoromethyl)imidazo[4,5-c]pyridine, 2-(6-bromo-3-ethylsulfonyl-imidazo[1,2-a]pyridin-2-yl)-6-(trifluoromethyl)pyrazolo[4,3-c]pyridine.

The active substances referred to as component 2, their preparation and their activity e. g. against harmful fungi is known (cf.: http://www.alanwood.net/pesticides/); these substances are commercially available. The compounds described by IUPAC nomenclature, their preparation and their pesticidal activity are also known (cf. Can. J. Plant Sci. 48(6), 587-94, 1968; EP-A 141 317; EP-A 152 031; EP-A 226 917; EP-A 243 970; EP-A 256 503; EP-A 428 941; EP-A 532 022; EP-A 1 028 125; EP-A 1 035 122; EP-A 1 201 648; EP-A 1 122 244, JP 2002316902; DE 19650197; DE 10021412; DE 102005009458; US 3,296,272; US 3,325,503; WO 98/46608; WO 99/14187; WO 99/24413; WO 99/27783; WO 00/29404; WO 00/46148; WO 00/65913; WO 01/54501; WO 01/56358; WO 02/22583; WO 02/40431; WO 03/10149; WO 03/11853; WO 03/14103; WO 03/16286; WO 03/53145; WO 03/61388; WO 03/66609; WO 03/74491; WO 04/49804; WO 04/83193; WO 05/120234; WO 05/123689; WO 05/123690; WO 05/63721; WO 05/87772; WO 05/87773; WO 06/15866; WO 06/87325; WO 06/87343; WO 07/82098; WO 07/90624, WO 10/139271, WO 11/028657, WO 12/168188, WO 07/006670, WO 11/77514; WO 13/047749, WO 10/069882, WO 13/047441, WO 03/16303, WO 09/90181, WO 13/007767, WO 13/010862, WO 13/127704, WO 13/024009, WO 13/24010, WO 13/047441, WO 13/162072, WO 13/092224, WO 11/135833, CN 1907024, CN 1456054, CN 103387541, CN 1309897, WO 12/84812, CN 1907024, WO 09094442, WO 14/60177, WO 13/116251, WO 08/013622, WO 15/65922, WO 94/01546, EP 2865265, WO 07/129454, WO 12/165511, WO 11/081174, WO 13/47441, WO 16/156241, WO 16/162265). Some compounds are identified by their CAS Registry Number which is separated by hyphens into three parts, the first consisting from two up to seven digits, the second consisting of two digits, and the third consisting of a single digit.

According to the invention, the solid material (dry matter) of the biopesticides (with the exception of oils such as Neem oil) are considered as active components (e. g. to be obtained after drying or evaporation of the extraction or suspension medium in case of liquid formulations of the microbial pesticides). The weight ratios and percentages used for a biological extract such as Quillay extract are based on the total weight of the dry content (solid material) of the respective extract(s).

The total weight ratios of compositions comprising at least one microbial pesticide in the form of viable microbial cells including dormant forms, can be determined using the amount of CFU of the respective microorganism to calculate the total weight of the respective active component with the following equation that 1 x 10¹⁰ CFU equals one gram of total weight of the respective active component. Colony forming unit is measure of viable microbial cells. In addition, CFU may also be understood as the number of (juvenile) individual nematodes in case of nematode biopesticides, such as *Steinernema feltiae.*

In the binary mixtures the weight ratio of the component 1) and the component 2) generally depends from the properties of the components used, usually it is in the range of from 1:10,000 to 10,000:1, often from 1:100 to 100:1, regularly from 1:50 to 50:1, preferably from 1:20 to 20:1, more preferably from 1:10 to 10:1, even more preferably from 1:4 to 4:1 and in particular from 1:2 to 2:1. According to further embodiments, the weight ratio of the component 1) and the component 2) usually is in the range of from 1000:1 to 1:1, often from 100: 1 to 1:1, regularly from 50:1 to 1:1, preferably from 20:1 to 1:1, more preferably from 10:1 to 1:1, even more preferably from 4:1 to 1:1 and in particular from 2:1 to 1:1. According to further embodiments, the weight ratio of the component 1) and the component 2) usually is in the range of from 20,000:1 to 1:10, often from 10,000:1 to 1:1, regularly from 5,000:1 to 5:1, preferably from 5,000:1 to 10:1, more preferably from 2,000:1 to 30:1, even more preferably from 2,000:1 to 100:1 and in particular from 1,000:1 to 100:1. According to further embodiments, the weight ratio of the component 1) and the component 2) usually is in the range of from 1:1 to 1:1000, often from 1:1 to 1:100, regularly from 1:1 to 1:50, preferably from 1:1 to 1:20, more preferably from 1:1 to 1:10, even more preferably from 1: 1 to 1:4 and in particular from 1:1 to 1:2. According to further embodiments, the weight ratio of the component 1) and the component 2) usually is in the range of from 10:1 to 1:20,000, often from 1:1 to 1:10,000, regularly from 1:5 to 1:5,000, preferably from 1:10 to 1:5,000, more preferably from 1:30 to 1:2,000, even more preferably from 1:100 to 1:2,000 to and in particular from 1:100 to 1:1,000.

In the ternary mixtures, i.e. compositions comprising the component 1) and component 2) and a compound III (component 3), the weight ratio of component 1) and component 2) depends from the properties of the active substances used, usually it is in the range of from 1:100 to 100:1, regularly from 1:50 to 50:1, preferably from 1:20 to 20:1, more preferably from 1:10 to 10:1 and in particular from 1:4 to 4:1, and the weight ratio of component 1) and component 3) usually it is in the range of from 1:100 to 100:1, regularly from 1:50 to 50:1, preferably from 1:20 to 20:1, more preferably from 1:10 to 10:1 and in particular from 1:4 to 4:1. Any further active components are, if desired, added in a ratio of from 20:1 to 1:20 to the component 1). These ratios are also suitable for mixtures applied by seed treatment.

When mixtures comprising microbial pesticides are employed in crop protection, the application rates range from 1 x 10⁶ to 5 x 10¹⁶ (or more) CFU/ha, preferably from 1 x 10⁸ to 1 x 10¹³ CFU/ha, and even more preferably from 1 x 10⁹ to 5 x 10¹⁵ CFU/ha and in particular from 1 x 10¹² to 5 x 10¹⁴ CFU/ha. In the case of nematodes as microbial pesticides (e. g. *Steinernema feltiae*), the application rates regularly range from 1 x 10⁵ to 1 x 10¹² (or more), preferably from 1 x 10⁸ to 1 x 10¹¹, more preferably from 5 x 10⁸ to 1 x 10¹⁰ individuals (e. g. in the form of eggs, juvenile or any other live stages, preferably in an infetive juvenile stage) per ha.

When mixtures comprising microbial pesticides are employed in seed treatment, the application rates generally range from 1 x 10⁶ to 1 x 10¹² (or more) CFU/seed, preferably from 1 x 10⁶ to 1 x 10⁹ CFU/seed. Furthermore, the application rates with respect to seed treatment generally range from 1 x 10⁷ to 1 x 10¹⁴ (or more) CFU per 100 kg of seed, preferably from 1 x 10⁹ to 1 x 10¹² CFU per 100 kg of seed.

Preference is given to mixtures comprising as component 2) at least one active substance selected from inhibitors of complex III at Qₒ site in group A), more preferably selected from compounds (A.1.1), (A.1.4), (A.1.8), (A.1.9), (A.1.10), (A.1.12), (A.1.13), (A.1.14), (A.1.17), (A.1.21), (A.1.25), (A.1.34) and (A.1.35); particularly selected from (A.1.1), (A.1.4), (A.1.8), (A.1.9), (A.1.13), (A.1.14), (A.1.17), (A.1.25), (A.1.34) and (A.1.35).

Preference is also given to mixtures comprising as component 2) at least one active substance selected from inhibitors of complex III at Qᵢ site in group A), more preferably selected from compounds (A.2.1), (A.2.3) and (A.2.4); particularly selected from (A.2.3) and (A.2.4).

Preference is also given to mixtures comprising as component 2) at least one active substance selected from inhibitors of complex II in group A), more preferably selected from compounds (A.3.2), (A.3.3), (A.3.4), (A.3.7), (A.3.9), (A.3.11), (A.3.12), (A.3.15), (A.3.16), (A.3.17), (A.3.18), (A.3.19), (A.3.20), (A.3.21), (A.3.22), (A.3.23), (A.3.28), (A.3.31), (A.3.32), (A.3.33), (A.3.34), (A.3.35), (A.3.36), (A.3.37), (A.3.38) and (A.3.39); particularly selected from (A.3.2), (A.3.3), (A.3.4), (A.3.7), (A.3.9), (A.3.12), (A.3.15), (A.3.17), (A.3.19), (A.3.22), (A.3.23), (A.3.31), (A.3.32), (A.3.33), (A.3.34), (A.3.35), (A.3.36), (A.3.37), (A.3.38) and (A.3.39).

Preference is also given to mixtures comprising as component 2) at least one active substance selected from other respiration inhibitors in group A), more preferably selected from compounds (A.4.5) and (A.4.11); in particular (A.4.11).

Preference is also given to mixtures comprising as component 2) at least one active substance selected from C14 demethylase inhibitors in group B), more preferably selected from compounds (B.1.4), (B.1.5), (B.1.8), (B.1.10), (B.1.11), (B.1.12), (B.1.13), (B.1.17), (B.1.18), (B.1.21), (B.1.22), (B.1.23), (B.1.25), (B.1.26), (B.1.29), (B.1.34), (B.1.37), (B.1.38), (B.1.43), (B.1.46), (B.1.53), (B.1.54) and (B.1.55); in particular from (B.1.5), (B.1.8), (B.1.10), (B.1.17), (B.1.22), (B.1.23), (B.1.25), (B.1.33), (B.1.34), (B.1.37), (B.1.38), (B.1.43) and (B.1.46).

Preference is also given to mixtures comprising as component 2) at least one active substance selected from Delta14-reductase inhibitors in group B), more preferably selected from compounds (B.2.4), (B.2.5), (B.2.6) and (B.2.8); in particular (B.2.4).

Preference is also given to mixtures comprising as component 2) at least one active substance selected from phenylamides and acyl amino acid fungicides in group C), more preferably selected from compounds (C.1.1), (C.1.2), (C.1.4) and (C.1.5); particularly selected from (C.1.1) and (C.1.4).

Preference is also given to mixtures comprising as component 2) at least one active substance selected from other nucleic acid synthesis inhibitors in group C), more preferably selected from compounds (C.2.6), (C.2.7) and (C.2.8).

Preference is also given to mixtures comprising as component 2) at least one active substance selected from group D), more preferably selected from compounds (D.1.1), (D.1.2), (D.1.5), (D.2.4) and (D.2.6); particularly selected from (D.1.2), (D.1.5) and (D.2.6).

Preference is also given to mixtures comprising as component 2) at least one active substance selected from group E), more preferably selected from compounds (E.1.1), (E.1.3), (E.2.2) and (E.2.3); in particular (E.1.3).

Preference is also given to mixtures comprising as component 2) at least one active substance selected from group F), more preferably selected from compounds (F.1.2), (F.1.4) and (F.1.5).

Preference is also given to mixtures comprising as component 2) at least one active substance selected from group G), more preferably selected from compounds (G.3.1), (G.3.3), (G.3.6), (G.5.1), (G.5.3), (G.5.4), (G.5.5), G.5.6), G.5.7), (G.5.8), (G.5.9), (G.5.10) and (G.5.11); particularly selected from (G.3.1), (G.5.1) and (G.5.3).

Preference is also given to mixtures comprising as component 2) at least one active substance selected from group H), more preferably selected from compounds (H.2.2), (H.2.3), (H.2.5), (H.2.7), (H.2.8), (H.3.2), (H.3.4), (H.3.5), (H.4.9) and (H.4.10); particularly selected from (H.2.2), (H.2.5), (H.3.2), (H.4.9) and (H.4.10).

Preference is also given to mixtures comprising as component 2) at least one active substance selected from group I), more preferably selected from compounds (1.2.2) and (1.2.5).

Preference is also given to mixtures comprising as component 2) at least one active substance selected from group J), more preferably selected from compounds (J.1.2), (J.1.5), (J.1.8), (J.1.11) and (J.1.12); in particular (J.1.5).

Preference is also given to mixtures comprising as component 2) at least one active substance selected from group K), more preferably selected from compounds (K.1.41), (K.1.42), (K.1.44), (K.1.47), (K.1.57), (K.1.58) and (K.1.59); particularly selected from (K.1.41), (K.1.44), (K.1.47), (K.1.57), (K.1.58) and (K.1.59).

The biopesticides from group L1) and/or L2) may also have insecticidal, acaricidal, molluscidal, pheromone, nematicidal, plant stress reducing, plant growth regulator, plant growth promoting and/or yield enhancing activity. The biopesticides from group L3) and/or L4) may also have fungicidal, bactericidal, viricidal, plant defense activator, plant stress reducing, plant growth regulator, plant growth promoting and/or yield enhancing activity. The biopesticides from group L5) may also have fungicidal, bactericidal, viricidal, plant defense activator, insecticidal, acaricidal, molluscidal, pheromone and/or nematicidal activity.

The microbial pesticides, in particular those from groups L1), L3) and L5), embrace not only the isolated, pure cultures of the respective microorganism as defined herein, but also its cellfree extract, its suspension in a whole broth culture and a metabolite-containing culture medium or a purified metabolite obtained from a whole broth culture of the microorganism.

Many of these biopesticides have been deposited under deposition numbers mentioned herein (the prefices such as ATCC or DSM refer to the acronym of the respective culture collection, for details see e. g. here: http://www.wfcc.info/ccinfo/collection/by acronym/), are referred to in literature, registered and/or are commercially available: mixtures of *Aureobasidium pullulans* DSM 14940 and DSM 14941 isolated in 1989 in Konstanz, Germany (e. g. blastospores in BlossomProtect^{®} from bio-ferm GmbH, Austria), *Azospirillum brasilense* Sp245 originally isolated in wheat reagion of South Brazil (Passo Fundo) at least prior to 1980 (BR 11005; e. g. GELFIX^{®} Gramíneas from BASF Agricultural Specialties Ltd., Brazil), *A. brasilense* strains Ab-V5 and Ab-V6 (e. g. in AzoMax from Novozymes BioAg Produtos papra Agricultura Ltda., Quattro Barras, Brazil or Simbiose-Maíz^{®} from Simbiose-Agro, Brazil; Plant Soil 331, 413-425, 2010), *Bacillus amyloliquefaciens* strain AP-188 (NRRL B-50615 and B-50331; US 8,445,255); *B. amyloliquefaciens* ssp. *plantarum* strains formerly also sometimes referred to as *B. subtilis*, recently together with *B*. *methylotrophicus,* and *B*. *velezensis* classified as *B*. *velezensis* (Int. J. Syst. Evol. Microbiol. 66, 1212-1217, 2016): *B*. a. ssp. *plantarum* or *B*. *velezensis* D747 isolated from air in Kikugawa-shi, Japan (US 20130236522 A1; FERM BP-8234; e. g. Double Nickel^{™} 55 WDG from Certis LLC, USA), *B*. a. ssp. *plantarum* or *B*. *velezensis* FZB24 isolated from soil in Brandenburg, Germany (also called SB3615; DSM 96-2; J. Plant Dis. Prot. 105, 181-197, 1998; e. g. Taegro^{®} from Novozyme Biologicals, Inc., USA), *B*. a. ssp. *plantarum* or *B*. *velezensis* FZB42 isolated from soil in Brandenburg, Germany (DSM 23117; J. Plant Dis. Prot. 105, 181-197, 1998; e. g. RhizoVital^{®} 42 from AbiTEP GmbH, Germany), *B*. a. ssp. *plantarum* or *B*. *velezensis* MBI600 isolated from faba bean in Sutton Bonington, Nottinghamshire, U.K. at least before 1988 (also called 1430; NRRL B-50595; US 2012/0149571 A1; e. g. Integral^{®} from BASF Corp., USA), *B*. a. ssp. *plantarum* or *B*. *velezensis* QST-713 isolated from peach orchard in 1995 in California, U.S.A. (NRRL B-21661; e. g. Serenade^{®} MAX from Bayer Crop Science LP, USA), *B*. a. ssp. *plantarum* or *B*. *velezensis* TJ1000 isolated in 1992 in South Dakoda, U.S.A. (also called 1BE; ATCC BAA-390; CA 2471555 A1; e. g. QuickRoots^{™} from TJ Technologies, Watertown, SD, USA); *B. firmus* CNCM I-1582, a variant of parental strain EIP-N1 (CNCM I-1556) isolated from soil of central plain area of Israel (WO 2009/126473, US 6,406,690; e. g. Votivo^{®} from Bayer CropScience LP, USA), *B*. *pumilus* GHA 180 isolated from apple tree rhizosphere in Mexico (IDAC 260707-01; e. g. PRO-MIX^{®} BX from Premier Horticulture, Quebec, Canada), *B. pumilus* INR-7 otherwise referred to as BU-F22 and BU-F33 isolated at least before 1993 from cucumber infested by *Erwinia tracheiphila* (NRRL B-50185, NRRL B-50153; US 8,445,255), *B. pumilus* KFP9F isolated from the rhizosphere of grasses in South Africa at least before 2008 (NRRL B-50754; WO 2014/029697; e. g. BAC-UP or FUSION-P from BASF Agricultural Specialities (Pty) Ltd., South Africa), *B. pumilus* QST 2808 was isolated from soil collected in Pohnpei, Federated States of Micronesia, in 1998 (NRRL B-30087; e. g. Sonata^{®} or Ballad^{®} Plus from Bayer Crop Science LP, USA), *B*. *simplex* ABU 288 (NRRL B-50304; US 8,445,255), *B. subtilis* FB17 also called UD 1022 or UD10-22 isolated from red beet roots in North America (ATCC PTA-11857; System. Appl. Microbiol. 27, 372-379, 2004; US 2010/0260735; WO 2011/109395); *B. thuringiensis* ssp. *aizawai* ABTS-1857 isolated from soil taken from a lawn in Ephraim, Wisconsin, U.S.A., in 1987 (also called ABG-6346; ATCC SD-1372; e. g. XenTari^{®} from BioFa AG, Münsingen, Germany), *B. t.* ssp. *kurstaki* ABTS-351 identical to HD-1 isolated in 1967 from diseased Pink Bollworm black larvae in Brownsville, Texas, U.S.A. (ATCC SD-1275; e. g. Dipel^{®} DF from Valent BioSciences, IL, USA), *B. t.* ssp. *kurstaki* SB4 isolated from *E*. *saccharina* larval cadavers (NRRL B-50753; e. g. Beta Pro^{®} from BASF Agricultural Specialities (Pty) Ltd., South Africa), *B. t.* ssp. *tenebrionis* NB-176-1, a mutant of strain NB-125, a wild type strain isolated in 1982 from a dead pupa of the beetle Tenebrio molitor (DSM 5480; EP 585 215 B1; e. g. Novodor^{®} from Valent BioSciences, Switzerland), *Beauveria bassiana* GHA (ATCC 74250; e. g. BotaniGard^{®} 22WGP from Laverlam Int. Corp., USA), *B*. *bassiana* JW-1 (ATCC 74040; e. g. Naturalis^{®} from CBC (Europe) S.r.l., Italy), *B*. *bassiana* PPRI 5339 isolated from the larva of the tortoise beetle *Conchyloctenia punctata* (NRRL 50757; e. g. BroadBand^{®} from BASF Agricultural Specialities (Pty) Ltd., South Africa), *Bradyrhizobium elkanii* strains SEMIA 5019 (also called 29W) isolated in Rio de Janeiro, Brazil and SEMIA 587 isolated in 1967 in the State of Rio Grande do Sul, from an area previously inoculated with a North American isolate, and used in commercial inoculants since 1968 (Appl. Environ. Microbiol. 73(8), 2635, 2007; e. g. GELFIX 5 from BASF Agricultural Specialties Ltd., Brazil), *B. japonicum* 532c isolated from Wisconsin field in U.S.A. (Nitragin 61A152; Can. J. Plant. Sci. 70, 661-666, 1990; e. g. in Rhizoflo^{®}, Histick^{®}, Hicoat^{®} Super from BASF Agricultural Specialties Ltd., Canada), *B*. *japonicum* E-109 variant of strain USDA 138 (INTA E109, SEMIA 5085; Eur. J. Soil Biol. 45, 28-35, 2009; Biol. Fertil. Soils 47, 81-89, 2011); *B. japonicum* strains deposited at SEMIA known from Appl. Environ. Microbiol. 73(8), 2635, 2007: SEMIA 5079 isolated from soil in Cerrados region, Brazil by Embrapa-Cerrados used in commercial inoculants since 1992 (CPAC 15; e. g. GELFIX 5 or ADHERE 60 from BASF Agricultural Specialties Ltd., Brazil), *B*. *japonicum* SEMIA 5080 obtained under lab condtions by Embrapa-Cerrados in Brazil and used in commercial inoculants since 1992, being a natural variant of SEMIA 586 (CB1809) originally isolated in U.S.A. (CPAC 7; e. g. GELFIX 5 or ADHERE 60 from BASF Agricultural Specialties Ltd., Brazil); *Burkholderia sp.* A396 isolated from soil in Nikko, Japan, in 2008 (NRRL B-50319; WO 2013/032693; Marrone Bio Innovations, Inc., USA), *Coniothyrium minitans* CON/M/91-08 isolated from oilseed rape (WO 1996/021358; DSM 9660; e. g. Contans^{®} WG, Intercept^{®} WG from Bayer CropScience AG, Germany), harpin (alpha-beta) protein (Science 257, 85-88, 1992; e. g. Messenger^{™} or HARP-N-Tek from Plant Health Care plc, U.K.), *Helicoverpa armigera* nucleopolyhedrovirus (HearNPV) (J. Invertebrate Pathol. 107, 112-126, 2011; e. g. Helicovex^{®} from Adermatt Biocontrol, Switzerland; Diplomata^{®} from Koppert, Brazil; Vivus^{®} Max from AgBiTech Pty Ltd., Queensland, Australia), *Helicoverpa zea* single capsid nucleopolyhedrovirus (HzSNPV) (e. g. Gemstar@ from Certis LLC, USA), *Helicoverpa zea* nucleopolyhedrovirus ABA-NPV-U (e. g. Heligen^{®} from AgBiTech Pty Ltd., Queensland, Australia), *Heterorhabditis bacteriophora* (e. g. Nemasys^{®} G from BASF Agricultural Specialities Limited, UK), *Isaria fumosorosea* Apopka-97 isolated from mealy bug on gynura in Apopka, Florida, U.S.A. (ATCC 20874; Biocontrol Science Technol. 22(7), 747-761, 2012; e. g. PFR-97^{™} or PreFeRal^{®} from Certis LLC, USA), *Metarhizium anisopliae* var. *anisopliae* F52 also called 275 or V275 isolated from codling moth in Austria (DSM 3884, ATCC 90448; e. g. Met52^{®} Novozymes Biologicals BioAg Group, Canada), *Metschnikowia fructicola* 277 isolated from grapes in the central part of Israel (US 6,994,849; NRRL Y-30752; e. g. formerly Shemer^{®} from Agrogreen, Israel), *Paecilomyces ilacinus* 251 isolated from infected nematode eggs in the Philippines (AGAL 89/030550; WO1991/02051; Crop Protection 27, 352-361, 2008; e. g. BioAct^{®}from Bayer CropScience AG, Germany and MeloCon^{®} from Certis, USA), *Paenibacillus alvei* NAS6G6 isolated from the rhizosphere of grasses in South Africa at least before 2008 (WO 2014/029697; NRRL B-50755; e.g. BAC-UP from BASF Agricultural Specialities (Pty) Ltd., South Africa), *Paenibacillus* strains isolated from soil samples from a variety of European locations including Germany: *P. epiphyticus* Lu17015 (WO 2016/020371; DSM 26971), *P. polymyxa* ssp. *plantarum* Lu16774 (WO 2016/020371; DSM 26969), *P. p.* ssp. *plantarum* strain Lu17007 (WO 2016/020371; DSM 26970); *Pasteuria nishizawae* Pn1 isolated from a soybean field in the mid-2000s in Illinois, U.S.A. (ATCC SD-5833; Federal Register 76(22), 5808, February 2, 2011; e.g. Clariva^{™} PN from Syngenta Crop Protection, LLC, USA), *Penicillium bilaiae* (also called P. *bilaii*) strains ATCC 18309 (= ATCC 74319), ATCC 20851 and/or ATCC 22348 (= ATCC 74318) originally isolated from soil in Alberta, Canada (Fertilizer Res. 39, 97-103, 1994; Can. J. Plant Sci. 78(1), 91-102, 1998; US 5,026,417, WO 1995/017806; e. g. Jump Start^{®}, Provide^{®} from Novozymes Biologicals BioAg Group, Canada), *Reynoutria sachalinensis* extract (EP 0307510 B1; e. g. Regalia^{®} SC from Marrone Biolnnovations, Davis, CA, USA or Milsana^{®} from BioFa AG, Germany), *Steinernema carpocapsae* (e. g. Millenium^{®} from BASF Agricultural Specialities Limited, UK), *S*. *feltiae* (e. g. Nemashield^{®} from BioWorks, Inc., USA; Nemasys^{®} from BASF Agricultural Specialities Limited, UK), *Streptomyces microflavus* NRRL B-50550 (WO 2014/124369; Bayer CropScience, Germany), *Trichoderma asperelloides* JM41R isolated in South Africa (NRRL 50759; also referred to as *T. fertile;* e. g. Trichoplus^{®} from BASF Agricultural Specialities (Pty) Ltd., South Africa), *T. harzianum* T-22 also called KRL-AG2 (ATCC 20847; BioControl 57, 687-696, 2012; e. g. Plantshield^{®} from BioWorks Inc., USA or SabrEx^{™} from Advanced Biological Marketing Inc., Van Wert, OH, USA).

According to another embodiment of the mixtures, the at least one pesticide II is selected from the groups L1) to L5):
L1) Microbial pesticides with fungicidal, bactericidal, viricidal and/or plant defense activator activity: *Aureobasidium pullulans* DSM 14940 and DSM 14941 (L1.1), *Bacillus amyloliquefaciens* AP-188 (L.1.2), *B. amyloliquefaciens* ssp. *plantarum* D747 (L.1.3), *B*. *amyloliquefaciens* ssp. *plantarum* FZB24 (L.1.4), *B. amyloliquefaciens* ssp. *plantarum* FZB42 (L.1.5), *B. amyloliquefaciens* ssp. *plantarum* MBI600 (L.1.6), *B. amyloliquefaciens* ssp. *plantarum* QST-713 (L.1.7), *B. amyloliquefaciens* ssp. *plantarum* TJ1000 (L.1.8), *B. pumilus* GB34 (L.1.9), *B. pumilus* GHA 180 (L.1.10), *B. pumilus* INR-7 (L.1.11), *B. pumilus* KFP9F (L.1.12), *B. pumilus* QST2808 (L.1.13), *B. simplex* ABU 288 (L.1.14), *B. subtilis* FB17 (L.1.15), *Coniothyrium minitans* CON/M/91-08 (L.1.16), *Metschnikowia fructicola* NRRL Y-30752 (L.1.17), *Paenibacillus alvei* NAS6G6 (L.1.18), *P. epiphyticus* Lu17015 (L.1.25), P. *polymyxa* ssp. *plantarum* Lu16774 (L.1.26), *P. p.* ssp. *plantarum* strain Lu17007 (L.1.27), *Penicillium bilaiae* ATCC 22348 (L.1.19), *P. bilaiae* ATCC 20851 (L.1.20), *Penicillium bilaiae* ATCC 18309 (L.1.21), *Streptomyces microflavus* NRRL B-50550 (L.1.22), *Trichoderma asperelloides* JM41R (L.1.23), *T. harzianum* T-22 (L.1.24);
L2) Biochemical pesticides with fungicidal, bactericidal, viricidal and/or plant defense activator activity: harpin protein (L.2.1), *Reynoutria sachalinensis* extract (L.2.2);
L3) Microbial pesticides with insecticidal, acaricidal, molluscidal and/or nematicidal activity: *Bacillus firmus* 1-1582 (L.3.1); *B. thuringiensis* ssp. *aizawai* ABTS-1857 (L.3.2), *B. t.* ssp. *kurstaki* ABTS-351 (L.3.3), *B. t.* ssp. *kurstaki* SB4 (L.3.4), *B. t.* ssp. *tenebrionis* NB-176-1 (L.3.5), *Beauveria bassiana* GHA (L.3.6), *B*. *bassiana* JW-1 (L.3.7), *B*. *bassiana* PPRI 5339 (L.3.8), *Burkholderia* sp. A396 (L.3.9), *Helicoverpa armigera* nucleopolyhedrovirus (HearNPV) (L.3.10), *Helicoverpa zea* nucleopolyhedrovirus (HzNPV) ABA-NPV-U (L.3.11), *Helicoverpa zea* single capsid nucleopolyhedrovirus (HzSNPV) (L.3.12), *Heterohabditis bacteriophora* (L.3.13), *Isaria fumosorosea* Apopka-97 (L.3.14), *Metarhizium anisopliae* var. *anisopliae* F52 (L.3.15), *Paecilomyceslilacinus* 251 (L.3.16), *Pasteuria nishizawae* Pn1 (L.3.17), *Steinernema carpocapsae* (L.3.18), *S*. *feltiae* (L.3.19);
L4) Biochemical pesticides with insecticidal, acaricidal, molluscidal, pheromone and/or nematicidal activity: cis-jasmone (L.4.1), methyl jasmonate (L.4.2), Quillay extract (L.4.3);
L5) Microbial pesticides with plant stress reducing, plant growth regulator, plant growth promoting and/or yield enhancing activity: *Azospirillum brasilense* Ab-V5 and Ab-V6 (L.5.1), *A. brasilense* Sp245 (L.5.2), *Bradyrhizobium elkanii* SEMIA 587 (L.5.3), *B. elkanii* SEMIA 5019 (L.5.4), *B. japonicum* 532c (L.5.5), *B. japonicum* E-109 (L.5.6), *B. japonicum* SEMIA 5079 (L.5.7), *B. japonicum* SEMIA 5080 (L.5.8).

The present invention furthermore relates to agrochemical compositions comprising a mixture of at least one compound I (component 1) and at least one biopesticide selected from the group L) (component 2), in particular at least one biopesticide selected from the groups L1) and L2), as described above, and if desired at least one suitable auxiliary.

The present invention furthermore relates to agrochemical compositions comprising a mixture of of at least one compound I (component 1) and at least one biopesticide selected from the group L) (component 2), in particular at least one biopesticide selected from the groups L3) and L4), as described above, and if desired at least one suitable auxiliary.

Preference is also given to mixtures comprising as pesticide II (component 2) a biopesticide selected from the groups L1), L3) and L5), preferably selected from strains denoted above as (L.1.2), (L.1.3), (L.1.4), (L.1.5), (L.1.6), (L.1.7), (L.1.8), (L.1.10), (L.1.11), (L.1.12), (L.1.13), (L.1.14), (L.1.15), (L.1.17), (L.1.18), (L.1.19), (L.1.20), (L.1.21), (L.1.25), (L.1.26), (L.1.27), (L.3.1); (L.3.9), (L.3.16), (L.3.17), (L.5.1), (L.5.2), (L.5.3), (L.5.4), (L.5.5), (L.5.6), (L.5.7), (L.5.8); (L.4.2), and (L.4.1); even more preferably selected from (L.1.2), (L.1.6), (L.1.7), (L.1.8), (L.1.11), (L.1.12), (L.1.13), (L.1.14), (L.1.15), (L.1.18), (L.1.19), (L.1.20), (L.1.21), (L.3.1); (L.3.9), (L.3.16), (L.3.17), (L.5.1), (L.5.2), (L.5.5), (L.5.6); (L.4.2), and (L.4.1). These mixtures are particularly suitable for treatment of propagation materials, i. e. seed treatment purposes and likewise for soil treatment. These seed treatment mixtures are particularly suitable for crops such as cereals, corn and leguminous plants such as soybean.

Preference is also given to mixtures comprising as pesticide II (component 2) a biopesticide selected from the groups L1), L3) and L5), preferably selected from strains denoted above as (L1.1), (L.1.2), (L.1.3), (L.1.6), (L.1.7), (L.1.9), (L.1.11), (L.1.12), (L.1.13), (L.1.14), (L.1.15), (L.1.17), (L.1.18), (L.1.22), (L.1.23), (L.1.24), (L.1.25), (L.1.26), (L.1.27), (L.2.2); (L.3.2), (L.3.3), (L.3.4), (L.3.5), (L.3.6), (L.3.7), (L.3.8), (L.3.10), (L.3.11), (L.3.12), (L.3.13), (L.3.14), (L.3.15), (L.3.18), (L.3.19); (L.4.2), even more preferably selected from (L.1.2), (L.1.7), (L.1.11), (L.1.13), (L.1.14), (L.1.15), (L.1.18), (L.1.23), (L.3.3), (L.3.4), (L.3.6), (L.3.7), (L.3.8), (L.3.10), (L.3.11), (L.3.12), (L.3.15), and (L.4.2). These mixtures are particularly suitable for foliar treatment of cultivated plants, preferably of vegetables, fruits, vines, cereals, corn, and leguminous crops such as soybeans.

The compositions comprising mixtures of active ingredients can be prepared by usual means, e. g. by the means given for the compositions of compounds I.

When living microorganisms, such as pesticides II from groups L1), L3) and L5), form part of the compositions, these can be prepared by usual means (Burges: Formulation of Microbial Biopesticides, Springer, 1998; WO 2008/002371, US 6,955,912, US 5,422,107).

### Examples:

### Synthetic process

### Example 1: Methyl (2E)-2-methoxyimino-2-[2-[[(E)-(1-methyl-3-phenyl-prop-2-ynylidene)amino]-oxymethyl]phenyl]acetate (examples numbered according to Table S below)

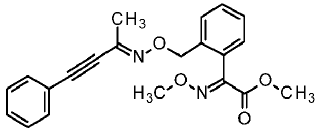

### Step 1: 4-Phenylbut-3-yn-2-one

To a stirred solution of phenyl acetylene (10 g, 1 eq.) in THF (150 ml) at -78 °C was added n-butyl lithium (73 ml, 1.6 M solution in hexane 1.2 eq.) dropwise and stirred at the same temperature for 30 minutes. Then ethyl acetate (12 ml, 1.2 eq.) in THF (15ml) was added dropwise at -78 °C followed by boron trifluoride diethyl ether complex (33 ml, 50% solution, 1.2 eq.) at the same temperature and the reaction mixture was stirred for 30 min. After completion of the reaction indicated by TLC, the reaction mixture was quenched with saturated aqueous ammonium chloride solution (200 ml) and then water (100 mL) was added and the mixture was extracted with ethyl acetate (3x 200 mL) and the combined organic phase was washed using brine and dried over sodium sulfate. The solvent was removed to obtain crude product, which was purified by combi flash column chromatography using 15-20% ethyl acetate in heptane as mobile phase to obtain 4-phenylbut-3-yn-2-one (10 g, 71% yield). ¹H NMR (500 MHz, DMSO-d6): δ 7.67-7.65(m, 2H), 7.59 - 7.56 (m, 1H), 7.37 - 7.15 (m, 2H), 2.45 (s, 3H).

### Step 2: 4-Phenylbut-3-yn-2-one oxime

To a stirred solution of 4-phenylbut-3-yn-2-one (200 mg, 1 eq.) in methanol (10 ml) and water (2.0 ml) at 25 °C hydroxylamine hydrochloride (190 mg, 2 eq.) and sodium acetate (394 mg, 2 eq.) were added. The mixture was stirred at about 20 °C for 4 h. After TLC indicated completion of the reaction, the mixture was evaporated to dryness and water (30 ml) added to it and extracted with ethyl acetate (3x 30 ml) and the combined organic phase was washed using brine and dried over sodium sulfate. The solvent was removed to obtain 220 mg crude 4-phenylbut-3-yn-2-one oxime as a mixture of both cis and trans (50:50) isomers, which was directly used for the next step without any purification.

### Step 3: Methyl (2E)-2-methoxyimino-2-[2-[[(E)-(1-methyl-3-phenyl-prop-2-ynylidene)amino]oxymethyl]phenyl]acetate

To a stirred solution of 4-phenylbut-3-yn-2-one oxime (15 g as a mixture of cis:trans isomers) in AcN (200 ml) at 10 °C, methyl (2*E*)-2-[2-(chloromethyl)phenyl]-2-methoxyimino-acetate (82 ml, 33% solution in DMF) was added followed by cesium carbonate (61.4 g, 2 eq.). The reaction mixture was then stirred at 25 °C for 16 h. After TLC indicated completion of the reaction, water (200 ml) was added and extracted with ethyl acetate (3x 200 ml). The combined organic phase was washed with brine and dried over sodium sulfate. The solvent was removed to obtain crude product, which was purified by column chromatography to obtain 7.5 g (21% yield) of pure title compound. ¹H NMR (500 MHz, DMSO-d6): δ 7.54-7.52 (m, 2H), 7.46-7.41 (m, 6H), 7.24-7.22 (m, 1H), 5.01 (s, 2H), 3.96 (s, 3H), 3.76 (s, 3H), 1.99 (s, 3H).

Alternatively, the title compound was obtained as follows.

### Step 2a: Methyl (2E)-2-[2-(aminooxymethyl)phenyl]-2-methoxyimino-acetate

To a stirred solution of methyl (2E)-2-[2-[(1,3-dioxoisoindolin-2-yl)oxymethyl]phenyl]-2-methoxyimino-acetate (400 mg, 1 eq.) in methanol (10 ml) at 25 °C, hydrazine hydrate (54 mg, 1 eq.) was added and stirred for 30 minutes at the same temperature. After TLC showed completion of the reaction, the solvent was evaporated and the crude reaction mixture was subjected to column chromatography to afford the pure product (150 mg).¹H NMR (300 MHz, DMSO-d6): δ 7.41-7.33 (m, 3H), 7.18-7.16 (m, 1H), 5.93 (s, 2H), 4.39 (s, 2H), 3.92 (s, 3H), 3.75 (s, 3H).

### Step 3a: Methyl (2E)-2-methoxyimino-2-[2-[[(E)-(1-methyl-3-phenyl-prop-2-ynylidene)amino]oxymethyl]phenyl]acetate

To a stirred solution of 4-phenylbut-3-yn-2-one (240 mg, 1.2 eq.) and methyl (2*E*)-2-[2-(aminooxymethyl)phenyl]-2-methoxyimino-acetate (330 mg, 1 eq.) in methanol (5 ml), acetic acid (0.2 ml) was added and heated at 65 °C and stirred at this temperature for 2 h. After TLC indicated completion of the reaction, the mixture was evaporated to dryness and water (50 ml) added to it and extracted with ethyl acetate (3x 30 ml) and the combined organic phase was washed using brine and dried over sodium sulfate. The crude product was purified by column chromatography using 20% ethyl acetate in heptane as mobile phase to obtain 160 mg (32 % yield) of the title compound.

### Example 2: (2E)-2-Methoxyimino-N-methyl-2-[2-[[(E)-(1-methyl-3-phenyl-prop-2-ynylidene)-amino]oxymethyl]phenyl]acetamide

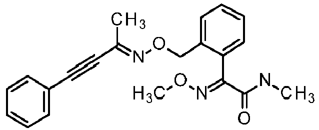

To a stirred solution of methyl (2E)-2-methoxyimino-2-[2-[[(E)-(1-methyl-3-phenyl-prop-2-ynylidene)amino]oxymethyl]phenyl]acetate (Example 1) (120 mg, 1 eq.) in THF (5 mL) at 25 °C was added methyl amine (0.4 mL, 40% solution in water) and was stirred for 4 h. After completion of the reaction as indicated by TLC, the reaction mixture was evaporated and washed with pentane (5 mL x 3 times) and was purified by combi flash column chromatography to obtain 11 mg (9% yield) of pure title compound.

### Example 15: Methyl (2E)-2-methoxyimino-2-[3-methyl-2-[[(E)-(1-methyl-3-phenyl-prop-2-ynylidene)amino]oxymethyl]phenyl]acetate

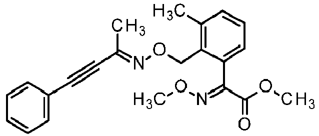

To a stirred solution of 4-phenylbut-3-yn-2-one oxime (1 g, as a mixture of cis:trans isomers, 1 eq.) in acetonitrile (15 ml), cesium carbonate (4.08 g, 2 eq.) and methyl (2*E*)-2-[2-(bromomethyl)-3-methyl-phenyl]-2-methoxyimino-acetate (1.7 g, 1 eq.) dissolved in acetonitrile (5 ml) were added at 25 °C and stirred at this temperature for 4 h. After TLC indicated completion of the reaction, the mixture was filtered through celite and washed with ethyl acetate (3x 30 ml). The filtrate was then evaporated and purified by column chromatography using 15% ethyl acetate in heptane as mobile phase to obtain 1.1 g (yield 51%) of the title compound.
¹H NMR (500 MHz, DMSO-d6): δ 7.54-7.52 (m, 2H), 7.47-7.42 (m, 3H), 7.34-7.29 (m, 2H), 7.04-7.02 (m, 1H), 5.01 (bs, 2H), 3.93 (s, 3H), 3.74 (s, 3H) 2.40 (s, 3H), 1.96 (s, 3H).

### Example 16: (2E)-2-Methoxyimino-N-methyl-2-[3-methyl-2-[[(E)-(1-methyl-3-phenyl-prop-2-ynylidene)amino]oxymethyl]phenyl]acetamide

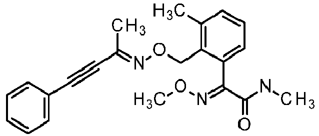

To a stirred solution of methyl (2*E*)-2-methoxyimino-2-[2-[[(*E*)-(1-methyl-3-phenyl-prop-2-ynylidene)amino]oxymethyl]phenyl]acetate (Example 15) (750 mg, 1 eq.) in THF (10 ml) at 25 °C, methyl amine (0.6 ml, 40% solution in water) was added and stirred for 5 h. After completion of the reaction as indicated TLC, water (50 ml) was added to the reaction mixture and was extracted by using ethyl acetate (3x 30 ml) and the combined organic phase was washed using brine and dried over sodium sulfate. The solvent was removed to obtain crude product, which was purified by column chromatography using 35% ethyl acetate in heptane as mobile phase to obtain 450 mg (60% yield) of pure title compound. ¹H NMR (500 MHz, DMSO-d6): δ 8.21 (s, 1H) 7.54-7.52 (m, 2H), 7.46-7.42 (m, 3H), 7.31-7.26 (m, 2H), 6.96-6.94 (m, 1H), 5.00 (bs, 2H), 3.88 (s, 3H), 2.70 (s, 3H) 2.39 (s, 3H), 1.96 (s, 3H).

### Example 12: methyl(2E)-2-methoxyimino-2-[2-[[(E)-[1-methyl-3-[4-(trifluoromethoxy)phenyl]prop-2-ynylidene]amino]oxymethyl]phenyl]acetate

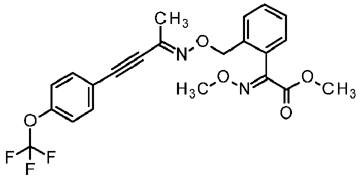

To a stirred solution of 4-[4-(trifluoromethoxy)phenyl]but-3-yn-2-one oxime (300 mg as a mixture of cis:trans isomers, 1 eq.) in acetonitrile (8 ml), cesium carbonate (820 mg, 2 eq.) and methyl (2*E*)-2-[2-(bromomethyl)phenyl]-2-methoxyimino-acetate (350 mg, 1 eq.) were added at 25 °C and stirred at this temperature for 2 h. After TLC indicated completion of the reaction, the mixture was filtered through Celite and washed with ethyl acetate (25 ml). The filtrate was then evaporated and purified by column chromatography using 15-20% ethyl acetate in heptane as mobile phase to obtain the title compound (500 mg, 90% yield). ¹H NMR (500 MHz, DMSO-d6): δ 7.69-7.67 (m, 2H), 7.46 - 7.24 (m, 5H), 7.24-7.22 (m, 1H), 5.01 (s, 2H), 3.95 (s, 3H), 3.76 (s, 3H), 1.99 (s, 3H).

### Example 11: (2E)-2-Methoxyimino-N-methyl-2-[2-[[(E)-[1-methyl-3-[4-(trifluoromethoxy)phenyl]-prop-2-ynylidene]amino]oxymethyl]phenyl]acetamide

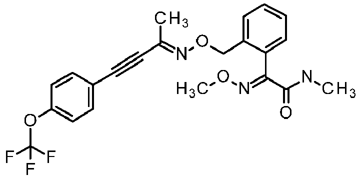

To a stirred solution of methyl (2*E*)-2-methoxyimino-2-[2-[[(*E*)-[1-methyl-3-[4-(trifluoromethoxy)phenyl]prop-2-ynylidene]amino]oxymethyl]phenyl]acetate (Example 12 above) in THF (6 ml) at room temperature, methyl amine (0.6 ml, 40% solution in water) was added and stirred for 2 h. After completion of the reaction as indicated TLC, water (15 ml) was added to the reaction mixture and was extracted by using ethyl acetate (3x 15ml) and the combined organic phase was washed using brine and dried over sodium sulfate. The solvent was removed to obtain crude product, which was purified by column chromatography using 30-35% ethyl acetate in heptane as mobile phase to obtain the title compound (220mg, 73% yield). ¹H NMR (500 MHz, DMSO-d6): δ 8.26 (s, 1H), 8.25 - 8.25 (d, J = 5Hz, 2H), 7.69 - 7.67 (m, 5H), 7.36-7.15 (m, 1H), 5.02 (s, 2H), 3.90 (s, 3H), 2.71 (d, J = 5Hz, 3H), 2.02 (s, 3H).

### Example 22: methyl (2E)-2-methoxyimino-2-[3-methyl-2-[[(E)-[1-methyl-3-[4-(trifluoromethoxy)-phenyl]prop-2-ynylidene]amino]oxymethyl]phenyl]acetate

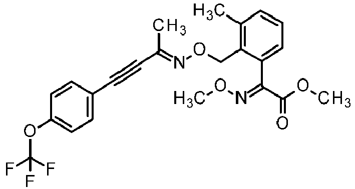

### Step 1: Trimethyl-[2-[4-(trifluoromethoxy)phenyl]ethynyl]silane

To a stirred solution of 1-iodo-4-(trifluoromethoxy)benzene (500 mg 1 eq.) in THF (5 ml) under nitrogen, triethylamine (3.5 ml, 3 eq.), trimethylsilyl acetylene (0.731 ml, 3 eq.), Cul (33 mg, 0.1 eq.) and PdCl₂(PPh₃)₂ (122 mg, 0.1 eq.) were added and stirred at 60 °C for 2 h. After TLC indicated completion of the reaction, the mixture was filtered through Celite and water (10 ml) was added. The organic phase was extracted by using ethyl acetate (3x 15 ml) and the combined organic phase was washed using brine and dried over sodium sulfate. The solvent was removed to obtain crude product, which was purified by column chromatography to obtain trimethyl-[2-[4-(trifluoromethoxy)phenyl]ethynyl]silane (400 mg, 89% yield). ¹H NMR (500 MHz, CDCl₃): δ 7.29 (d, J = 8.00 Hz, 2H), 6.95 (d, J = 8.00 Hz, 2H), 0.06 (s, 9H).

### Step 2: 1-Ethynyl-4-(trifluoromethoxy)benzene

To a stirred solution of trimethyl-[2-[4-(trifluoromethoxy)phenyl]ethynyl]silane (4.4 g, 1 eq.) in THF (44 ml), tetrabutylammonium fluoride (TBAF) (1.7 ml, 1 M solution in THF) was added dropwise at 0 °C. The resulting solution was stirred at 0 to 5 °C for 5 min. After completion of the reaction as indicated by TLC, the reaction mixture was quenched by adding water (50 ml) at 0 °C. The organic phase was extracted by using ethyl acetate (3x 25 ml) and the combined organic phase was washed using brine and dried over sodium sulfate. The solvent was removed to obtain crude product, which was purified by column chromatography using 2-5% ethyl acetate in heptane as mobile phase to obtain 1-ethynyl-4-(trifluoromethoxy)benzene (1 g, 41% yield). ¹H NMR (500 MHz, CDCl₃): δ 7.43 (d, J = 8.00 Hz, 2H), 7.08 (d, J = 8.00 Hz, 2H), 3.01 (s, 1H).

### Step 3: 4-[4-(Trifluoromethoxy)phenyl]but-3-yn-2-one

To a stirred solution of 1-ethynyl-4-(trifluoromethoxy)benzene (1 g, 1 eq.) in THF (10 ml) at -78°C, 2.5 M solution of n-butyl lithium (2.58 ml, 2.5 M in hexane, 1.2 eq.) was added dropwise and stirred at the same temperature for 20 min. Ethyl acetate (0.63 ml, 1.2 eq.) in THF (2 ml) was then added dropwise at -78 °C followed by boron trifluoride diethyl ether complex (0.72 ml, 1.2 eq.) at the same temperature and stirred the reaction mixture for 1 h. After completion of the reaction as indicated by TLC, the reaction mixture was quenched with saturated ammonium chloride solution and extracted with ethyl acetate (3x 15 ml) and the combined organic phase was washed using brine and dried over sodium sulfate. After removal of the solvent, the crude product was purified by column chromatography using 15-20% ethyl acetate in heptane as mobile phase to obtain 4-[4-(trifluoromethoxy)phenyl]but-3-yn-2-one (0.5 g, 41 % yield). ¹H NMR (500 MHz, CDCl₃): δ 7.64 (d, J = 8.00 Hz, 2H), 7.26 (d, J = 8.00 Hz, 2H), 3.1 (s, 3H).

### Step 4: 4-[4-(Trifluoromethoxy)phenyl]but-3-yn-2-one oxime

To a stirred solution of 4-[4-(trifluoromethoxy)phenyl]but-3-yn-2-one (500 mg, 1 eq.) in a mixture of methanol: water (4:1) at 10 °C, hydroxylamine hydrochloride (302 mg, 2 eq.) and sodium acetate (359 mg, 2 eq.) were added. The mixture was stirred at 10 to 15 °C for 2 h. After TLC indicated completion of the reaction, the mixture was evaporated to dryness and water (15 ml) was added and extracted with ethyl acetate (3x 15 ml). The combined organic phase was washed using brine and dried over sodium sulfate and solvent was removed to obtain 4-[4-(trifluoromethoxy)phenyl]but-3-yn-2-one oxime (500 mg, 93% yield) as a mixture of *E* and *Z* isomers (50:50), which was directly used for the next step without any purification.

### Step 5: Methyl (2E)-2-methoxyimino-2-[3-methyl-2-[[(E)-[1-methyl-3-[4-(trifluoromethoxy)phenyl]prop-2-ynylidene]amino]oxymethyl]phenyl]acetate

To a stirred solution of 4-[4-(trifluoromethoxy)phenyl]but-3-yn-2-one oxime (250 mg as a mixture of cis:trans isomers, 1 eq.) in acetonitrile (8 ml), cesium carbonate (670 mg, 2 eq.) and methyl (2*E*)-2-[2-(bromomethyl)-3-methyl-phenyl]-2-methoxyimino-acetate (308 mg, 1 eq.) were added at 25 °C and stirred at this temperature for 2 h. After TLC indicated completion of the reaction, the mixture was filtered through Celite and washed with ethyl acetate (25 ml). The filtrate was then evaporated and purified by column chromatography using 15-20% ethyl acetate in heptane as mobile phase to obtain the title compound (400 mg, 84% yield). ¹H NMR (500 MHz, DMSO-d6): δ 7.77 (d, J = 8 Hz, 2H), 7.49 (d, J = 8 Hz, 2H), 7.34 - 7.29 (m, 2H), 7.01 - 7.00 (m, 1H), 5.50 (bs, 1H), 5.00 (bs, 1H), 3.86 (s, 3H), 3.65 (s, 3H), 2.46 (s, 3H), 2.05 (s, 3H).

### Example 21: (2E)-2-Methoxyimino-N-methyl-2-[3-methyl-2-[[(E)-[1-methyl-3-[4-(trifluoromethoxy)phenyl]prop-2-ynylidene]amino]oxymethyl]phenyl]acetamide

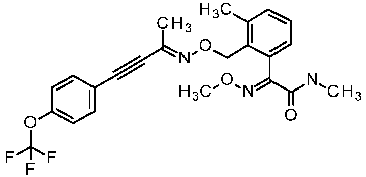

To a stirred solution of methyl (2*E*)-2-methoxyimino-2-[3-methyl-2-[[(*E*)-[1-methyl-3-[4-(trifluoromethoxy)phenyl]prop-2-ynylidene]amino]oxymethyl]phenyl]acetate (Example 22 above) (300 mg, 1 eq) in THF (6 ml) at 25 °C, methyl amine (0.6 ml, 40% solution in water) was added and stirred for 2 h. After completion of the reaction as indicated TLC, water (15 ml) was added to the reaction mixture and was extracted by using ethyl acetate (3x 15ml) and the combined organic phase was washed using brine and dried over sodium sulfate. After removal of the solvent, the crude product was purified by column chromatography using 30-35% ethyl acetate in heptane as mobile phase to obtain the title compound (220 mg 71% yield). ¹H NMR (500 MHz, DMSO-d6): δ 8.2 (bs, 1H), 7.67 (d, J = 5 Hz, 2H), 7.44 (d, J = 10 Hz, 2H), 7.30 - 7.27 (m, 2H), 6.9 - 7.00 (m, 1H), 5.00 (bs, 2H), 3.3 (s, 3H), 2.68 (d, 3H), 2.39 (s, 3H), 1.97 (s, 3H).

The following examples in Table S were synthesized as described above and characterized by LCMS as described in Table L.

**Table L: LCMS Method**

| **Method details** | **Device details** |
|---|---|
| Column: Agilent Eclipse Plus C18 | LCMS2020 (Shimadzu) |
| (50 mm × 4.6 mm × 3µ) | Ionization source: ESI |
| Mobile Phase: | Mass range: 100 - 800 amu |
| A: 10 mM Ammonium formate in water. | Polarity: Dual (positive and negative |
| B: 0.1 % Formic acid in acetonitrile. | simultaneous scan); Mode: Scan |
| Gradient: 10 % B to 100 % B in 1.5 min. Hold | LC System: Nexera High pressure gradient |
| 1 min 100 % B. 1 min 10 % B. | system, Binary pump |
| Run time: 3.50 or 3.75 min. | Detector: PDA |
| Flow: 1.2 ml/min; Column oven: 30°C/40°C | Scanning wavelength: 220 nm / max plot |

**Table S:**

| **No** | **Structure** | **Rₜ [min]** | **Mass** |
|---|---|---|---|
| 1 | 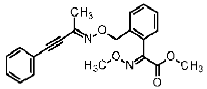 | 2.04 | 365 |
| 2 | 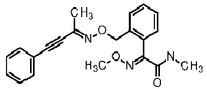 | 1.93 | 364 |
| 3 | 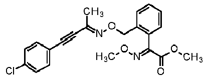 | 2.21 | 399 |
| 4 | 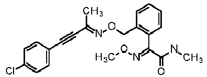 | 2.1 | 398 |
| 5 | 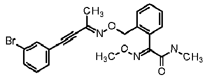 | 2.15 | 444 |
| 6 | 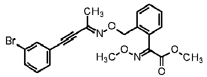 | 2.07 | 443 |
| 7 | 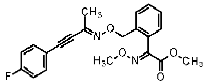 | 2.11 | 383 |
| 8 | 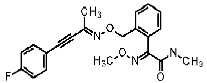 | 2.04 | 382 |
| 9 | 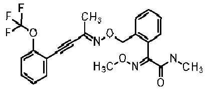 | 2.10 | 448 |
| 10 | 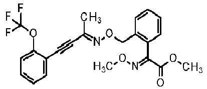 | 2.23 | 449 |
| 11 | 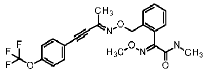 | 2.14 | 448 |
| 12 | 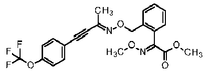 | 2.23 | 449 |
| 13 | 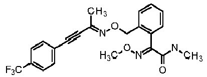 | 2.13 | 432 |
| 14 | 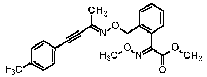 | 2.21 | 433 |
| 15 | 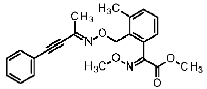 | 2.17 | 379 |
| 16 | 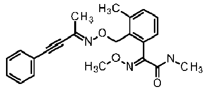 | 2.05 | 378 |
| 17 | 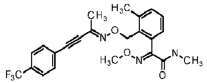 | 2.18 | 446 |
| 18 | 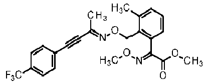 | 2.26 | 447 |
| 19 | 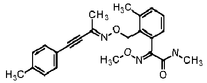 | 2.16 | 392 |
| 20 | 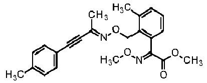 | 2.27 | 383 |
| 21 | 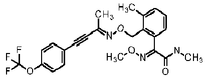 | 2.19 | 462 |
| 22 | 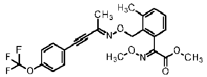 | 2.08 | 463 |
| 23 | 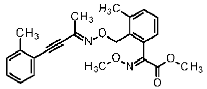 | 2.24 | 393 |
| 24 | 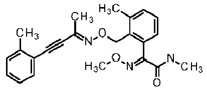 | 2.14 | 392 |
| 25 | 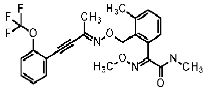 | 2.17 | 462 |
| 26 | 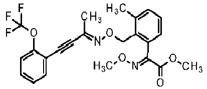 | 2.25 | 463 |
| 27 | 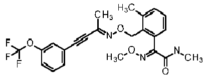 | 2.19 | 462 |
| 28 | 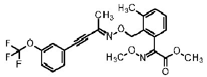 | 2.28 | 463 |
| 29 | 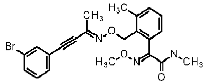 | 2.19 | 456 |
| 30 | 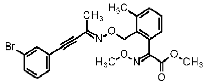 | 2.3 | 459 |
| 31 | 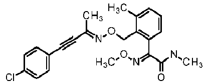 | 2.16 | 412 |
| 32 | 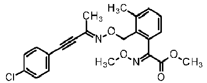 | 2.2 | 413 |
| 33 | 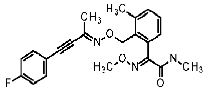 | 2.07 | 396 |
| 34 | 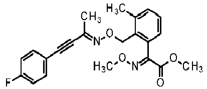 | 2.18 | 397 |

### Biological studies

### Green House

The compound was dissolved in a mixture of acetone and/or dimethylsulfoxide and the wetting agent/emulsifier Wettol, which is based on ethoxylated alkylphenoles, in a ratio (volume) solvent-emulsifier of 99 to 1 to give a total volume of 5 ml. Subsequently, water was added to total volume of 100 ml. This stock solution was then diluted with the described solvent-emulsifier-water mixture to the final concentration given in the table below.

### Use example 1. Protective control of soybean rust on soybeans caused by Phakopsora pachyrhizi (PHAKPA P2)

Leaves of potted soybean seedlings were sprayed to run-off with the previously described spray solution, containing the concentration of active ingredient or their mixture as described below. The plants were allowed to air-dry. The trial plants were cultivated for 2 days in a greenhouse chamber at 23-27 °C and a relative humidity between 60 and 80 %. Then the plants were inoculated with spores of *Phakopsora pachyrhizi.* The strain used contains the amino acid substitution F129L in the mitochondrial cytochrome b protein conferring resistance to Qo inhibitors. To ensure the success the artificial inoculation, the plants were transferred to a humid chamber with a relative humidity of about 95 % and 20 to 24 °C for 24 hr. The trial plants were cultivated for up to 14 days in a greenhouse chamber at 23 to 27 °C and a relative humidity between 60 and 80 %. The extent of fungal attack on the leaves was visually assessed as % diseased leaf area, the disease level of untreated controls was usually higher than 85 %.

### Use example 2. Protective control of soybean rust on soybeans caused by Phakopsora pachyrhizi (PHAKPA P6)

Leaves of potted soybean seedlings were sprayed to run-off with the previously described spray solution, containing the concentration of active ingredient as described below. The plants were allowed to air-dry. The trial plants were cultivated for six days in a greenhouse chamber at 23-27 °C and a relative humidity between 60 and 80 %. Then the plants were inoculated with spores of *Phakopsora pachyrhizi.* The strain used contains the amino acid substitution F129L in the mitochondrial cytochrome b protein conferring resistance to Qo inhibitors. To ensure the success the artificial inoculation, the plants were transferred to a humid chamber with a relative humidity of about 95 % and 23 to 27 °C for 24 hr. The trial plants were cultivated for up to 14 days in a greenhouse chamber at 23 to 27 °C and a relative humidity between 60 and 80 %. The extent of fungal attack on the leaves was visually assessed as % diseased leaf area, the disease level of untreated controls was usually higher than 85 %.

The results of the abovementioned use examples are given in the following Tables. All test results below are given for the control of phytopathogenic fungi containing the amino acid substitution F129L in the mitochondrial cytochrome b protein conferring resistance to Qo inhibitors.

**Table 1:**

| | | **PHAKPA Disease level (%)** | |
|---|---|---|---|
| **No.** | **Structure** | **P2 at 16 ppm** | **P6 at 16 ppm** |
| 1 | 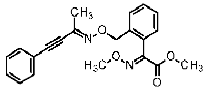 | 38 | 58 |
| 2 | 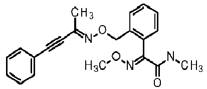 | 97 | 90 |
| 3 | 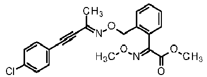 | 70 | 70 |
| 4 | 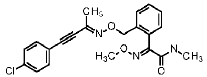 | 3 | 7 |
| 5 | 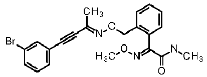 | 1 | 7 |
| 6 | 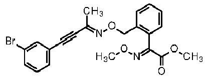 | 25 | 43 |
| 7 | 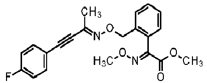 | 30 | 50 |
| 8 | 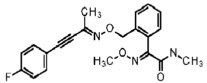 | 0 | 0.7 |
| 9 | 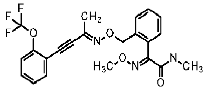 | 0 | 5 |
| 10 | 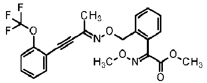 | 70 | 83 |
| 11 | 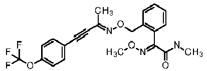 | 53 | 67 |
| 12 | 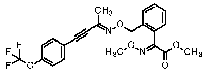 | 93 | 100 |
| 16 | 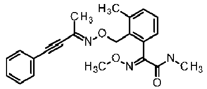 | 0 | 0 |
| 19 | 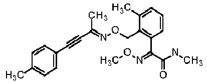 | 0 | 0.6 |
| 20 | 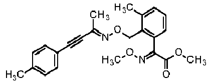 | 2 | 1 |
| 21 | 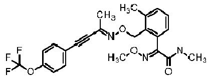 | 25 | 33 |
| 22 | 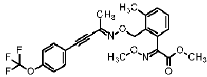 | 93 | 97 |
| 23 | 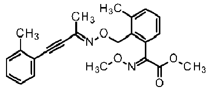 | 0 | 0 |
| 25 | 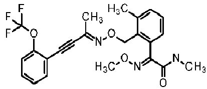 | 0 | 0 |
| 26 | 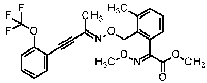 | 2 | 6 |
| 27 | 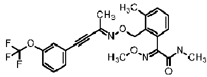 | 0 | 0 |
| 28 | 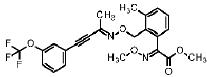 | 0 | 0 |
| 29 | 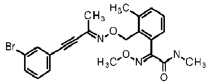 | 1 | 0 |
| 30 | 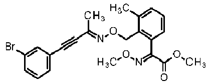 | 0 | 0 |
| 31 | 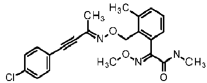 | 0 | 5 |
| 32 | 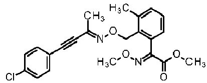 | 12 | 35 |
| 33 | 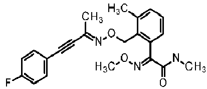 | 0 | 0 |
| 34 | 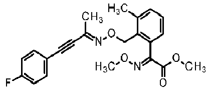 | 0 | 0 |

## Claims

1. Compounds of formula I 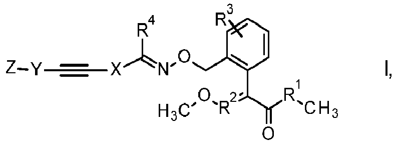 wherein
R¹ is selected from O and NH;
R² is selected from CH and N;
R³ is selected from hydrogen, halogen, CN, C₁-C₄-alkyl, C₂-C₄-alkenyl, C₂-C₄-alkynyl, C₁-C₄-haloalky, C₂-C₄-haloalkenyl, C₂-C₄-haloalkynyl, C₃-C₆-cycloalkyl, -O-C₁-C₄-alkyl, -O-C₁-C₄-haloalkyl, -O-C₃-C₆-cycloalkyl, -C₁-C₂-alkyl-C₃-C₆-cycloalkyl, phenyl, 3- to 6-membered heterocycloalkyl and 5- or 6-membered heteroaryl,
wherein said heterocycloalkyl and heteroaryl besides carbon atoms contain 1, 2 or 3 heteroatoms selected from N, O and S provided that such heterocycle cannot contain 2 contiguous atoms selected from O and S,
wherein said phenyl, heterocycloalkyl and heteroaryl are bound directly or via an oxygen atom or via a C₁-C₂-alkylene linker, and wherein said phenyl and heteroaryl are unsubstituted or substituted by 1, 2 or 3 identical or different substituents selected from halogen, CN, NH₂, NO₂, C₁-C₄-alkyl, C₁-C₄-haloalkyl, -O-C₁-C₄-alkyl and -O-C₁-C₄-haloalkyl;
R⁴ is selected from hydrogen, C₁-C₆-alkyl, C₂-C₄-alkenyl, C₂-C₄-alkynyl, C₁-C₆-haloalkyl, C₂-C₄-haloalkenyl, C₂-C₄-haloalkynyl, -(C₁-C₂-alkyl)-O-(C₁-C₂-alkyl), -(C₁-C₂-alkyl)-O-(C₁-C₂-haloalkyl) and -C₁-C₄-alkyl-C₃-C₆-cycloalkyl;
X, Y , independently of each other, are a direct bond or the divalent group -CL¹L²-;
L¹, L², independently of each other, are selected from hydrogen, C₁-C₃-alkyl, C₁-C₃-haloalkyl, C₂-C₃-haloalkenyl, C₂-C₃-haloalkynyl, -(C₁-C₂-alkyl)-O-(C₁-C₂-alkyl), -(C₁-C₂-alkyl)-O-(C₁-C₂-haloalkyl), cyclopropyl and -C₁-C₂-alkyl-cyclopropyl; or
L¹ and L², together with the interjacent carbon atom, form a cyclopropyl;
wherein the cyclic moieties of L¹ and L², independently of each other, are unsubstituted or carry 1 or 2 identical or different groups R^{L}:
R^{L} is selected from halogen, CN, NO₂, C₁-C₄-alkyl, C₁-C₄-haloalkyl, -O-C₁-C₄-alkyl, and -O-C₁-C₄-haloalkyl;
Z is selected from C₃-C₆-cycloalkyl, phenyl, 3- to 6-membered heterocycloalkyl, 3- to 6-membered heterocycloalkenyl and 5- or 6-membered heteroaryl,
wherein said heterocycloalkyl, heterocycloalkenyl and heteroaryl besides carbon atoms contain 1, 2 or 3 heteroatoms selected from N, O and S provided that such heterocycle cannot contain 2 contiguous atoms selected from O and S,
and wherein Z is unsubstituted or carries 1, 2, 3 or up to the maximum number of identical or different groups R^{a}:
R^{a} is selected from halogen, CN, C₁-C₄-alkyl, C₂-C₄-alkenyl, C₂-C₄-alkynyl, -O-C₁-C₄-alkyl, -C(=N-O-C₁-C₄-alkyl)-C₁-C₄-alkyl, -C(=O)-C₁-C₄-alkyl, -O-CH₂-C(=N-O-C₁-C₄-alkyl)-C₁-C₄-alkyl, C₃-C₆-cycloalkyl, C₃-C₆-cycloalkenyl, -C₁C₂-alkyl-C₃-C₆-cycloalkyl, -O-C₃-C₆-cycloalkyl, phenyl, 3- to 6-membered heterocycloalkyl, 3- to 6-membered heterocycloalkenyl and 5- or 6-membered heteroaryl,
wherein said heterocycloalkyl, heterocycloalkenyl and heteroaryl besides carbon atoms contain 1, 2 or 3 heteroatoms selected from N, O and S provided that such heterocycle cannot contain 2 contiguous atoms selected from O and S,
wherein said phenyl, heterocycloalkyl, heterocycloalkenyl and heteroaryl are bound directly or via an oxygen atom or via a C₁-C₂-alkylene linker, and wherein the aliphatic and cyclic moieties of R^{a} are unsubstituted or carry 1, 2, 3, 4 or up to the maximum number of identical or different groups R^{b}:
R^{b} is selected from halogen, CN, NO₂, C₁-C₄-alkyl, C₁-C₄-haloalkyl, -O-C₁-C₄-alkyl, and -O-C₁₋C₄-haloalkyl;
and in form or stereoisomers and tautomers thereof, and the N-oxides and the agriculturally acceptable salts thereof.

2. The compounds according to claim 1, wherein R¹ is selected from O and NH; and R² is selected from CH and N, provided that R² is N in case R¹ is NH.

3. The compounds according to any one of the claims 1 to 2, wherein R² is N.

4. The compounds according to any one of the claims 1 to 3, wherein R³ is selected from hydrogen, CN, halogen, C₁-C₂-alkyl, C₁-C₂-haloalkyl, C₂-C₄-alkenyl, C₃-C₄-cycloalkyl, -O-C₁-C₂-alkyl and -O-C₁-C₂-haloalkyl.

5. The compounds according to claim 4, wherein R³ is selected from hydrogen, halogen, C₁-C₂-alkyl, and C₁-C₂-haloalkyl.

6. The compounds according to any one of the claims 1 to 5, wherein R³ is ortho position to the methyl oxime side chain.

7. The compounds according to any one of the claims 1 to 6, wherein R⁴ is selected from halogen, C₁-C₂-alkyl, C₁-C₂-haloalky, C₃-C₄-cycloalkyl, -O-C₁-C₂-alkyl and -O-C₁-C₂-haloalkyl.

8. The compounds according to any one of the claims 1 to 7, wherein X is a direct bond.

9. The compounds according to any one of the claims 1 to 8, wherein Y is a direct bond.

10. The compounds according to any one of the claims 1 to 9, wherein Z is selected from C₃-C₄-cycloalkyl, phenyl and 5- or 6-membered heteroaryl, wherein said heteroaryl besides carbon atoms contain 1, 2 or 3 heteroatoms selected from N, O and S provided that such heterocycle cannot contain 2 contiguous atoms selected from O and S, wherein said phenyl, and wherein Z is unsubstituted or carries 1, 2 or 3 identical or different groups R^{a} selected from halogen, CN, C₁-C₂-alkyl, C₁--O-C₁-C₂-alkyl, -O-C₁-C₂-haloalkyl, C₃-C₄-cycloalkyl and C₃-C₄-halocycoalkyl.

11. The compounds according to claim 10, wherein Z is phenyl, and wherein Z is unsubstituted or carries 1, 2 or 3 identical or different groups R^{a} selected from halogen, CN, C₁-C₂-alkyl, C₁-C₂-haloalkyl, -O-C₁-C₂-alkyl, -O-C₁-C₂-haloalkyl, C₃-C₄-cycloalkyl and C3-C4-halocycloalkyl.

12. Agrochemical compositions comprising an auxiliary and at least one compound of formula I as defined in any of claims 1 to 11 or in the form of a stereoisomer and tautomer thereof or an agriculturally acceptable salt or N-oxide thereof.

13. Use of the compounds as defined in any of the claims 1 to 11 or a composition as defined in claim 12 for combating phytopathogenic fungi.

14. A method for combating phytopathogenic fungi comprising:
treating curatively and/or preventively the plants or the plant propagation material of said plants that are at risk of being diseased from the said phytopathogenic fungi, and/or
applying to the said phytopathogenic fungi, at least one compound of formula I as defined in any of the claims 1 to 11 or an agrochemical composition as defined in claim 12.

15. The use according to claim 13 or the method according to claim 14, wherein the phytopathogenic fungi contain an amino acid substitution F129L in the mitochondrial cytochrome b protein conferring resistance to Qo inhibitors.
